# EUROPEAN PATENT SPECIFICATION

(11) **EP 2 834 375 B1**
(45) Date of publication and mention of the grant of the patent: **05.05.2021**
(21) Application number: 13772940.6
(22) Date of filing: 14.03.2013
(51) Int. Cl.: C12N 15/31, C12Q 1/689

(54) **SEQUENCES FOR DETECTION AND IDENTIFICATION OF METHICILLIN-RESISTANT STAPHYLOCOCCUS AUREUS (MRSA) OF MREJ TYPE XXI**
SEQUENZEN ZUM NACHWEIS UND ZUR IDENTIFIZIERUNG VON METHICILLINRESISTENTEM STAPHYLOCOCCUS AUREUS (MRSA) VOM MREJ-TYP XXI
SÉQUENCES PERMETTANT DE DÉTECTER ET D'IDENTIFIER LE STAPHYLOCOCCUS AUREUS RÉSISTANT À LA MÉTHICILLINE (SARM) DE TYPE MREJ XXI

(30) Priority: 06.04.2012 US 201261621368 P
(43) Date of publication of application: 11.02.2015
(73) Proprietor: Geneohm Sciences Canada, Inc., Québec, QC G1P 4S5 (CA)
(72) Inventor: MENARD, Christian, St-Lambert-de-Lauzon QC G0S 2W0 (CA); ROGER-DALBERT, Celine, Borel, QC G1X 4T8 (CA)
(74) Representative: Vossius & Partner Patentanwälte Rechtsanwälte mbB
(86) International application number: PCT/IB2013/000900
(87) International publication number: WO 2013/150376

(56) References cited:
- WO-A1-2011/038197
- WO-A2-02/099034
- WO-A2-02/099034
- WO-A2-2004/055205
- WO-A2-2007/044873
- WO-A2-2008/140612
- US-A1- 2008 220 428
- HULETSKY A ET AL: "New real-time PCR assay for rapid detection of methicillin-resistant Staphylococcus aureus directly from specimens containing a mixture of staphylococci", JOURNAL OF CLINICAL MICROBIOLOGY, AMERICAN SOCIETY FOR MICROBIOLOGY, US, vol. 42, no. 5, 1 May 2004 (2004-05-01), pages 1875-1884, XP003003502, ISSN: 0095-1137, DOI: 10.1128/JCM.42.5.1875-1884.2004
- HULETSKY, A. ET AL.: 'New real-real time PCR assay for rapid detection of methicillin-resistant Staphylococcus aureus directly from specimens containing a mixture of staphylococci.' JOURNAL OF CLINICAL MICROBIOLOGY. vol. 42, no. 5, May 2004, pages 1875 - 1884, XP003003502
- ITO T ET AL: "Structural comparison of three types of staphylococcal cassette chromosome mec integrated in the chromosome in methicillin-resistant Staphylococcus aureus", ANTIMICROBIAL AGENTS AND CHEMOTHERAPY, AMERICAN SOCIETY FOR MICROBIOLOGY, vol. 45, no. 5, 1 May 2001 (2001-05-01), pages 1323-1336, XP002238384, ISSN: 0066-4804, DOI: 10.1128/AAC.45.5.1323-1336.2001

## Description

### Field

Disclosed herein are molecular diagnostic tools for the detection of methicillin-resistant *Staphylococcus aureus.*

### Related Art

The coagulase-positive species *Staphylococcus aureus* (*S. aureus*) is well documented as a human opportunistic pathogen (Murray et al. Eds, 1999, Manual of Clinical Microbiology, 7th ed., ASM Press, Washington, D.C.). Nosocomial infections caused by *S*. *aureus* are a major cause of morbidity and mortality. Some of the most common infections caused by *S. aureus* involve the skin, and they include furuncles or boils, cellulitis, impetigo, and postoperative wound infections at various sites. Some of the more serious infections produced by *S. aureus* are bacteremia, pneumonia, osteomyelitis, acute endocarditis, myocarditis, pericarditis, cerebritis, meningitis, scalded skin syndrome, and various abscesses. Food poisoning mediated by staphylococcal enterotoxins is another important syndrome associated with *S. aureus.* Toxic shock syndrome, a community-acquired disease, has also been attributed to infection or colonization with toxigenic *S. aureus.*

Methicillin-resistant *S. aureus* (MRSA) emerged in the 1980s as a major clinical and epidemiologic problem in hospitals (Oliveira et al., (2002) Lancet Infect Dis. 2:180-9). MRSA are resistant to all β-lactams including penicillins, cephalosporins, carbapenems, and monobactams, which are the most commonly used antibiotics to cure *S. aureus* infections.

MRSA infections can only be treated with toxic and more costly antibiotics, which are normally used as the last line of defense. Since MRSA can spread easily from patient to patient via personnel, hospitals over the world are confronted with the problem of controlling MRSA.

Not that long ago, the only way to know a strain's resistance was to perform a manual antimicrobial susceptibility testing. Antimicrobial susceptibility testing suffers from many drawbacks, including the extensive time (at least 48 hours) before the results are available, a lack of reproducibility, a lack of standardization of the process, and user errors. Consequently, there is a need to develop rapid and simple screening or diagnostic tests for detection and/or identification of MRSA to reduce its dissemination and improve the diagnosis and treatment of infected patients.

There is a need for compositions and methods for quick and sensitive detection of MRSA.

The invention relates to the embodiments defined in the claims.

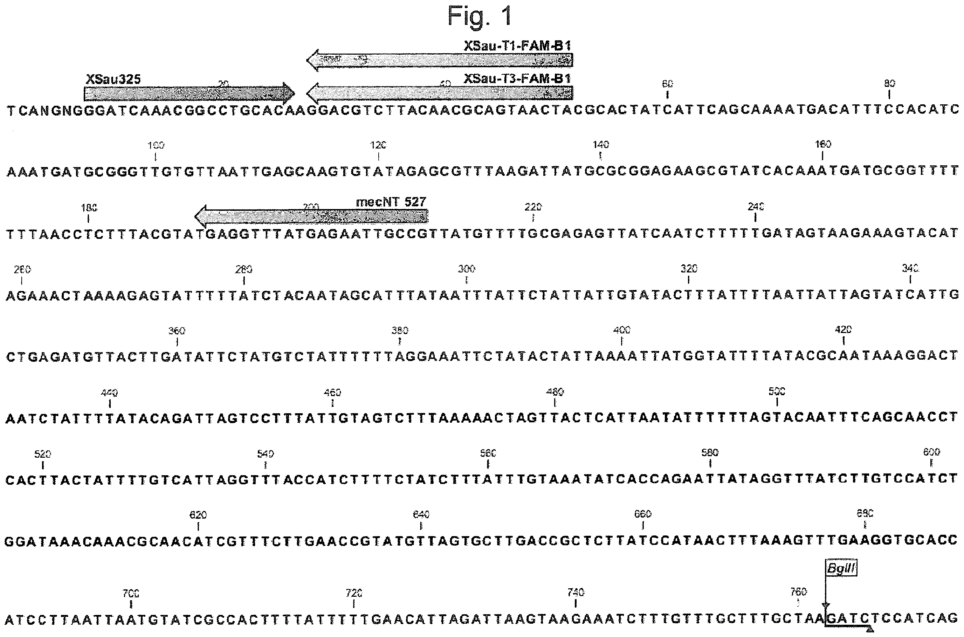

In particular, the present invention relates to the nucleic acids of the sequence shown in Figure 1 (SEQ ID NO.: 188) or the complement of said sequence.

The invention further relates to an oligonucleotide comprising the nucleic acids of SEQ ID NO.: 2, or the complement thereof, that specifically hybridizes to the nucleic acid of the present invention.

The invention further relates to a composition for the detection of methicillin-resistant *Staphylococcus aureus* (MRSA) comprising MREJ type xxi nucleic acids, said *S. aureus* comprising a *Staphylococcal* cassette chromosome *mec* (*SCCmec*) element including a *mecA* homolog (*mecA*_{LGA251}), said *SCCmec* cassette being inserted into *S*. *aureus* chromosomal DNA, thereby generating a polymorphic right extremity junction (MREJ) type xxi sequence that comprises polymorphic sequences from the right extremity and chromosomal DNA adjoining the polymorphic right extremity, said composition comprising:
a first amplification primer, said first amplification primer between 10 and 45 nucleotides in length, wherein said first amplification primer specifically hybridizes under standard PCR conditions to the polymorphic right extremity sequences of the MREJ type xxi nucleic acids of the sequence of the invention and is fully complementary to the polymorphic right extremity sequences of the MREJ type xxi nucleic acids of the invention, and
a second amplification primer, said second amplification primer between 10 and 45 nucleotides in length, wherein said second amplification primer specifically hybridizes under standard PCR conditions to *S. aureus* chromosomal sequences located within 1 kilobase from the insertion point of the *SCCmec* element into the chromosomal DNA, and wherein said first and second amplification primer together generate an amplicon of the right extremity junction of MREJ type xxi nucleic acids under the standard PCR conditions in the presence of MRSA comprising MREJ type xxi nucleic acids, wherein said amplicon comprises a MREJ type xxi-specific sequence.

The invention further relates to a method for the detection of methicillin-resistant *Staphylococcus aureus* (MRSA) comprising MREJ type xxi nucleic acids, said *S. aureus* comprising a *Staphylococcal* cassette chromosome mec (*SCCmec*) element including a *mecA* homolog (*mecA*_{LGA251})*,* said *SCCmec* cassette being inserted into *S. aureus* chromosomal DNA, thereby generating a polymorphic right extremity junction (MREJ) type xxi sequence that comprises polymorphic sequences from the right extremity and chromosomal DNA adjoining the polymorphic right extremity, said method comprising:
contacting a test sample with a first amplification primer, said first amplification primer between 10 and 45 nucleotides in length, wherein said first amplification primer specifically hybridizes under standard PCR conditions to the polymorphic right extremity sequences of the MREJ type xxi sequence of the sequence of the invention; contacting the sample with a second amplification primer between 10 and 45 nucleotides in length, wherein said second amplification primer hybridizes under the standard PCR conditions to the *orfX* gene of *S. aureus,* and wherein said first and second amplification primer together generate an amplicon of the *SCCmec* right extremity junction (MREJ) xxi nucleic acids under the standard PCR conditions in the presence of MRSA comprising MREJ type xxi nucleic acids, wherein said amplicon comprises a MREJ type xxi-specific sequence; and
determining whether an amplicon of the MREJ type xxi-specific nucleic acids is generated following the contacting step.

### SUMMARY

The embodiments disclosed herein are based, in part, upon the discovery that certain strains of *Staphylococcus aureus,* including those that harbor a *mecA* homolog gene, *mecA*_{LGA251}*,* share the same sequence located at the right extremity of the *SCCmec* region of the MRSA nucleic acids, *i.e.,* the polymorphic right extremity junction. Provided herein are methods and compositions that can be used to detect these MRSA strains, which were heretofore undetectable by conventional commercial molecular based assays. Also provided herein are compositions and methods that allow for the further (*e.g*., either simultaneous or sequential) detection of *Staphylococcus aureus* generally, and/or for the further detection of *mecA* and/or *mecA*_{LGA251*,*} in addition to MRSA strains.

Accordingly, provided herein are methods and compositions for the detection of MRSA that harbor an MREJ type xxi sequence. Some embodiments described herein provide a composition for the detection of methicillin-resistant *Staphylococcus aureus* (MRSA) having MREJ type xxi nucleic acids. The MRSA can include a *Staphylococcal* cassette chromosome mec (*SCCmec*) element including a *mecA* homolog (*mecA*_{LGA251}*,* or *mecC*), wherein the *SCCmec* cassette is inserted into *S. aureus* chromosomal DNA, thereby generating a polymorphic right extremity junction (MREJ) type xxi sequence that comprises polymorphic sequences from the right extremity and chromosomal DNA adjoining the polymorphic right extremity. The composition can include a first amplification primer that is between 10 and 45 nucleotides in length, and that specifically hybridizes under standard PCR conditions to the polymorphic right extremity sequences of the MREJ type xxi nucleic acids. In some embodiments described herein, the first amplification primer specifically hybridizes to the nucleic acid sequence of SEQ ID NO:1 or the complement thereof under said standard PCR conditions.

The compositions disclosed herein can further include a second amplification primer between 10 and 45 nucleotides in length that specifically hybridizes under standard PCR conditions to *S. aureus* chromosomal sequences located within 1 kilobase from the insertion point of the *SCCmec* element into the chromosomal DNA. Preferably, the first and second amplification primers together generate an amplicon of the right extremity junction of MREJ type xxi nucleic acids under the standard PCR conditions in the presence of MRSA comprising MREJ type xxi nucleic acids. Accordingly, in some embodiments described herein, the second amplification primer specifically hybridizes under standard PCR conditions to *orfX.* The compositions disclosed herein can further include a probe, *e.g*., an oligonucleotide probe comprises a fluorescence emitter moiety and a fluorescence quencher moiety, that specifically hybridizes to the amplicon of the MREJ type xxi nucleic acids under the standard PCR conditions.

The compositions disclosed herein can include one or more additional amplification primers between 10 and 45 nucleotides in length that specifically hybridize to one or more polymorphic *SCCmec* right extremity sequences from an MREJ type i to xx MRSA, *i.e.,* to one or more polymorphic *SCCmec* right extremity sequences selected from the group consisting of SEQ ID NOs: 5 to 29.

The methods and compositions can also include further oligonucleotides, *i.e.,* that are configured to specifically amplify *mecA* and/or *mecA*_{LGA251}/*mecC* sequences, and/or that are configured to specifically amplify *Staphylococcus aureus-specific* sequences.

Accordingly, some embodiments described herein provide compositions wherein the first amplification primer is at least 80% identical to SEQ ID NO:2. In some embodiments described herein, the compositions can include a second amplification primer that is at least 80% identical to SEQ ID NO:3. In some embodiments described herein, the composition can include a probe, wherein the probe is at least 80% identical to SEQ ID NO:4 or 82.

In some embodiments described herein, the compositions disclosed herein are provided in dried form, *e.g*., lyophilized or the like.

Also provided herein are methods for the detection of methicillin-resistant *Staphylococcus aureus* (MRSA) comprising MREJ type xxi nucleic acids, and for the detection of MREJ type xxi nucleic acids, wherein the *S. aureus* includes a *Staphylococcal* cassette chromosome mec *(SCCmec)* element including a *mecA* homolog (*mecA*_{LGA251} or *mecC*). The SCC*mec* cassette can be inserted into *S. aureus* chromosomal DNA, thereby generating a polymorphic right extremity junction (MREJ) type xxi sequence that comprises polymorphic sequences from the right extremity (MREP sequences) and chromosomal DNA adjoining the polymorphic right extremity. Accordingly, in some embodiments described herein, the methods can include the steps of providing a test sample; contacting the sample with a first amplification primer between 10 and 45 nucleotides in length, that specifically hybridizes under standard PCR conditions to the polymorphic right extremity sequences of the MREJ type xxi sequence; wherein the contacting is performed under conditions wherein an amplicon of the mec right extremity junction of the MREJ type xxi nucleic acids is generated if *S*. *aureus* comprising MREJ type xxi nucleic acids is present in the sample. The method can also include the step of determining whether or not an amplicon of the MREJ type xxi nucleic acids is generated following the contacting step. In some embodiments described herein, the first amplification primer specifically hybridizes to the nucleic acid sequence of SEQ ID NO: 1 under said standard PCR conditions.

In some embodiments described herein, the method can include contacting the sample with a second amplification primer between 10 and 45 nucleotides in length that hybridizes under the standard PCR conditions to the *orfX* gene of *S. aureus,* wherein said first and second amplification primer together generate an amplicon of the *SCCmec* right extremity junction (MREJ) region sequence of the *SCCmec* right extremity junction of MRSA under the standard PCR conditions in the presence MREJ type xxi nucleic acids.

As described herein, the method may further comprise contacting the sample with a probe, wherein said probe specifically hybridizes to the amplicon of the *SCCmec* right extremity junction (MREJ) region sequence of MREJ type xxi nucleic acids under the standard PCR conditions. In some embodiments described herein, the probe includes a fluorescence emitter moiety and a fluorescence quencher moiety.

In some embodiments described herein, the contacting step also includes contacting the sample with one or more additional amplification primers, wherein said one or more additional amplification primers are between 10 and 45 nucleotides in length that specifically hybridizes to one or polymorphic *SCCmec* right extremity sequence from an MREJ type i to xx MRSA. In some embodiments described herein, the contacting step also includes contacting the sample with one or more additional amplification primers between 10 and 45 nucleotides in length that specifically hybridizes to and are configured to generate an amplicon of *mecA* sequences, *mecC* sequences and/or *Staphylococcus aureus*-specific sequences. Non-limiting examples of *S*. *aureus-specific* sequences include, but are not limited to *nuc* sequences, rRNA sequences, *femB* sequences, Sa442 sequences, and the like. The skilled artisan will readily appreciate, however, that any sequence that is unique to *Staphylococcus aureus* can be used in the embodiments described herein.

Accordingly, the contacting step of the methods described herein can include performing a nucleic acid amplification reaction, such as PCR, strand displacement amplification (SDA), for example multiple displacement amplification (MDA), loop-mediated isothermal amplification (LAMP), ligase chain reaction (LCR), immuno-amplification, nucleic acid sequence based amplification (NASBA), self-sustained sequence replication (3SR), or rolling circle amplification. In some preferred embodiments described herein, the method includes performing multiplex PCR. In some preferred embodiments described herein, the method includes performing real-time PCR.

### BRIEF DESCRIPTION OF THE DRAWINGS

**Figure 1** shows the sequence of a type xxi MREJ region. Also shown are the locations of various primers and probes disclosed in the embodiments described herein.

### DETAILED DESCRIPTION

It is to be understood that both the foregoing general description and the following detailed description are exemplary and explanatory only and are not intended to limit the scope of the current teachings. In this application, the use of the singular includes the plural unless specifically stated otherwise. Also, the use of "comprise", "contain", and "include", or modifications of those root words, for example but not limited to, "comprises", "contained", and "including", are not intended to be limiting. Use of "or" means "and/or" unless stated otherwise. The term "and/or" means that the terms before and after can be taken together or separately. For illustration purposes, but not as a limitation, "X and/or Y" can mean "X" or "Y" or "X and Y".

Whenever a range of values is provided herein, the range is meant to include the starting value and the ending value and any value or value range there between unless otherwise specifically stated. For example, "from 0.2 to 0.5" means 0.2, 0.3, 0.4, 0.5; ranges there between such as 0.2-0.3, 0.3-0.4, 0.2-0.4; increments there between such as 0.25, 0.35, 0.225, 0.335, 0.49; increment ranges there between such as 0.26-0.39; and the like.

Provided herein are compositions and methods for the improved detection of methicillin-resistant *Staphylococcus aureus* (MRSA), and in particular, methods of detecting *S*. *aureus* harboring a *mecA* homolog gene, such as a CC130 or ccl30 *S. aureus* strain. Methicillin resistance in *Staphylococcus aureus* is due to the gene product of the *mecA* gene, encoding for the penicillin binding protein 2a (PBP-2a), a β-lactam-resistant transpeptidase. *mecA* is absent from methicillin-sensitive *S. aureus,* but is widely distributed among other species of staphylococci, including coagulase negative staphylococci, (CoNS), such as *Staphylococcus epidermidis, Staphylococcus haemolyticus, Staphylococcus capitis, S. saprophyticus S.lentus, S. hominus, S*. *cohnii, S. delphini, S. xylosus, S. muscae, S. schleiferi, S. coagulans,* and others. The *mecA* gene is highly conserved (Ubukata et al., 1990, Antimicrob. Agents Chemother. 34:170-172). In methicillin-resistant staphylococci, *mecA* is present in a genetic element termed staphylococcal cassette chromosome mec (*SCCmec*), which is inserted into the chromosome of staphylococci.

*SCCmec* cassettes range from 20kb to more than 60kb in length, and include site specific recombinase genes and transposable elements, in addition to the *mecA* gene. *SCCmec* cassettes are inserted at a fixed location, termed "*attBscc*" within the chromosomal DNA of *Staphylococcus aureus,* and which is located at the 3' end of an open reading frame termed "*orfX.*" Huletsky et al. (2004) J. Clin. Microbiol. 42(5):1875-1884. MRSA strains have been classified based upon the organization of the *SCCmec* cassettes (termed "*SCCmec* typing). Different SCCmecs have been classified according to their recombinase genes, and the genetic organization of *mecI* and *mecR* genes, which are regulators of *mecA.*

Many molecular assays for the detection and identification of MRSA involve the detection of the *mecA* gene. Since *mecA* is also found in CoNS, however, detection of *mecA* alone is not sufficient to determine the presence of MRSA, as methicillin-resistant CoNS will also test positive. In order to address this problem, assays have been developed in which *S*. *aureus-specific* genes are detected, in addition to *mecA. See,* Schuenck et al., Res. Microbiol., (2006), in press, Shittu et al., (2006), Diagn Microbiol Infect Dis. Jul 17, Grisold et al., (2006), Methods Mol. Biol.345 : 79-89, Costa et al., (2005), Diag. Microbiol. and Infect. Dis, 51: 13-17, Mc Donald et al., (2005), J. Clin. Microbiol., 43: 6147-6149, Zhang et al., (2005), J. Clin. Microbiol. 43: 5026-5033, Hagen et al. (2005), Int J Med Microbiol. 295:77-86, Maes, et al. (2002) J. Clin. Microbiol. 40:1514-1517, Saito et al., (1995) J. Clin. Microbiol. 33:2498-2500; Ubukata et al., (1992) J. Clin. Microbiol. 30:1728-1733; Murakami et al., (1991) J. Clin. Microbiol. 29:2240-2244; Hiramatsu et al., (1992) Microbiol. Immunol. 36:445-453). Furthermore, Levi and Towner (2003), J. Clin. Microbiol., 41:3890-3892 and Poulsen et al. (2003), J Antimicrob Chemother., 51:419-421 describe detection of methicillin resistance in coagulase-negative Staphylococci and in *S*. *aureus* using the EVIGENE™ MRSA Detection kit.

However, because the *mecA* gene is widely distributed in both *S. aureus* and coagulase-negative staphylococci, each of the methods described above are incapable of discriminating between samples containing both methicillin-sensitive *S. aureus* ("MSSA") and methicillin-resistant coagulase-negative staphylococci, and samples that contain only MRSA or that have both methicillin-sensitive *S. aureus* and MRSA.

To address this problem, Hiramatsu et al. developed a PCR-based assay specific for MRSA that utilizes primers that hybridize to the right extremities of DNA of *SCCmec* types I-III in combination with primers specific to the *S. aureus* chromosome, which corresponds to the nucleotide sequence on the right side of the *SCCmec* integration site. (US patent 6,156,507, hereinafter the '''507 patent"). More recently, Zhang et al., (2005), J. Clin. Microbiol. 43: 5026-5033, described a multiplex assay for subtyping SCCmec types I to V MRSA. Nucleotide sequences surrounding the *SCCmec* integration site in other staphylococcal species (e.g., *S. epidermidis* and *S. haemolyticus*) are different from those found in *S. aureus,* therefore multiplex PCR assays that utilize oligonucleotides that hybridize to the right extremities of *SCCmec* and the *S. aureus* chromosome have the advantage of being specific for the detection of MRSA.

The PCR assay described in the '507 patent also led to the development of "MREP typing" (*mec* right extremity polymorphism) of *SCCmec* DNA (Ito et al., (2001) Antimicrob. Agents Chemother. 45:1323-1336; Hiramatsu et al., (1996) J. Infect. Chemother. 2:117-129). The MREP typing method takes advantage of the fact that the nucleotide sequences of the three MREP types differ at the right extremity of *SCCmec* DNAs adjacent to the integration site among the three types of *SCCmec.*

The term "MREJ" refers to the mec right extremity junction <<mec right extremity junction>>. MREJ region nucleic acid sequences are approximately 1 kilobase (kb) in length and include sequences from the *SCCmec* right extremity as well as bacterial chromosomal DNA to the right of the *SCCmec* integration site. Strains that were classified as MREP types i-iii correspond to MREJ types i-iii. MREJ types iv to xx have been previously characterized Huletsky et al., (2004), J. Clin. Microbiol. 42:1875-1884; International Patent Application PCT/CA02/00824, United States Patent Application 2008/0227087.

Recently, Garcia-Alvarez et al. reported on bacterial strains that were confirmed by ribosomal RNA analysis to be *S. aureus,* and which exhibited resistance to methicillin using routine antibiotic susceptibility testing. Garcia-Alvarez et al. (2011) Lancet 11:595-603. Surprisingly, however, these strains tested negative as MRSA using the assays described above, which rely upon the detection of *mecA* or the detection of known MREJ regions. Assay specificity is limited to identify MREJ regions. Garcia-Alvarez et al. demonstrated that the strains harbored a novel *mecA* homolog that shared limited homology with known *mecA* genes, *i.e.,* 63% identity at the amino acid level and 70% identity at the DNA level, explaining the inability of the assays relying upon the detection of the *mecA* gene to detect these MRSA. The *mecA* homolog was termed *mecA*_{LGA251}*,* also known herein as *mecC.* As the assays utilizing MREJ amplification were also unable to detect any of the *mecA*_{LGA251} MRSA strains, *mecA*_{LGA251} MRSA strains would be incorrectly identified as false negative. This error in diagnosing and identifying MRSA could have serious impact on the patient health outcome.

Several strains of *S. aureus* that tested positive as methicillin resistant using standard antibiotic susceptibility testing, yet that were not detected as MRSA using conventional, commercial molecular assays were analyzed further. The strains are listed in Table 1, below.

**TABLE 1**

| *Staphylococcus aureus* strain designation | Country of Origin |
|---|---|
| IDI-6112 | France |
| IDI-6113 | France |
| IDI-6121 | United Kingdom |
| IDI-6122 | United Kingdom |
| IDI-6123 | United Kingdom |
| IDI-6125 | United Kingdom |
| IDI-6126 | United Kingdom |
| IDI-6127 | United Kingdom |
| IDI-6128 | United Kingdom |
| IDI-6129 | United Kingdom |
| IDI-6130 | United Kingdom |
| IDI-6131 | United Kingdom |
| IDI-6132 | United Kingdom |
| IDI-6133 | United Kingdom |
| IDI-6134 | United Kingdom |
| IDI-6135 | United Kingdom |
| IDI-6136 | United Kingdom |
| IDI-6137 | United Kingdom |
| IDI-6138 | United Kingdom |
| IDI-6139 | United Kingdom |
| IDI-6140 | United Kingdom |
| IDI-6141 | United Kingdom |
| IDI-6142 | United Kingdom |
| IDI-6143 | United Kingdom |
| IDI-6144 | United Kingdom |
| IDI-6145 | United Kingdom |
| IDI-6146 | United Kingdom |
| IDI-6147 | Denmark |
| IDI-6148 | Denmark |
| IDI-6149 | Denmark |
| IDI-6150 | Denmark |
| IDI-6151 | Denmark |
| IDI-6152 | Denmark |
| IDI-6153 | Denmark |
| IDI-6154 | Denmark |
| IDI-6155 | Denmark |
| IDI-6156 | Denmark |
| IDI-6157 | Denmark |
| IDI-6158 | Denmark |
| IDI-6159 | Denmark |
| IDI-6160 | Denmark |
| IDI-6161 | Denmark |
| IDI-6162 | Denmark |
| IDI-6163 | Denmark |
| IDI-6164 | Denmark |
| IDI-6165 | Denmark |
| IDI-6166 | Denmark |
| IDI-6167 | Denmark |
| IDI-6168 | France |
| IDI-6170 | France |
| IDI-6171 | France |
| IDI-6208 | Germany |
| IDI-6209 | Germany |
| IDI-6211 | Germany |
| IDI-6213 | Germany |
| IDI-6214 | Germany |
| IDI-6215 | Germany |
| IDI-6216 | Germany |
| IDI-6217 | Germany |
| IDI-6218 | Germany |
| IDI-6219 | Germany |
| IDI-6220 | Germany |
| IDI-6221 | Germany |

As described herein, the present disclosure is based, in part, upon the surprising finding that the vast majority of MRSA strains analyzed that harbor the *mecA* homolog gene, *mecA*_{LGA251}/*mecC,* share the same MREJ sequence. Based upon this surprising finding, compositions and methods for the improved detection of MRSA are provided herein. The compositions and methods disclosed herein advantageously allow for reliable and rapid detection and identification of *mecA*_{LGA251} MRSA strains.

### Oligonucleotides

According to some embodiments disclosed herein, oligonucleotides are provided, for example amplification primers and/or sequence-specific probes. As used herein, the terms "primer" and "probe" include, but are not limited to oligonucleotides. Preferably, the oligonucleotide primers and/or probes disclosed herein can be between 8 and 45 nucleotides in length. For example, the primers and or probes can be at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or more nucleotides in length. Primers and/or probes can be provided in any suitable form, included bound to a solid support, liquid, and lyophilized, for example. The primer and probe sequences disclosed herein can be modified to contain additional nucleotides at the 5' or the 3' terminus, or both. The skilled artisan will appreciate, however, that additional bases to the 3' terminus of amplification primers (not necessarily probes) are generally complementary to the template sequence. The primer and probe sequences disclosed herein can also be modified to remove nucleotides at the 5' or the 3' terminus. The skilled artisan will appreciate that in order to function for amplification, the primers or probes will be of a minimum length and annealing temperature as disclosed herein.

Oligonucleotide primers and probes can bind to their targets at an annealing temperature, which is a temperature less than the melting temperature (Tₘ). As used herein, "Tₘ" and "melting temperature" are interchangeable terms which refer to the temperature at which 50% of a population of double-stranded polynucleotide molecules becomes dissociated into single strands. Formulae for calculating the Tₘ of polynucleotides are well known in the art. For example, the Tₘ may be calculated by the following equation: Tₘ =69.3+0.41 x.(G+C)%-6-50/L, wherein L is the length of the probe in nucleotides. The Tₘ of a hybrid polynucleotide may also be estimated using a formula adopted from hybridization assays in 1 M salt, and commonly used for calculating Tₘ for PCR primers: [(number of A+T) x 2°C +(number of G+C) x 4°C]. *See, e.g.,* C. R. Newton et al. PCR, 2nd ed., Springer-Verlag (New York: 1997), p. 24. Other more sophisticated computations exist in the art, which take structural as well as sequence characteristics into account for the calculation of Tₘ. The melting temperature of an oligonucleotide can depend on complementarity between the oligonucleotide primer or probe and the binding sequence, and on salt conditions. In some embodiments described herein, an oligonucleotide primer or probe provided herein has a Tₘ of less than about 90°C in 50mM KCl, 10 mM Tris-HCl buffer, for example about 89°C, 88, 87, 86, 85, 84, 83, 82, 81, 80 79, 78, 77, 76, 75, 74, 73, 72, 71, 70, 69, 68, 67, 66, 65, 64, 63, 62, 61, 60, 59, 58, 57, 56, 55, 54, 53, 52, 50, 49, 48, 47, 46, 45, 44, 43, 42, 41, 40, 39°C, or less, including ranges between any two of the listed values.

As discussed in further detail below, in some embodiments described herein, the primers disclosed herein, *e.g*., amplification primers, can be provided as an amplification primer pair, *e.g*., comprising a forward primer and a reverse primer (first amplification primer and second amplification primer). Preferably, the forward and reverse primers have Tₘ's that do not differ by more than 10°C, *e.g.,* that differ by less than 10°C, less than 9°C, less than 8°C, less than 7°C, less than 6°C, less than 5°C, less than 4°C, less than 3°C, less than 2°C, or less than 1°C.

The primer and probe sequences may be modified by having nucleotide substitutions (relative to the target sequence) within the oligonucleotide sequence, provided that the oligonucleotide contains enough complementarity to hybridize specifically to the target nucleic acid sequence. In this manner, at least 1, 2, 3, 4, or up to about 5 nucleotides can be substituted. As used herein, the term "complementary" refers to sequence complementarity between regions of two polynucleotide strands or between two regions of the same polynucleotide strand. A first region of a polynucleotide is complementary to a second region of the same or a different polynucleotide if, when the two regions are arranged in an antiparallel fashion, at least one nucleotide of the first region is capable of base pairing with a base of the second region. Therefore, it is not required for two complementary polynucleotides to base pair at every nucleotide position. "Fully complementary" refers to a first polynucleotide that is 100% or "fully" complementary to a second polynucleotide and thus forms a base pair at every nucleotide position. "Partially complementary" also refers to a first polynucleotide that is not 100% complementary (e.g., 90%, or 80% or 70% complementary) and contains mismatched nucleotides at one or more nucleotide positions. In some embodiments described herein, an oligonucleotide includes a universal base.

As used herein, the term "hybridization" is used in reference to the pairing of complementary (including partially complementary) polynucleotide strands. Hybridization and the strength of hybridization (i.e., the strength of the association between polynucleotide strands) is impacted by many factors well known in the art including the degree of complementarity between the polynucleotides, stringency of the conditions involved affected by such conditions as the concentration of salts, the melting temperature of the formed hybrid, the presence of other components (e.g., the presence or absence of polyethylene glycol), the molarity of the hybridizing strands and the G:C content of the polynucleotide strands. In some embodiments described herein, e.g., embodiments described herein providing more than one oligonucleotide, the oligonucleotides are designed such that the Tₘ of one oligonucleotide is within 2°C of the Tₘ of the other oligonucleotide. An extensive guide to the hybridization of nucleic acids is found in Tijssen (1993) Laboratory Techniques in Biochemistry and Molecular Biology - Hybridization with Nucleic Acid Probes, Part I, Chapter 2 (Elsevier, New York); and Ausubel et al, eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Plainview, New York). As discussed further herein, the term "specific hybridization" or "specifically hybridizes" refers to the hybridization of a polynucleotide, e.g., an oligonucleotide primer or probe or the like to a target sequence, such as a sequence to be quantified in a sample, a positive control target nucleic acid sequence, or the like, and not to unrelated sequences, under conditions typically used for nucleic acid amplification.

In some embodiments described herein, the primers and/or probes include oligonucleotides that hybridize to a target nucleic acid sequence over the entire length of the oligonucleotide sequence. Such sequences can be referred to as "fully complementary" with respect to each other. Where an oligonucleotide is referred to as "substantially complementary" with respect to a nucleic acid sequence herein, the two sequences can be fully complementary, or they may form mismatches upon hybridization, but retain the ability to hybridize under stringent conditions or standard nucleic acid amplification conditions as discussed below. As used herein, the term "substantially complementary" refers to the complementarity between two nucleic acids, e.g., the complementary region of the oligonucleotide and the target sequence. The complementarity need not be perfect; there may be any number of base pair mismatches that between the two nucleic acids. However, if the number of mismatches is so great that no hybridization can occur under even the least stringent of hybridization conditions, the sequence is not a substantially complementary sequence. When two sequences are referred to as "substantially complementary" herein, it is meant that the sequences are sufficiently complementary to the each other to hybridize under the selected reaction conditions. The relationship of nucleic acid complementarity and stringency of hybridization sufficient to achieve specificity is well known in the art and described further below in reference to sequence identity, melting temperature and hybridization conditions. Therefore, substantially complementary sequences can be used in any of the detection methods disclosed herein. Such probes can be, for example, perfectly complementary or can contain from 1 to many mismatches so long as the hybridization conditions are sufficient to allow, for example discrimination between a target sequence and a non-target sequence. Accordingly, substantially complementary sequences can refer to sequences ranging in percent identity from 100, 99, 98, 97, 96, 95, 94, 93, 92, 91, 90, 89, 88, 87, 86, 85, 84, 83, 82, 81, 80, 75, 70 or less, or any number in between, compared to the reference sequence. For example, the oligonucleotides disclosed herein can contain 1, 2, 3, 4, 5, or more mismatches and/or degenerate bases, as compared to the target sequence to which the oligonucleotide hybridizes, with the proviso that the oligonucleotides are capable of specifically hybridizing to the target sequence under, for example, standard nucleic acid amplification conditions.

Accordingly, by way of example, the term "stringent hybridization conditions" can refer to either or both of the following: a) 6 x SSC at about 45°C, followed by one or more washes in 0.2 x SSC, 0.1% SDS at 65°C, and b) 400 mM NaCl, 40 mM PIPES pH 6.4, 1 mM EDTA, 50°C or 70°C for 12-16 hours, followed by washing. In some embodiments described herein, the term "stringent conditions" can refer to standard nucleic acid amplification (*e.g.,* PCR) conditions.

In some embodiments described herein, the sample or specimen is contacted with a set of amplification primers under standard nucleic acid amplification conditions, which are discussed in further detail below. For a review of PCR technology, including standard nucleic acid amplification conditions such as PCR conditions, applied to clinical microbiology, see DNA Methods in Clinical Microbiology, Singleton P., published by Dordrecht; Boston: Kluwer Academic, (2000) Molecular Cloning to Genetic Engineering White, B.A. Ed. in Methods in Molecular Biology 67: Humana Press, Totowa (1997) and "PCR Methods and Applications", from 1991 to 1995 (Cold Spring Harbor Laboratory Press). Non-limiting examples of "nucleic acid amplification conditions" and "PCR conditions" include the conditions disclosed in the references cited herein, such as, for example, 50 mM KCl, 10 mM Tris-HCl (pH 9.0), 0.1% Triton X-100, 2.5 mM MgCl₂, with an annealing temperature of 72°C; or 4mM MgCl₂, 100mM Tris, pH 8.3, 10mM KCl, 5mM (NH₄)₂SO₄, 0.15mg BSA, 4% Trehalose, with an annealing temperature of 59°C, or 50 mM KCl, 10 mM Tris-HCl (pH 9.0), 0.1% Triton X-100, 2.5 mM MgCl₂, with an annealing temperature of 55°C, or the like.

The primers described herein can be prepared using techniques known in the art, including, but not limited to, cloning and digestion of the appropriate sequences and direct chemical synthesis. Chemical synthesis methods that can be used to make the primers of the described herein, include, but are not limited to, the phosphotriester method described by Narang et al. (1979) Methods in Enzymology 68:90, the phosphodiester method disclosed by Brown et al. (1979) Methods in Enzymology 68:109, the diethylphosphoramidate method disclosed by Beaucage et al. (1981) Tetrahedron Letters 22:1859, and the solid support method described in U.S. Patent No. 4,458,066. The use of an automated oligonucleotide synthesizer to prepare synthetic oligonucleotide primers described herein is also contemplated herein.

Accordingly, some embodiments described herein relate to compositions that comprise oligonucleotides (*e.g*., an amplification primers and probes) that specifically hybridize (*e.g.,* under standard nucleic acid amplification conditions, *e.g.,* standard PCR conditions, and/or stringent hybridization conditions) to the polymorphic *SCCmec* right extremity sequences in MRSA strains that have MREJ type xxi sequences. For example, in some embodiments described herein, provided are compositions that comprise oligonucleotides that specifically hybridize to the polymorphic *SCCmec* right extremity sequences present in SEQ ID NO: 1, or the complement thereof *e.g*., within nucleotide positions 1 to 164 of SEQ ID NO:1, or its complement. An exemplary oligonucleotide that specifically hybridizes to the polymorphic *SCCmec* right extremity sequences of MREJ type xxi, including the polymorphic right extremity sequences within SEQ ID NO: 1, is provided in SEQ ID NO:2, or the complement thereof. Thus, provided herein are oligonucleotides that are substantially complementary to SEQ ID NO:2 or the complement thereof, as well as oligonucleotides containing 1, 2, 3, 4 or more mismatches or universal nucleotides relative to SEQ ID NO:2 or the complement thereof, *e.g*., including oligonucleotides that are at least 80% identical (for example at least 81%, 82%, 83%, 84%, 85%, 86%, 87%, 88%, 89%, 90%, 91%, 92%, 93%, 94%, 95%, 96%, 97%, 98%, 99% or 100% identical to SEQ ID NO: 2 or the complement thereof.

In some embodiments described herein, the compositions and methods can include oligonucleotides, e.g., amplification primers or sequence-specific probes, that specifically hybridize to one or more polymorphic right extremity sequences within MREJ regions other than MREJ type xxi. Accordingly, some embodiments described herein provide oligonucleotides, *e.g*., amplification primers or sequence-specific probes that specifically hybridize (under standard nucleic acid amplification conditions, and/or stringent hybridization conditions) to polymorphic right extremity sequences within one or more MREJ regions selected from MREJ type i regions, MREJ type ii regions, MREJ type iii regions, MREJ type iv regions, MREJ type v regions, MREJ type vi regions, MREJ type vii regions, MREJ type viii regions, MREJ type ix regions, MREJ type x regions, MREJ type xi regions, MREJ type xii regions, MREJ type xiii regions, MREJ type xiv regions, MREJ type xv regions, MREJ type xvi regions, MREJ type xvii regions, MREJ type xviii regions, MREJ type xix regions, and MREJ type xx regions.

In some embodiments described herein, the compositions and methods can include oligonucleotides, *e.g*., amplification primers that specifically hybridize to, and are capable of generating an amplicon of, *mecA* sequences, or a fragment thereof. Accordingly, some embodiments described herein include oligonucleotides, *e.g*., amplification primers that specifically hybridize to and are capable of generating an amplicon of sequences within SEQ ID NO:156. Some embodiments described herein include oligonucleotides, *e.g*., amplification primers that specifically hybridize to, and are capable of generating an amplicon of, *mecC* sequences, *e.g*., sequences within SEQ ID NO:157, or a fragment thereof. Some embodiments described herein include oligonucleotides, e.g., amplification primers that specifically hybridize to and are capable of generating an amplicon of *Staphylococcus aureus*-specific sequences. For example, some embodiments described herein provide oligonucleotides that specifically hybridize to and are capable of generating an amplicon of *nuc* sequences (*e.g.,* sequences derived from SEQ ID NO:158), *femB* sequences (*e.g.,* sequences derived from SEQ ID NO: 159), Sa442 sequences (*e.g*., from Martineau,et al. 1998, J. Clin. Microbiol. 36(3):618-623) (SEQ ID NO:160).

In some embodiments described herein, provided is an oligonucleotide that specifically hybridizes to the polymorphic right extremity sequences of an MREJ type xxi region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type i region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type ii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type iii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type iv region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type v region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type vi region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type vii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type viii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type ix region under standard conditions for nucleic acid amplification, and/or stringent hybridization conditions. In some embodiments described herein, the sequence specific oligonucleotides (*e.g.* amplification primers and sequence specific probes) disclosed herein specifically hybridize to the polymorphic right extremity sequences of an MREJ type x region under standard conditions for nucleic acid amplification, and/or stringent hybridization conditions. In some embodiments described herein, the sequence specific oligonucleotides (*e.g.* amplification primers and sequence specific probes) disclosed herein specifically hybridize to the polymorphic right extremity sequences of an MREJ type xi region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xiii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xiv region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xv region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xvi region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xvii region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xviii region under standard conditions for nucleic acid amplification, and/or stringent hybridization conditions. In some embodiments described herein, the sequence specific oligonucleotides (*e.g.* amplification primers and sequence specific probes) disclosed herein specifically hybridize to the polymorphic right extremity sequences of an MREJ type xix region under standard conditions for nucleic acid amplification, and/or stringent hybridization conditions. In some embodiments described herein, the sequence specific oligonucleotides (*e.g*. amplification primers and sequence specific probes) disclosed herein specifically hybridize to the polymorphic right extremity sequences of an MREJ type xx region under standard conditions for nucleic acid amplification, and/or stringent hybridization conditions. In some embodiments described herein, the sequence specific oligonucleotides (*e.g*., amplification primers and/or sequence specific probes) disclosed herein specifically hybridize to *mecA* sequences. In some embodiments described herein, the sequence specific oligonucleotides (*e.g*., amplification primers and/or sequence specific probes) disclosed herein specifically hybridize to *mecC* sequences. In some embodiments described herein, the sequence specific oligonucleotides (*e.g*., amplification primers and/or sequence specific probes) disclosed herein specifically hybridize to *nuc* sequences. In some embodiments described herein, the sequence specific oligonucleotides (*e.g*., amplification primers and/or sequence specific probes) disclosed herein specifically hybridize to *Sa442* sequences. In some embodiments described herein, the sequence specific oligonucleotides (*e.g*., amplification primers and/or sequence specific probes) disclosed herein specifically hybridize to *femB* sequences.

Exemplary MREJ region sequences related to the embodiments disclosed herein include, for example:

| MREJ type | Exemplified in SEQ ID NO(s): |
|---|---|
| i | 5 |
| ii | 6 |
| iii | 7 |
| iv | 8 |
| v | 9 |
| vi | 10 |
| vii | 11 |
| viii | 12 |
| ix | 13 |
| x | 14 |
| xi | 15, 16 and 17 |
| xii | 18 |
| xiii | 19, 20, 21 |
| xiv | 22 |
| xv | 23 |
| xvi | 24 |
| xvii | 25 |
| xviii | 26 and 27 |
| xix | 28 |
| xx | 29 |
| xxi | 1 |

In addition to the oligonucleotide of SEQ ID NO:2, discussed above, which specifically hybridizes to the polymorphic right extremity sequences within MREJ type xxi regions, oligonucleotides that are specific for one or more polymorphic sequences within MREJ region sequences, or for *S. aureus* chromosomal DNA sequences, useful in the embodiments disclosed herein include, but are not limited to the following:

| Specific for: | SEQ ID NO |
|---|---|
| MREJ type xi primer | 30 |
| MREJ type xi primer | 31 |
| MREJ type xii primer | 32 |
| MREJ type xii primer | 33 |
| MREJ type ix, xiii, xiv primer | 34 |
| MREJ type xv primer | 35 |
| MREJ type xv primer | 36 |
| MREJ type xv primer | 37 |
| MREJ type xv primer | 38 |
| MREJ type i, ii and xvi primer | 39 |
| MREJ type xvii primer | 40 |
| MREJ type xvii primer | 41 |
| MREJ type xvii primer | 42 |
| MREJ type xviii primer | 43 |
| MREJ type xix primer | 44 |
| MREJ type xx primer | 45 |
| orfX | 46 |
| orfX r | 47 |
| orfX | 49 |
| orfX | 50 |
| orfX | 51 |
| orfX | 52 |
| orfX | 53 |
| orfX | 54 |
| orfX | 55 |
| orfX | 56 |
| orfX | 57 |
| orfX | 58 |
| orfX | 59 |
| orfX | 60 |
| orfX | 61 |
| orfX | 62 |
| orfX | 63 |
| orfX | 64 |
| MREJ types i and ii | 65 |
| MREJ types i and ii | 66 |
| MREJ types i and ii | 67 |
| MREJ type ii | 68 |
| MREJ type ii | 69 |
| MREJ type iii | 70 |
| MREJ type iii | 71 |
| MREJ type iii | 72 |
| MREJ type iv | 73 |
| MREJ type v | 74 |
| MREJ type vi | 75 |
| MREJ type vi | 76 |
| MREJ type vii | 77 |
| MREJ type vii | 78 |
| MREJ type viii | 79 |
| MREJ type viii | 80 |
| MREJ type ix | 81 |
| MREJ type x | 83 |
| nuc | 161 |
| nuc | 162 |
| nuc | 163 |
| nuc | 164 |
| nuc | 165 |
| nuc | 166 |
| nuc | 167 |
| nuc | 168 |
| nuc | 169 |
| nuc | 170 |
| nuc | 171 |
| nuc | 172 |
| Sa442 | 173 |
| Sa442 | 174 |
| femB | 175 |
| femB | 176 |
| mecA | 177 |
| mecA | 178 |
| mecA | 179 |
| mecC | 180 |
| mecC | 181 |
| mecC | 182 |
| mecC | 183 |
| mecA | 184 |
| mecA | 185 |
| mecA | 186 |
| mecC | 187 |

In addition to the foregoing, the skilled artisan will appreciate that the compositions and methods disclosed herein can include primers and/or probes for the specific detection of the right extremity region of the *SCCmec* sequences in various MRSA strains in addition to those mentioned herein above. By way of example, in some embodiments described herein, the compositions and methods disclosed herein include sequences, e.g., primers and/or probes, described in International Patent Application Publication No. WO 08/080620, including variants thereof, and complements thereof. Accordingly, the compositions and methods disclosed herein can include primers and/or probes listed below:

**TABLE 2**

| SEQ ID NO: | Sequence (5'-3') |
|---|---|
| 84 | GCA ATT CAC ATA AAC CTC ATA TGT TC |
| 85 | ACC TCA TAT GTT CTG ATA CAT TCA |
| 86 | GCA ATT CAC ATA AAC CTC ATA T |
| 87 | CAT AAC AGC AAT TCA CAT AAA CCT C |
| 88 | TAA CAG CAA TTC ACA TAA ACC T |
| 89 | CGC TAT TAT TTA CTT GAA ATG AAA GAC |
| 90 | CTT GAA ATG AAA GAC TGC GGA |
| 91 | TTG CTT CAC TAT AAG TAT TCA GTA TAA AGA ATT TAC TTG AAA TGA AAG ACT GCG |
| 92 | ATT TAC TTG AAA TGA AAG ACT GCG |
| 93 | AAA GAA TAT TTC GCT ATT ATT TAC TTG AA |
| 94 | TCA GTA TAA AGA ATA TTT CGC TAT TAT TT |
| 95 | TGA AAT GAA AGA CTG CGG AG |
| 96 | AAC CTC ATA TGT TCT GAT ACA TTC AAA |
| 97 | TAT GTC AAA AAT CAT GAA CCT CAT TAC T |
| 98 | CAT AAC AGC AAT TCA CAT AAA CCT C |
| 99 | GAC TGC GGA GGC TAA CT |
| 100 | ATC CCT TTA TGA AGC GGC |
| 101 | TGA AAT GAA AGA CTG CGG AG |
| 102 | GCA AGG TAT AAT CCA ATA TTT CAT ATA TGT |
| 103 | AGT TCC ATA ATC AAT ATA ATT TGT ACA GT |
| 104 | ACA TCG TAT GAT ATT GCA AGG TA |
| 105 | CTT TCA TTC TTT CTT GAT TCC ATT AG |
| 106 | CAC TCT ATA AAC ATC GTA TGA TAT TGC |
| 107 | TTC TTA ATT TAA TTG TAG TTC CAT AAT CAA |
| 108 | AAT TAT ACA CAA CCT AAT TTT TAG TTT TAT |
| 109 | AAT TTT TAG TTT TAT TTA TGA TAC GCT TC |
| 110 | ACA CAA CCT AAT TTT TAG TTT TAT TTA TGA |
| 111 | TTT ATT AAA CAC TCT ATA AAC ATC GTA TGA |
| 112 | TCA CAT CTC ATT AAA TTT TTA AAT TAT ACA C |
| 113 | CCA CAT CTC ATT AAA TTT TTA AAT TAT ACA C |
| 114 | ATA TTA TAC ACA ATC CGT TTT TTA GTT TTA |
| 115 | ACA CAA TCC GTT TTT TAG TTT TAT TTA TG |
| 116 | TTC TAA TTT ATT TAA CAT AAA ATC AAT CCT |
| 117 | CAA TCC TTT TTA TAT TTA AAA TAT ATT ATA CAC |
| 118 | AAG TCG CTT TGC CTT TGG GTC A |
| 119 | TAC AAA GTC GCT TTG CCT TTG GGT CA |
| 120 | GGC CGT TTG ATC CGC CAA T |
| 121 | AAG TCG CTT TGC CCT TGG GTA |
| 122 | AAG TCG CTT TGC CCT TGG GT |
| 123 | AAG TCG CTT TGC CCT TGG GTC A |
| 124 | AAG TCG CTT TGC CCT TGG G |
| 125 | CAA GAA TTG AAC CAA CGC AT |
| 126 | CAA TGA CGA ATA CAT AGT CGC TTT GCC CTT |
| 127 | CGT TTG ATC CGC CAA TGA CGA |
| 128 | GCC AAT CCT TCG GAA GAT AGC A |
| 129 | ATT AAC ACA ACC CGC ATC |
| 130 | GTC GCT TTG CCC TTG GGT C |
| 131 | TCG CTT TGC CCT TGG GTC AT |
| 132 | GGC CGT TTG ATC CGC CAA T |
| 133 | GTC CTT GTG CAG GCC GTT TGA T |
| 134 | CTT GGG TCA TGC GTT GGT TCA ATT |
| 135 | CGA ATA CAA AGT CGC TTT GCC CTT GGG |
| 136 | ATG CGT TGG TTC AAT TCT TG |
| 137 | GCG TTG GTT CAA TTC TTG GG |
| 138 | ACC CAA GGG CAA AGC GAC TT |
| 139 | GGT AAT GCG TTG GTT CAA TTC TTG |
| 140 | ACA AAG TCG CTA TGC CCT TGG GTC A |
| 141 | CTT TCC TTG TAT TTC TAA TGT AAT GAC TG |
| 142 | TTG ATG TGG GAA TGT CAT TTT GCT GAA |
| 143 | GCG TTG GTT CAA TTC TTG GGC CAA T |
| 144 | GTT GGT TCA ATT CTT GGG CCA ATC CTT CG |
| 145 | CGA ATA CAA AGT CGC TTT GCC CTT GG |
| 146 | GCC AAT GAC GAA TAC AAA GTC GCT TTG CC |
| 147 | TGG GCC AAT CCT TCG GAA GAT AGC A |
| 148 | ATG CGT TGG TTC GAT TCT TG |
| 149 | CAT GCG TTG GTT CGA TTC TTG |
| 150 | AAG TCG CTT TGC CCT TGG G |
| 151 | CAT GCG TTG GTT CGA TTC TTG |
| 152 | AAG TCG CTT TGC CCT TGG GTC AT |
| 153 | TGC TCA ATT AAC ACA ACC CGC ATC A |
| 154 | GCC GCG CTG CTC AAT TAA CAC AAC CCG CGC GGC |
| 155 | GCC GCG CAT GCG TTG GTT CAA TTC TGC GCG GC |

Accordingly, in exemplary embodiments described herein, provided are methods and compositions for the detection of MRSA comprising MREJ xxi, and one or more MREJ types selected from the group consisting of MREJ type i-xx. For example, some embodiments described herein provide for the detection and/or identification of MRSA comprising type i, ii, iii and xxi MREJ nucleic acids, using the MREJ-specific and *S. aureus* chromosomal DNA-specific oligonucleotides disclosed herein. Some embodiments described herein provide for the detection and/or identification of MRSA comprising type i, ii, iii, iv and xxi MREJ sequences, using a combination of MREJ-specific oligonucleotides as described herein. Some embodiments described herein provide for the detection of MRSA comprising type i, ii, iii, iv, v, vii, and xxi MREJ nucleic acids, using a combination of MREJ-specific oligonucleotides disclosed herein. Some embodiments described herein provide for the identification of MRSA comprising type i, ii, iii, iv, v, vii, and xxi MREJ nucleic acids, using a combination of MREJ-specific oligonucleotides disclosed herein. Some embodiments described herein provide for the detection of MRSA comprising type i, ii, iii, iv, v, vii, and xxi MREJ nucleic acids, using a combination of MREJ-specific oligonucleotides disclosed herein. Some embodiments described herein provide for the identification of MRSA comprising type i, ii, iii, iv, v, vii, and xxi MREJ nucleic acids, using a combination of MREJ-specific oligonucleotides disclosed herein. Some embodiments described herein provide for the detection of MRSA comprising type i, ii, iii, iv, v, vi, vii, ix, xiii, xiv, and xxi nucleic acids, using a combination of MREJ-specific oligonucleotides disclosed herein. Some embodiments described herein provide for the identification of MRSA comprising type i, ii, iii, iv, v, vi, vii, ix, xiii, xiv, and xxi nucleic acids, using a combination of MREJ-specific oligonucleotides disclosed herein. Some embodiments described herein provide for the detection and/or identification of MRSA comprising type i, ii, iii, iv, vii, xvi, and xxi nucleic acids, using a combination of MREJ-specific disclosed herein.

### Probes

In some embodiments described herein, sequence-specific probes are provided. Probes include, but are not limited to oligonucleotides as described herein. In some embodiments described herein, sequence-specific probes disclosed herein specifically hybridize to a target sequence, such as an MREJ type xxi region nucleic acid sequence. For example, in some embodiments described herein, sequence specific probes disclosed herein specifically hybridize to SEQ ID NO:1, or the complement thereof, or a subsequence thereof (e.g., an amplicon of a region within SEQ ID NO:1). In some embodiments described herein, the sequence-specific probe specifically hybridizes to, and is fully or substantially complementary a nucleotide sequence flanked by the binding sites of a forward primer and reverse primer disclosed herein. In some embodiments described herein, the sequence specific probes comprise at least 5, 6, 7, 8, 9, 10, 11, 12, 13, 14, 15, 16, 17, 18, 19, 20, 21, 22, 23, 24, or 25 nucleotides of SEQ ID NO: 2 or 3, such that the sequence specific probe overlaps with the binding site of an amplification primer disclosed herein.

In some embodiments described herein, sequence-specific probes that hybridize to *orfX* are provided. In some embodiments described herein, sequence specific probes that hybridize to *mecA* are provided. In some embodiments described herein, sequence specific probes that hybridize to *mecC* are provided. In some embodiments described herein, sequence specific probes that hybridize to *nuc* are provided. In some embodiments described herein, sequence specific probes that hybridize to *femB* are provided. In some embodiments described herein, sequence specific probes that hybridize to *Sa442* are provided.

The skilled artisan will readily appreciate that cognate pairs of amplification primers and sequence-specific probes can be provided together. That is, in embodiments described herein where amplification primers that specifically hybridize to and generate an amplicon for a particular sequence are provided, embodiments disclosed herein can also include a sequence-specific probe that hybridizes to and is specific for the amplicon.

In some embodiments described herein, the sequence specific probes disclosed herein specifically hybridize to the polymorphic right extremity sequences of an MREJ type xxi region under standard conditions for nucleic acid amplification, and/or stringent hybridization conditions. In some embodiments described herein, the sequence specific probes disclosed herein specifically hybridize to the polymorphic right extremity sequences of an MREJ type i region under standard conditions for nucleic acid amplification, and/or stringent hybridization conditions. In some embodiments described herein, the sequence specific probes disclosed herein specifically hybridize to the polymorphic right extremity sequences of an MREJ type ii region under standard conditions for nucleic acid amplification, and/or stringent hybridization conditions. In some embodiments described herein, the sequence specific probes disclosed herein specifically hybridize to the polymorphic right extremity sequences of an MREJ type iii region under standard conditions for nucleic acid amplification, and/or stringent hybridization conditions. In some embodiments described herein, the sequence specific probes disclosed herein specifically hybridize to the polymorphic right extremity sequences of an MREJ type iv region under standard conditions for nucleic acid amplification, and/or stringent hybridization conditions. In some embodiments described herein, the sequence specific probes disclosed herein specifically hybridize to the polymorphic right extremity sequences of an MREJ type v region under standard conditions for nucleic acid amplification, and/or stringent hybridization conditions. In some embodiments described herein, the sequence specific probes disclosed herein specifically hybridize to the polymorphic right extremity sequences of an MREJ type vi region under standard conditions for nucleic acid amplification, and/or stringent hybridization conditions. In some embodiments described herein, the sequence specific probes disclosed herein specifically hybridize to the polymorphic right extremity sequences of an MREJ type vii region under standard conditions for nucleic acid amplification, and/or stringent hybridization conditions. In some embodiments described herein, the sequence specific probes disclosed herein specifically hybridize to the polymorphic right extremity sequences of an MREJ type viii region under standard conditions for nucleic acid amplification, and/or stringent hybridization conditions. In some embodiments described herein, the sequence specific probes disclosed herein specifically hybridize to the polymorphic right extremity sequences of an MREJ type ix region under standard conditions for nucleic acid amplification, and/or stringent hybridization conditions. In some embodiments described herein, the sequence specific probes disclosed herein specifically hybridize to the polymorphic right extremity sequences of an MREJ type x region under standard conditions for nucleic acid amplification, and/or stringent hybridization conditions. In some embodiments described herein, the sequence specific probes disclosed herein specifically hybridize to the polymorphic right extremity sequences of an MREJ type xi region under standard conditions for nucleic acid amplification, and/or stringent hybridization conditions. In some embodiments described herein, the sequence specific probes disclosed herein specifically hybridize to the polymorphic right extremity sequences of an MREJ type xii region under standard conditions for nucleic acid amplification, and/or stringent hybridization conditions. In some embodiments described herein, the sequence specific probes disclosed herein specifically hybridize to the polymorphic right extremity sequences of an MREJ type xiii region under standard conditions for nucleic acid amplification, and/or stringent hybridization conditions. In some embodiments described herein, the sequence specific probes disclosed herein specifically hybridize to the polymorphic right extremity sequences of an MREJ type xiv region under standard conditions for nucleic acid amplification, and/or stringent hybridization conditions. In some embodiments described herein, the sequence specific probes disclosed herein specifically hybridize to the polymorphic right extremity sequences of an MREJ type xv region under standard conditions for nucleic acid amplification, and/or stringent hybridization conditions. In some embodiments described herein, the sequence specific probes disclosed herein specifically hybridize to the polymorphic right extremity sequences of an MREJ type xvi region under standard conditions for nucleic acid amplification, and/or stringent hybridization conditions. In some embodiments described herein, the sequence specific probes disclosed herein specifically hybridize to the polymorphic right extremity sequences of an MREJ type xvii region under standard conditions for nucleic acid amplification, and/or stringent hybridization conditions. In some embodiments described herein, the sequence specific probes disclosed herein specifically hybridize to the polymorphic right extremity sequences of an MREJ type xviii region under standard conditions for nucleic acid amplification, and/or stringent hybridization conditions. In some embodiments described herein, the sequence specific probes disclosed herein specifically hybridize to the polymorphic right extremity sequences of an MREJ type xix region under standard conditions for nucleic acid amplification, and/or stringent hybridization conditions. In some embodiments described herein, the sequence specific probes disclosed herein specifically hybridize to the polymorphic right extremity sequences of an MREJ type xx region under standard conditions for nucleic acid amplification, and/or stringent hybridization conditions. In some embodiments described herein, the sequence specific probes disclosed herein specifically hybridize to *mecA* sequences under standard conditions for nucleic acid amplification, and/or stringent hybridization conditions. In some embodiments described herein, the sequence specific probes disclosed herein specifically hybridize to *mecC* sequences under standard conditions for nucleic acid amplification, and/or stringent hybridization conditions. In some embodiments described herein, the sequence specific probes disclosed herein specifically hybridize to *nuc* sequences under standard conditions for nucleic acid amplification, and/or stringent hybridization conditions. In some embodiments described herein, the sequence specific probes disclosed herein specifically hybridize to *femB* sequences under standard conditions for nucleic acid amplification, and/or stringent hybridization conditions. In some embodiments described herein, the sequence specific probes disclosed herein specifically hybridize to *Sa442* sequences under standard conditions for nucleic acid amplification, and/or stringent hybridization conditions.

In some embodiments described herein, the sequence specific probes disclosed herein specifically hybridize to *S. aureus* chromosomal sequences located within 1 kilobase from the insertion point of the *SCCmec* element into the chromosomal DNA. For example, in some embodiments described herein, provided is a sequence specific probe that specifically hybridizes to *S. aureus orfX* sequences under standard conditions for nucleic acid amplification, and/or stringent hybridization conditions. In some embodiments described herein, provided is a sequence specific probe that hybridizes to orfSA0022 under standard conditions for nucleic acid amplification. In some embodiments described herein, the sequence specific probes disclosed herein specifically hybridize to the MREP sequences, *e.g*., MREP type i, ii, iii, iv, v, vi, vii, viii, ix, x, xi, xii, xiii, xiv, xv, xvi, xvii, xviii, xix, xx, or xxi sequences. In some embodiments described herein, more than one sequence specific probe is provided. For example, in some embodiments described herein, sequence specific probes that specifically hybridize to MREP type xxi sequences are provided in combination with one, two, three, four, five, six, seven, eight, nine, ten, eleven, twelve, thirteen, fourteen, fifteen, sixteen, seventeen, eighteen, nineteen or twenty, or more, sequence specific probes.

In some embodiments described herein, oligonucleotide probes can include a detectable moiety. For example, in some embodiments described herein, the oligonucleotide probes disclosed herein can comprise a radioactive label. Non-limiting examples of radioactive labels include ³H, ¹⁴C, ³²P, and ³⁵S. In some embodiments described herein, oligonucleotide probes can include one or more non-radioactive detectable markers or moieties, including but not limited to ligands, fluorophores, chemiluminescent agents, enzymes, and antibodies. Other detectable markers for use with probes, which can enable an increase in sensitivity of the method described herein, include biotin and radio-nucleotides. It will become evident to the person of ordinary skill that the choice of a particular label dictates the manner in which it is bound to the probe. For example, oligonucleotide probes labeled with one or more dyes, such that upon hybridization to a template nucleic acid, a detectable change in fluorescence is generated. While non-specific dyes may be desirable for some applications, sequence-specific probes can provide more accurate measurements of amplification. One configuration of sequence-specific probe can include one end of the probe tethered to a fluorophore, and the other end of the probe tethered to a quencher. When the probe is unhybridized, it can maintain a stem-loop configuration, in which the fluorophore is quenched by the quencher, thus preventing the fluorophore from fluorescing. When the probe is hybridized to a template nucleic sequence, it is linearized, distancing the fluorophore from the quencher, and thus permitting the fluorophore to fluoresce. Another configuration of sequence-specific probe can include a first probe tethered to a first fluorophore of a FRET pair, and a second probe tethered to a second fluorophore of a FRET pair. The first probe and second probe can be configured to hybridize to sequences of an amplicon that are within sufficient proximity to permit energy transfer by FRET when the first probe and second probe are hybridized to the same amplicon.

In some embodiments described herein, the sequence specific probe comprises an oligonucleotide as disclosed herein conjugated to a fluorophore. In some embodiments described herein, the probe is conjugated to two or more fluorophore. Examples of fluorophores include: xanthene dyes, e.g., fluorescein and rhodamine dyes, such as fluorescein isothiocyanate (FITC), 2-[ethylamino)-3-(ethylimino)-2-7-dimethyl-3H-xanthen-9-yl]benzoic acid ethyl ester monohydrochloride (R6G)(emits a response radiation in the wavelength that ranges from about 500 to 560 nm), 1,1,3,3,3',3'-Hexamethylindodicarbocyanine iodide (HIDC) (emits a response radiation in the wavelength that ranged from about 600 to 660 nm), 6-carboxyfluorescein (commonly known by the abbreviations FAM and F), 6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g. Cy3, Cy5 and Cy7 dyes; coumarins, e.g., umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3 (emits a response radiation in the wavelength that ranges from about 540 to 580 nm), Cy5 (emits a response radiation in the wavelength that ranges from about 640 to 680 nm), etc; BODIPY dyes and quinoline dyes. Specific fluorophores of interest include: Pyrene, Coumarin, Diethylaminocoumarin, FAM, Fluorescein Chlorotriazinyl, Fluorescein, R110, Eosin, JOE, R6G, HIDC, Tetramethylrhodamine, TAMRA, Lissamine, ROX, Napthofluorescein, Texas Red, Napthofluorescein, Cy3, and Cy5, and the like.

In some embodiments described herein, the probe is conjugated to a quencher. A quencher can absorb electromagnetic radiation and dissipate it as heat, thus remaining dark. Example quenchers include Dabcyl, NFQ's, such as BHQ-1 or BHQ-2 (Biosearch), IOWA BLACK FQ (IDT), and IOWA BLACK RQ (IDT). In some embodiments described herein, the quencher is selected to pair with a fluorphore so as to absorb electromagnetic radiation emitted by the fluorophore. Flourophore/quencher pairs useful in the compositions and methods disclosed herein are well-known in the art, and can be found, e.g., described in S. Marras, "Selection of Fluorophore and Quencher Pairs for Fluorescent Nucleic Acid Hybridization Probes" available at the world wide web site molecular-beacons.org/download/marras,mmb06%28335%293.pdf.

In some embodiments described herein, a fluorophore is attached to a first end of the probe, and a quencher is attached to a second end of the probe. Attachment can include covalent bonding, and can optionally include at least one linker molecule positioned between the probe and the fluorophore or quencher. In some embodiments described herein, a fluorophore is attached to a 5' end of a probe, and a quencher is attached to a 3' end of a probe. In some embodiments described herein, a fluorophore is attached to a 3' end of a probe, and a quencher is attached to a 5' end of a probe. Examples of probes that can be used in quantitative nucleic acid amplification include molecular beacons, SCORPION™ probes (Sigma), TAQMAN™ probes (Life Technologies) and the like. Other nucleic acid detection technologies that are useful in the embodiments disclosed herein include, but are not limited to nanoparticle probe technology (*See,* Elghanian, et al. (1997) Science 277:1078-1081.) and Amplifluor probe technology (*See,* U.S. Pat. No's: 5,866,366; 6,090,592; 6,117,635; and 6,117,986).

Some embodiments disclosed herein provide probes that specifically hybridize to an MREJ type xxi sequence, or an amplicon of an MREJ type xxi sequence, *e.g*., SEQ ID NO: 1, or a subsequence thereof. Accordingly, some embodiments disclosed herein provide a probe that hybridizes to an amplicon from the amplification of a template comprising SEQ ID NO: 1 using the amplification primers SEQ ID NOs: 2 and 3. By way of example only, in some embodiments described herein, the probe can comprise, consist essentially of, or consist of the sequence of SEQ ID NO: 4, or a variant thereof, is provided. In some embodiments described herein, the probe comprises a fluorophore and/or quencher as described herein. In some embodiments described herein, the probe can comprise, consist essentially of, or consist of the sequence of SEQ ID NO: 82, or a variant thereof, is provided. In some embodiments described herein, the probe comprises a fluorophore and/or quencher as described herein. In preferred embodiments described herein, probes can comprise SEQ ID NO: 4 or SEQ ID NO:82, or variants thereof, with the fluorophore 6-carboxyfluorescein ("FAM") attached to the 5' end of the probe, and the quencher IOWA BLACK Black-hole Quencher® 2 (IDT) ("BHQ") attached to the 3' end of the probe.

### Kits

Also provided herein are kits. The kits, primers and probes disclosed herein can be used to detect and/or identify MRSA of MREJ type xxi (and, in some embodiments described herein, additionally to detect and/or identify MRSA of one or more of MREJ types i-xx), in both *in vitro* and/or *in situ* applications. For example, it is contemplated that the kits may be used in combination with any previously described primers/probes for detecting MRSA of MREJ types i to xx. It is also contemplated that the diagnostic kits, primers and probes disclosed herein can be used alone or in combination with any other assay suitable to detect and/or identify microorganisms, including but not limited to: any assay based on nucleic acids detection, any immunoassay, any enzymatic assay, any biochemical assay, any lysotypic assay, any serological assay, any differential culture medium, any enrichment culture medium, any selective culture medium, any specific assay medium, any identification culture medium, any enumeration culture medium, any cellular stain, any culture on specific cell lines, and any infectivity assay on animals.

Accordingly, in some embodiments described herein the kits disclosed herein will include an oligonucleotide that specifically binds to the polymorphic right extremity sequences of MREJ type xxi sequences (e.g., SEQ ID NO:1 or the complement thereof) under standard nucleic acid amplification conditions, and/or stringent hybridization conditions. For example, in some embodiments described herein, the kits disclosed herein will include an oligonucleotide (*e.g*., a first amplification primer) that comprises, consists essentially of, or consists of SEQ ID NO:2, or a variant thereof. In some embodiments described herein, the kit can also include one or more additional oligonucleotides, *e.g*., a second amplification primer such as an oligonucleotide that comprises, consists essentially of, or consists of SEQ ID NO:3, or a variant thereof, that, together with a first amplification primer will generate an amplicon of the polymorphic right extremity junction of MREJ type xxi sequences. In some embodiments described herein, the kit can also include a probe as described herein. For example, in some embodiments described herein, the kit can include an oligonucleotide probe that comprises, consists essentially of, or consists of SEQ ID NO:4, or a variant thereof.

In some embodiments described herein, the kits disclosed herein can include, in addition to an oligonucleotide that specifically binds to the polymorphic right extremity sequences of MREJ type xxi sequences under standard amplification conditions, one or more oligonucleotides that specifically bind to one or more of the *SCCmec* polymorphic right extremity sequences (MREP) within MREJ type i, ii, iii, iv, v, vi, vii, viii, ix, x, xi, xii, xiii, xiv, xv, xvi, xvii, xviii, xix, or xx. In some embodiments described herein, the disclosed herein can include, in addition to an oligonucleotide that specifically binds to the polymorphic right extremity sequences of MREJ type xxi sequences under standard amplification conditions, one or more oligonucleotides that specifically bind to *mecA* sequences. In some embodiments described herein, the kits disclosed herein can include, in addition to an oligonucleotide that specifically binds to the polymorphic right extremity sequences of MREJ type xxi sequences under standard amplification conditions, one or more oligonucleotides that specifically bind to *mecC* sequences. In some embodiments described herein, the kits disclosed herein can include, in addition to an oligonucleotide that specifically binds to the polymorphic right extremity sequences of MREJ type xxi sequences under standard amplification conditions, one or more oligonucleotides that specifically bind to *nuc* sequences. In some embodiments described herein, the kits disclosed herein can include, in addition to an oligonucleotide that specifically binds to the polymorphic right extremity sequences of MREJ type xxi sequences under standard amplification conditions, one or more oligonucleotides that specifically bind to *S. aureus-*specific nucleotide sequences, *e.g., femB* sequences. In some embodiments described herein, the kits disclosed herein can include, in addition to an oligonucleotide that specifically binds to the polymorphic right extremity sequences of MREJ type xxi sequences under standard amplification conditions, one or more oligonucleotides that specifically bind to Sa442 sequences.

In some embodiments described herein, provided are kits containing the reagents and compositions to carry out the methods described herein. Such a kit can comprise a carrier being compartmentalized to receive in close confinement therein one or more containers, such as tubes or vials. One of the containers may contain at least one unlabeled or detectably labeled primer or probe disclosed herein. The primer or primers can be present in lyophilized form or in an appropriate buffer as necessary. One or more containers may contain one or more enzymes or reagents to be utilized in, for example, nucleic acid amplification reactions reactions. These enzymes may be present by themselves or in admixtures, in lyophilized form or in appropriate buffers. Exemplary enzymes useful in nucleic acid amplification reactions as disclosed herein include, but are not limited to, FASTSTART™ Taq DNA polymerase, APTATAQ™ DNA polymerase (Roche), KLENTAQ 1™ DNA polymerase (AB peptides Inc.), HOTGOLDSTAR™ DNA polymerase (Eurogentec), KAPATAQ™ HotStart DNA polymerase, KAPA2G™ Fast HotStart DNA polymerase (Kapa Biosystemss), PHUSION™ Hot Start DNA Polymerase (Finnzymes), or the like.

Additionally, the kits disclosed herein can include all of the additional elements necessary to carry out the methods disclosed herein, such as buffers, extraction reagents, enzymes, pipettes, plates, nucleic acids, nucleoside triphosphates, filter paper, gel materials, transfer materials, autoradiography supplies, and the like.

In some embodiments described herein, the kits include additional reagents that are required for or convenient and/or desirable to include in the reaction mixture prepared during the methods disclosed herein, where such reagents include: one or more polymerases; an aqueous buffer medium (either prepared or present in its constituent components, where one or more of the components may be premixed or all of the components may be separate), and the like. The various reagent components of the kits may be present in separate containers, or may all be pre-combined into a reagent mixture for combination with template nucleic acid.

In addition to the above components, in some embodiments described herein, the kits can also include instructions for practicing the methods disclosed herein. These instructions can be present in the kits in a variety of forms, one or more of which may be present in the kit. One form in which these instructions can be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address that may be used via the internet to access the information at a removed site. Any convenient means may be present in the kits.

### Methods

Provided herein are methods for the detection, identification and/or quantification of MRSA having MREJ type xxi nucleic acids from a sample. In some embodiments described herein, the methods include the step of contacting the sample to be analyzed with an oligonucleotide that specifically hybridizes to the polymorphic right extremity sequences of an *SCCmec* MREJ type xxi region under standard nucleic acid amplification conditions and/or stringent hybridization conditions.

In some embodiments described herein, a sample to be tested for the presence of an MRSA having an *SCCmec* MREJ type xxi region is processed prior to performing the methods disclosed herein. For example, in some embodiments described herein, the sample can be isolated, concentrated, or subjected to various other processing steps prior to performing the methods disclosed herein. For example, in some embodiments described herein, the sample can be processed to isolate nucleic acids from the sample prior to contacting the sample with the oligonucleotides, as disclosed herein. As used herein, the phrase "isolate nucleic acids" refers to the purification of nucleic acids from one or more cellular components. The skilled artisan will appreciate that samples processed to "isolate nucleic acids" therefrom can include components and impurities other than nucleic acids. In some embodiments described herein, the methods disclosed herein are performed on the sample without culturing the sample *in vitro.* In some embodiments described herein, the methods disclosed herein are performed on the sample without isolating nucleic acids from the sample prior to contacting the sample with oligonucleotides as disclosed herein.

### i. Non-Amplification Based Methods

In some embodiments described herein, the oligonucleotide comprises a detectable moiety, as described elsewhere herein, and the specific hybridization of the oligonucleotide to the polymorphic right extremity sequences of an *SCCmec* MREJ type xxi region can be detected, e.g., by direct or indirect means. Accordingly, some embodiments described herein for the detection and/or identification of methicillin-resistant *Staphylococcus aureus* (MRSA) comprising MREJ type xxi nucleic acids, that include the steps of providing a test sample; and contacting the sample with an oligonucleotide probe that specifically hybridizes to the polymorphic right extremity sequences of an *SCCmec* MREJ type xxi region under standard nucleic acid amplification conditions and/or stringent hybridization conditions, wherein the oligonucleotide probe is between 10 and 45 nucleotides in length, and comprises a detectable moiety, wherein the contacting is performed under conditions allowing for the specific hybridization of the primer to the mec right extremity junction of the MREJ type xxi sequence if *S. aureus* comprising MREJ type xxi sequences is present in the sample. The presence and/or amount of probe that is specifically bound to mec right extremity junction of the MREJ type xxi sequence (if present in the sample being tested) can be determined, wherein bound probe is indicative of the presence of an MRSA having *SCCmec* MREJ type xxi sequences in the sample. In some embodiments described herein, the amount of bound probe is used to determine the amount of MRSA having *SCCmec* MREJ type xxi sequences in the sample.

Similarly, in some embodiments described herein, non-amplification based methods can be used to detect additional nucleotide sequences. For example, in some embodiments described herein, the methods disclosed herein can include the use of non-amplification based methods to detect the presence and/or amount of other MREJ sequences, *e.g.,* one or more of MREJ types i, ii, iii, iv, v, vi, vii, viii, ix, x, xi, xii, xiii, xiv, xv, xvi, xvii, xviii, xix, or xx, or any combination thereof, in addition to MREJ type xxi sequences. In some embodiments described herein, the methods disclosed herein can include the use of non-amplification based methods to detect the presence of *mecA* sequences, in addition to MREJ (or MREP) type xxi sequences. In some embodiments described herein, the methods disclosed herein can include the use of non-amplification based methods to detect the presence of *mecC* sequences, in addition to (MREP) MREJ type xxi sequences. In some embodiments described herein, the methods disclosed herein can include the use of non-amplification based methods to detect the presence of *S. aureus* specific sequences, such as *nuc* sequences, *femB* sequences, Sa442 sequences, 16S rRNA sequences, or the like in addition to (MREP) MREJ type xxi sequences. Accordingly, in an exemplary embodiment described herein, provided herein are methods and compositions for the simultaneous detection of MREJ type xxi, i, ii, iii, iv, vii, *mecA, mecC,* and *nuc* sequences.

The determining step can be achieved using any methods known to those skilled in the art, including but not limited to, *in situ* hybridization, following the contacting step. The detection of hybrid duplexes (*i.e.,* of a probe specifically bound to polymorphic right extremity sequences from an MREJ type xxi region) can be carried out by a number of methods. Typically, hybridization duplexes are separated from unhybridized nucleic acids and the labels bound to the duplexes are then detected. Such labels refer to radioactive, fluorescent, biological or enzymatic tags or labels of standard use in the art. A label can be conjugated to either the oligonucleotide probes or the nucleic acids derived from the biological sample. Those skilled in the art will appreciate that wash steps may be employed to wash away excess sample/target nucleic acids or oligonucleotide probe (as well as unbound conjugate, where applicable). Further, standard heterogeneous assay formats are suitable for detecting the hybrids using the labels present on the oligonucleotide primers and probes.

Thus, according to some embodiments described herein, the methods disclosed herein can include, for example ELISA (*e.g*., in a dipstick format, a multi-well format, or the like) using art-recognized methods.

### ii. Amplification-Based Methods

In some embodiments described herein, the methods for the detection and/or identification of methicillin-resistant *Staphylococcus aureus* (MRSA) comprising MREJ type xxi nucleic acids, that include the steps of providing a test sample; and contacting the sample with an oligonucleotide probe that specifically hybridizes to the polymorphic right extremity sequences of an *SCCmec* MREJ type xxi region under standard nucleic acid amplification conditions and/or stringent hybridization conditions, wherein the oligonucleotide probe is between 10 and 45 nucleotides in length.

In some embodiments described herein, the sample is contacted under standard amplification conditions, or conditions allowing for the specific hybridization and extension of the primer to the mec right extremity polymorphic sequence (MREP sequence) of the MREJ type xxi sequence if *S*. *aureus* comprising MREJ type xxi sequences is present in the sample. Accordingly, the methods include the step of specific amplification of MREJ type xxi nucleic acids from samples, e.g., to generate amplicons or amplification products that include the mec right extremity junction of an MREJ type xxi sequence. In some embodiments described herein, the sample is contacted under standard amplification conditions, or conditions allow for the specific hybridization of a primer pair, *e.g.,* a first primer that hybridizes to the *mec* right extremity polymorphic sequence (MREP sequence) and a second primer that hybridizes to the S. aureus chromosomal sequence adjacent to the *SCCmec* right extremity, *i.e., orfX* in order to generate an amplicon across the *SCCmec* - chromosomal junction. Some embodiments described herein provide methods to generate *SCCmec* right extremity junction sequence data by contacting a sample under standard amplification conditions, or conditions allow for the specific hybridization of a primer pair, *e.g.,* a first primer that hybridizes to the *mec* right extremity polymorphic sequence (MREP sequence) and a second primer that hybridizes to the *S. aureus* chromosomal sequence adjacent to the *SCCmec* right extremity, *i.e., orfX* in order to generate an amplicon across the *SCCmec* - chromosomal junction.

In some embodiments described herein, the sample is contacted, *e.g*., simultaneously with (as in multiplex PCR), or sequentially to the contacting with the MREJ type xxi-specific oligonucleotide(s), under the same standard amplification conditions, with additional primers that allow for the specific amplification of one or more additional MREJ type sequences, *e.g*., one or more of MREJ type i, ii, iii, iv, v, vi, vii, viii, ix, x, xi, xii, xiii, xiv, xv, xvi, xvii, xviii, xix, or xx sequences. In some embodiments described herein, the sample is contacted, *e.g*., simultaneously with (multiplex PCR), or sequentially to, the contacting with MREJ type xxi-specific oligonucleotide(s), under the same standard amplification conditions, with additional primers that allow for the specific amplification *of mecA* and/or *mecC* sequences. In some embodiments described herein, the sample is contacted, *e.g*., simultaneously with (multiplex PCR), or sequentially to, the contacting with MREJ type xxi-specific oligonucleotide(s), under the same standard amplification conditions, with additional primers that allow for the specific amplification of one or more sequences that is unique to *S*. *aureus* (*S. aureus*-specific sequences), such as *nuc, femB, Sa442,* and the like. Accordingly, in some embodiments described herein, the sample is contacted with primers specific for MREP type xxi, i, ii, iii, iv, v, vii, ix, xiii, xiv, and xxi sequences, as well as *mecA, mecC* and *nuc* sequences.

In some embodiments described herein, the methods include the identification of a specific type of sequence (*e.g*., an MREJ type xxi sequence). For example, in embodiments described herein involving the specific amplification of only MREJ tye xxi sequences in simplexm the presence or the absence of an amplicon is indicative of the presence or absence of MREJ type xxi sequences in the sample. In some embodiments described herein, the methods involve the specific amplification of additional sequences, *e.g.,* MREJ sequences, *mec* sequences, *S*. *aureus* specific sequences, and the like, in multiplex with the specific amplification of MREJ type xxi sequences. In the embodiments described herein that involve multiplex amplification, in some embodiments described herein, the methods can include the identification or detection of specific sequences, *e.g*., by using sequence specific probes that hybridize to only one amplicon. In some embodiments described herein, the methods do not discriminate between some or all of the different possible amplicons present in the sample after amplification. For example, in some embodiments described herein, a sequence specific probe that hybridizes to *orfX* can be used to detect the presence of amplicons of one or more MREJ types. In some embodiments described herein, the methods include the detection of an amplification product, without the specific detection of a particular sequence.

Several methods for the specific amplification of target nucleic acids are known in the art, and are useful in the embodiments disclosed herein. Non-limiting examples of amplification methods include Polymerase Chain Reaction (PCR; see Saiki et al., 1985, Science 230:1350-1354, herein incorporated by reference), Ligase Chain Reaction (LCR; see Wu et al., 1989, Genomics 4:560-569; Barringer et al., 1990, Gene 89:117-122; Barany, 1991, Proc. Natl. Acad. Sci. USA 88:189-193, all of which are incorporated herein by reference), Transcription Mediated Amplification (TMA; see Kwoh et al., 1989, Proc. Natl. Acad. Sci. USA 86:1173-1177, herein incorporated by reference), Self-Sustaining Sequence Replication (3SR; see Guatelli et al., 1990, Proc. Natl. Acad. Sci. USA 87:1874-1878, herein incorporated by reference), Rolling Circle Amplification (RCA), Nucleic Acid Sequence Based Amplification (NASBA), Q β replicase system (Lizardi et al., 1988, BioTechnology 6:1197-1202, herein incorporated by reference) and Strand Displacement Amplification (SDA; see Walker et al., 1992, Proc. Natl. Acad. Sci. USA 89:392-396; Walker et al., 1992, Nuc. Acids. Res. 20:1691-1696; and EP 0 497 272, all of which are incorporated herein by reference)) including thermophilic SDA (tSDA).

In various embodiments described herein, the methods disclosed herein are useful for detecting the presence of *SCCmec* MREJ type xxi nucleic acids or sequences in clinical samples. For example, in some embodiments described herein, the methods disclosed herein are useful for detecting and identifying *S. aureus* having type xxi MREJ regions samples having concentration of bacteria that is within physiological ranges (i.e., the concentration of bacteria in a sample collected from a subject infected with the bacteria). Thus, a sample can be directly screened without the need for isolating, concentrating, or expanding (e.g., culturing) the bacterial population in order to detect the presence of MRSA having MREJ type xxi nucleic acids. In various embodiments described herein, the methods disclosed herein are capable of detecting the presence of a MRSA having MREJ type xxi nucleic acids from a sample that has a concentration of bacteria of about 1 CFU/ml, 10 CFU/ml, 100 CFU/ml, 1x 10³ CFU/ml, 1x 10³ CFU/ml, about 1 x 10⁴ CFU/ml, about 1x 10⁵ CFU/ml, or about 1x 10⁶ CFU/ml, or any number in between.

In some embodiments described herein, the methods described herein, the methods include the performance of PCR or qPCR in order to generate an amplicon. Numerous different PCR and qPCR protocols are known in the art and exemplified herein below and can be directly applied or adapted for use using the presently described compositions for the detection and/or identification of MRSA having MREJ type xxi nucleic acids in a sample.

Generally, in PCR, a target polynucleotide sequence is amplified by reaction with at least one oligonucleotide primer or pair of oligonucleotide primers. The primer(s) specifically hybridize to a complementary region of the target nucleic acid and a DNA polymerase extends the primer(s) to amplify the target sequence. Under conditions sufficient to provide polymerase-based nucleic acid amplification products, a nucleic acid fragment of one size dominates the reaction products (the target polynucleotide sequence that is the amplification product). The amplification cycle is repeated to increase the concentration of the single target polynucleotide sequence. The reaction can be performed in any thermocycler commonly used for PCR. However, preferred are cyclers with real-time fluorescence measurement capabilities, for example, SMARTCYCLER® (Cepheid, Sunnyvale, CA), ABI PRISM 7700® (Applied Biosystems, Foster City, CA), ROTOR-GENE™; (Corbett Research, Sydney, Australia), LIGHTCYCLER® (Roche Diagnostics Corp, Indianapolis, IN), ICYCLER® (Biorad Laboratories, Hercules, CA) and MX4000® (Stratagene, La Jolla, CA)

Some embodiments described herein provide methods including Quantitative PCR (qPCR) (also referred as real-time PCR). qPCR can provide quantitative measurements, and also provide the benefits of reduced time and contamination. As used herein, "quantitative PCR" (or "real time qPCR") refers to the direct monitoring of the progress of a PCR amplification as it is occurring without the need for repeated sampling of the reaction products. In qPCR, the reaction products may be monitored via a signaling mechanism (e.g., fluorescence) as they are generated and are tracked after the signal rises above a background level but before the reaction reaches a plateau. The number of cycles required to achieve a detectable or "threshold" level of fluorescence (herein referred to as cycle threshold or "CT") varies directly with the concentration of amplifiable targets at the beginning of the PCR process, enabling a measure of signal intensity to provide a measure of the amount of target nucleic acid in a sample in real time.

Methods for setting up PCR and qPCR are well known to those skilled in the art. The reaction mixture minimally comprises template nucleic acid (*e.g.,* as present in test samples, except in the case of a negative control as described below) and oligonucleotide primers and/or probes in combination with suitable buffers, salts, and the like, and an appropriate concentration of a nucleic acid polymerase. As used herein, "nucleic acid polymerase" refers to an enzyme that catalyzes the polymerization of nucleoside triphosphates. Generally, the enzyme will initiate synthesis at the 3'-end of the primer annealed to the target sequence, and will proceed in the 5'-direction along the template until synthesis terminates. An appropriate concentration includes one that catalyzes this reaction in the presently described methods. Known DNA polymerases useful in the methods disclosed herein include, for example, *E. coli* DNA polymerase I, T7 DNA polymerase, *Thermus thermophilus* (Tth) DNA polymerase, *Bacillus stearothermophilus* DNA polymerase, *Thermococcus litoralis* DNA polymerase, *Thermus aquaticus* (Taq) DNA polymerase and *Pyrococcus furiosus* (Pfu) DNA polymerase, FASTSTART™ Taq DNA polymerase, APTATAQ™ DNA polymerase (Roche), KLENTAQ 1™ DNA polymerase (AB peptides Inc.), HOTGOLDSTAR™ DNA polymerase (Eurogentec), KAPATAQ™ HotStart DNA polymerase,KAPA2G™ Fast HotStart DNA polymerase (Kapa Biosystemss), PHUSION™ Hot Start DNA Polymerase (Finnzymes), or the like.

In addition to the above components, the reaction mixture of the present methods includes primers, probes, and deoxyribonucleoside triphosphates (dNTPs).

Usually the reaction mixture will further comprise four different types of dNTPs corresponding to the four naturally occurring nucleoside bases, i.e., dATP, dTTP, dCTP, and dGTP. In the methods of the invention, each dNTP will typically be present in an amount ranging from about 10 to 5000 µM, usually from about 20 to 1000 µM, about 100 to 800 µM, or about 300 to 600 µM.

The reaction mixture can further include an aqueous buffer medium that includes a source of monovalent ions, a source of divalent cations, and a buffering agent. Any convenient source of monovalent ions, such as potassium chloride, potassium acetate, ammonium acetate, potassium glutamate, ammonium chloride, ammonium sulfate, and the like may be employed. The divalent cation may be magnesium, manganese, zinc, and the like, where the cation will typically be magnesium. Any convenient source of magnesium cation may be employed, including magnesium chloride, magnesium acetate, and the like. The amount of magnesium present in the buffer may range from 0.5 to 10 mM, and can range from about 1 to about 6 mM, or about 3 to about 5 mM. Representative buffering agents or salts that may be present in the buffer include Tris, Tricine, HEPES, MOPS, and the like, where the amount of buffering agent will typically range from about 5 to 150 mM, usually from about 10 to 100 mM, and more usually from about 20 to 50 mM, where in certain preferred embodiments described herein the buffering agent will be present in an amount sufficient to provide a pH ranging from about 6.0 to 9.5, for example, about pH 6.0, 6.5, 7.0, 7.5, 8.0, 8.5, 9.0, or 9.5. Other agents that may be present in the buffer medium include chelating agents, such as EDTA, EGTA, and the like. In some embodiments described herein, the reaction mixture can include BSA, or the like. In addition, in some embodiments described herein, the reactions can include a cryoprotectant, such as trehalose, particularly when the reagents are provided as a master mix, which can be stored over time.

In preparing a reaction mixture, the various constituent components may be combined in any convenient order. For example, the buffer may be combined with primer, polymerase, and then template nucleic acid, or all of the various constituent components may be combined at the same time to produce the reaction mixture.

Alternatively, commercially available premixed reagents can be utilized in the methods disclosed herein, according to the manufacturer's instructions, or modified to improve reaction conditions (e.g., modification of buffer concentration, cation concentration, or dNTP concentration, as necessary), including, for example, TAQMAN® Universal PCR Master Mix (Applied Biosystems), OMNIMIX® or SMARTMIX® (Cepheid), IQ™ Supermix (BioRad Laboratories), LIGHTCYCLER® FastStart (Roche Applied Science, Indianapolis, IN), or BRILLIANT® QPCR Master Mix (Stratagene, La Jolla, CA).

The reaction mixture can be subjected to primer extension reaction conditions ("conditions sufficient to provide polymerase-based nucleic acid amplification products"), i.e., conditions that permit for polymerase-mediated primer extension by addition of nucleotides to the end of the primer molecule using the template strand as a template. In many embodiments described herein, the primer extension reaction conditions are amplification conditions, which conditions include a plurality of reaction cycles, where each reaction cycle comprises: (1) a denaturation step, (2) an annealing step, and (3) a polymerization step. As discussed below, in some embodiments described herein, the amplification protocol does not include a specific time dedicated to annealing, and instead comprises only specific times dedicated to denaturation and extension. The number of reaction cycles will vary depending on the application being performed, but will usually be at least 15, more usually at least 20, and may be as high as 60 or higher, where the number of different cycles will typically range from about 20 to 40. For methods where more than about 25, usually more than about 30 cycles are performed, it may be convenient or desirable to introduce additional polymerase into the reaction mixture such that conditions suitable for enzymatic primer extension are maintained.

The denaturation step comprises heating the reaction mixture to an elevated temperature and maintaining the mixture at the elevated temperature for a period of time sufficient for any double-stranded or hybridized nucleic acid present in the reaction mixture to dissociate. For denaturation, the temperature of the reaction mixture will usually be raised to, and maintained at, a temperature ranging from about 85 to 100°C, usually from about 90 to 98°C, and more usually from about 93 to 96°C, for a period of time ranging from about 3 to 120 sec, usually from about 3 sec.

Following denaturation, the reaction mixture can be subjected to conditions sufficient for primer annealing to template nucleic acid present in the mixture (if present), and for polymerization of nucleotides to the primer ends in a manner such that the primer is extended in a 5' to 3' direction using the nucleic acid to which it is hybridized as a template, i.e., conditions sufficient for enzymatic production of primer extension product. In some embodiments described herein, the annealing and extension processes occur in the same step. The temperature to which the reaction mixture is lowered to achieve these conditions will usually be chosen to provide optimal efficiency and specificity, and will generally range from about 50 to 85°C, usually from about 55 to 70°C, and more usually from about 60 to 68°C. In some embodiments described herein, the annealing conditions can be maintained for a period of time ranging from about 15 sec to 30 min, usually from about 20 sec to 5 min, or about 30 sec to 1 minute, or about 30 seconds.

This step can optionally comprise one of each of an annealing step and an extension step with variation and optimization of the temperature and length of time for each step. In a two-step annealing and extension, the annealing step is allowed to proceed as above. Following annealing of primer to template nucleic acid, the reaction mixture will be further subjected to conditions sufficient to provide for polymerization of nucleotides to the primer ends as above. To achieve polymerization conditions, the temperature of the reaction mixture will typically be raised to or maintained at a temperature ranging from about 65 to 75°C, usually from about 67 to 73°C and maintained for a period of time ranging from about 15 sec to 20 min, usually from about 30 sec to 5 min. In some embodiments described herein, the methods disclosed herein do not include a separate annealing and extension step. Rather, the methods include denaturation and extension steps, without any step dedicated specifically to annealing.

The above cycles of denaturation, annealing, and extension may be performed using an automated device, typically known as a thermal cycler. Thermal cyclers that may be employed are described elsewhere herein as well as in U.S. Patent Nos. 5,612,473; 5,602,756; 5,538,871; and 5,475,610.

The methods described herein can also be used in non-PCR based applications to detect a target nucleic acid sequence, where such target may be immobilized on a solid support. Methods of immobilizing a nucleic acid sequence on a solid support are known in the art and are described in Ausubel et ah, eds. (1995) Current Protocols in Molecular Biology (Greene Publishing and Wiley-Interscience, NY), and in protocols provided by the manufacturers, e.g., for membranes: Pall Corporation, Schleicher & Schuell; for magnetic beads: Dynal; for culture plates: Costar, Nalgenunc; for bead array platforms: Luminex and Becton Dickinson; and, for other supports useful according to the embodiments described herein, CPG, Inc.

Variations on the exact amounts of the various reagents and on the conditions for the PCR or other suitable amplification procedure (e.g., buffer conditions, cycling times, etc.) that lead to similar amplification or detection/quantification results are known to those of skill in the art and are considered to be equivalents. In one embodiment described herein, the subject qPCR detection has a sensitivity of detecting fewer than 50 copies (preferably fewer than 25 copies, more preferably fewer than 15 copies, still more preferably fewer than 10 copies, *e.g.* 5, 4, 3, 2, or 1 copy) of target nucleic acid (i.e., MREJ type xxi nucleic acids) in a sample.

### Controls

The assays disclosed herein can optionally include controls. PCR or qPCR reactions disclosed herein may contain various controls. Such controls can include a "no template" negative control, in which primers, buffer, enzyme(s) and other necessary reagents (e.g., MgCl₂, nucleotides, and the like) are cycled in the absence of added test sample. This ensures that the reagents are not contaminated with polynucleotides that are reactive with the primers, and that produce spurious amplification products. In addition to "no template" controls, negative controls can also include amplification reactions with non-specific target nucleic acid included in the reaction, or can be samples prepared using any or all steps of the sample preparation (from nucleic acid extraction to amplification preparation) without the addition of a test sample (e.g., each step uses either no test sample or a sample known to be free of carbapenem-resistant microorganisms).

In some embodiments described herein, the methods disclosed herein can include a positive control, e.g., to ensure that the methods and reagents are performing as expected. The positive control can include known target that is unrelated to the MREJ type xxi target nucleic acids disclosed herein. Prior to amplification, the positive control nucleic acid (*e.g.,* in the form of a plasmid that is either linearized or non-linearized) can be added to the amplification reaction. A single reaction may contain either a positive control template, a negative control, or a sample template, or a single reaction may contain both a sample template and a positive control. Preferably, the positive control will comprise sequences that are substantially complementary to the MREJ type xxi forward and reverse amplification primers derived from the MREJ type xxi sequences disclosed herein, such that an amplification primer pair used to amplify MREJ type xxi sequences will also amplify control nucleic acids under the same assay conditions. In some embodiments described herein, the amplicon generated from the positive control template nucleic acids is larger than the target amplicon. Preferably, aside from the sequences in a positive control nucleic acid that are complementary or substantially complementary to the forward and reverse primers, the positive control nucleic acid will not share substantial similarity with the target amplicon/MREJ type xxi sequences disclosed herein. In other words, outside from the forward and reverse primers, the positive control amplicon is preferably less than 80%, less than 70%, less than 60%, less than 50%, less that 40%, less than 30%, less than 20%, and even more preferably, less than 10% identical with the positive control polynucleotide, e.g., when the sequence identity is compared using NCBI BLAST ALIGN tools.

Positive and/or negative controls can be used in setting the parameters within which a test sample will be classified as having or not having an MRSA having MREJ type xxi sequences. For example, in a qPCR reaction, the cycle threshold at which an amplicon is detected in a positive control sample can be used to set the threshold for classifying a sample as "positive," and the cycle threshold at which an amplicon is detected in a negative control sample can be used to set the threshold for classifying a sample as "negative." The CT from a single reaction may be used for each control, or the median or mean of replicate samples may be used. In yet another embodiment described herein, historical control values may be used. The minimum level of detection for each of the negative and the positive controls is typically set at the lower end of the 95% confidence interval of the mean CT across multiple reactions. This value can be adjusted depending on the requirements of the diagnostic assay.

Preferably, PCR controls should be performed at the same time as the test sample, using the same reagents, in the same amplification reaction.

Some embodiments described herein provide for the determination of the identity and/or amount of target amplification products, during the amplification reaction, e.g., in real-time. For example, some embodiments described herein relate to taking measurements of, for example, probe that is specifically bound to target amplicon nucleic acids, and/or positive control amplicons (*e.g*., as indicated by fluorescence). In some embodiments described herein, rather than using sequence-specific oligonucleotide probes, the methods can utilize non-sequence specific probes, which bind non- specifically to double-stranded nucleic acid, *e.g*., intercalating agents or the like. Intercalating agents have a relatively low fluorescence when unbound, and a relatively high fluorescence upon binding to double-stranded nucleic acids. As such, intercalating agents can be used to monitor the accumulation of double strained nucleic acids during a nucleic acid amplification reaction. Examples of such non-specific dyes include intercalating agents such as SYBR Green I™ (Molecular Probes), propidium iodide, ethidium bromide, LC green, SYTO9, EVAGREEN® fluorescent dye, CHROMOFY®, BEBO, and the like, that fluoresces and produces a detectable signal in the presence of double stranded nucleic acids. Measurements may be taken at a specified point during each cycle of an amplification reaction, *e.g*., after each extension step (prior to each denaturation step). Regardless of whether a sequence specific oligonucleotide probe or a non-sequence specific oligonucleotide probe is used, measurements of the amount of probe that is specifically bound to target amplicon nucleic acids, and/or positive control amplicons can be taken continuously throughout each cycle.

Alternatively, in some embodiments described herein, the identity/amount of the amplicons (*e.g*., target and/or positive control) can be confirmed after the amplification reaction is completed, using standard molecular techniques including (for example) Southern blotting, dot blotting and the like.

### EXAMPLES

The following examples are provided to demonstrate particular situations and settings in which this technology may be applied.

### EXAMPLE 1: DETECTION AND IDENTIFICATION OF MREJ TYPE XXI MRSA FROM

### CLINICAL SAMPLES

A qPCR reaction to detect and identify MRSA having MREJ type xxi nucleic acids is performed. Clinical samples are collected from patients using Amies liquid swabs (Copan Diagnostics, Inc).

DNA is optionally isolated from the clinical samples using the BD GeneOhm™ Lysis kit (Becton Dickinson) pursuant to manufacturer's instructions. A sample of the isolated DNA is contacted with primers that specifically hybridize under standard amplification conditions to *S*. *aureus* species-specific *orfX* sequences and to the polymorphic right extremity sequences of MREJ type xxi, *i.e.,* 0.2-0.7 µM each of SEQ ID NOs: 2, and 3. 0.3µM dNTPs (Roche), 4mM MgCl₂ (SIGMA), 2.8 units FASTSTART® Taq polymerase (Roche), 100mM Tris, pH 8.3 (EMD), 10mM KCl (LaboratoireMat), 5mM (NH₄)₂SO₄ (SIGMA), 0.15 mg/mL BSA (SIGMA) 4% trehalose (SIGMA). The reaction also includes molecular beacon probes that specifically hybridize to amplification products of the right extremity junction of MREJ type xxi detectable, and which include detectable moieties detectable on the BD MAX™ (Becton Dickinson), SMARTCYCLER® (Cepheid) apparatus, or other apparatus configured for real-time PCR at FAM, Texas Red and Tet channels are added to the reaction mixture.

PCR is carried out in a BD MAX™ (Becton Dickinson) or SMARTCYCLER® (Cepheid) using the same cycling parameters as follows:

| **Stage** | **Status** | **Temp (°C)** | **Sec** | **Optics** |
|---|---|---|---|---|
| 1 | Hold | 95 | 900 | off |
| 2 | Repeat 45 times | 95 | 1 -5 | off |
| | | 56 - 58 | 9, 10, or 15 | on |
| | | 72 | 10-20 | off |

The cycle threshold (CT) in FAM, Texas-Red, and TET channels is determined using the BD MAX™ or SMARTCYCLER® software.

### EXAMPLE 2: MULTIPLEX DETECTION OF MREJ TYPES I-XXI MRSA FROM CLINICAL SAMPLES

A multiplex amplification reaction is performed to detect the presence of MRSA having any of MREJ types i- vii, ix, xiii, xiv and xxi is performed. Clinical samples are collected from patients using Amies liquid swabs (Copan Diagnostics, Inc).

DNA is optionally isolated from the clinical samples using the BD GeneOhm™ Lysis kit (Becton Dickinson) pursuant to manufacturer's instructions. A sample of the isolated DNA is contacted with primers that specifically hybridize under standard amplification conditions to *S. aureus* species-specific *orfX* sequences and to the polymorphic right extremity sequences of MREJ type i- vii, ix, xiii, xiv and xxi and *i.e.,* 0.2-0.7 µM each of SEQ ID NOs: 2, 3, 39, 77, and 81, 0.3µM dNTPs (Roche), 4mM MgCl₂ (SIGMA), 2.8 units FASTSTART® Taq polymerase (Roche), 100mM Tris, pH 8.3 (EMD), 10mM KCl (LaboratoireMat), 5mM (NH₄)₂SO₄ (SIGMA), 0.15 mg/mL BSA (SIGMA) 4% trehalose (SIGMA). The reaction also includes molecular beacon probes that specifically hybridize to amplification products of the right extremity junction of MREJ type xxi detectable, and which include detectable moieties detectable on the BD MAX™ (Becton Dickinson), SMARTCYCLER® (Cepheid) apparatus, or other apparatus configured for real-time PCR at FAM, Texas Red and Tet channels are added to the reaction mixture, *i.e.,* SEQ ID NO:4.

PCR is carried out in a BD MAX™ (Becton Dickinson) or SMARTCYCLER® (Cepheid) using the same cycling parameters as follows:

| **Stage** | **Status** | **Temp (°C)** | **Sec** | **Optics** |
|---|---|---|---|---|
| 1 | Hold | 95 | 900 | off |
| 2 | Repeat 45 times | 95 | 1 -5 | off |
| | | 56 - 58 | 9, 10, or 15 | on |
| | | 72 | 10-20 | off |

The cycle threshold (CT) in FAM, Texas-Red, and TET channels is determined using the BD MAX™ or SMARTCYCLER® software. The CT is used to determine whether MRSA of any of MREJ types i-vii, xvi, ix, xiii, xiv and xxi are present.

### EXAMPLE 3: MREJ TYPE XXI SEUQENCES ARE ASSOCIATED WITH THE MECA HOMOLOG, MECC

PCR amplification of the MREJ type xxi region and the *mecC* gene was performed on 51 isolates of MRSA isolated from either bovine or human hosts.

A list of the isolates is provided in the table below:

| **Strain** | **MREJ type (confirmed by sequencing)** | **Host** | **Location** | **MREJ type** |
|---|---|---|---|---|
| **IDI6121** | xxi | Bovine | Somerset, England | xxi |
| **IDI6122** | xxi | Bovine | Somerset, England | xxi |
| **IDI6123** | xxi | Bovine | Bury St Edmunds, England | xxi |
| **IDI6124** | Unknown | Bovine | Langford, England | Unknown |
| **IDI6125** | xxi | Bovine | Bury St Edmunds, England | xxi |
| **IDI6126** | xxi | Bovine | Sutton Bonington, England | xxi |
| **IDI6127** | xxi | Bovine | Sutton Bonington, England | xxi |
| **IDI6128** | xxi | Bovine | Sutton Bonington, England | xxi |
| **IDI6129** | xxi | Bovine | Sutton Bonington, England | xxi |
| **IDI6130** | xxi | Bovine | Sutton Bonington, England | xxi |
| **IDI6131** | xxi | Bovine | Sutton Bonington, England | xxi |
| **IDI6132** | xxi | Bovine | Sutton Bonington, England | xxi |
| **IDI6133** | xxi | Bovine | Sutton Bonington, England | xxi |
| **IDI6134** | xxi | Bovine | Thirsk, England | xxi |
| **IDI6135** | xxi | Human | Tayside, Scotland | xxi |
| **IDI6136** | xxi | Human | Lothian, Scotland | xxi |
| **IDI6137** | xxi | Human | Scotland | xxi |
| **IDI6138** | xxi | Human | Scotland | xxi |
| **IDI6139** | xxi | Human | Scotland | xxi |
| **IDI6140** | xxi | Human | Scotland | xxi |
| **IDI6141** | xxi | Human | Scotland | xxi |
| **IDI6142** | xxi | Human | Scotland | xxi |
| **IDI6143** | xxi | Human | Scotland | xxi |
| **IDI6144** | xxi | Human | Scotland | xxi |
| **IDI6145** | xxi | Human | Scotland | xxi |
| **IDI6146** | xxi | Human | Scotland | xxi |
| **IDI6147** | xxi | Human | Denmark | xxi |
| **IDI6148** | xxi | Human | Denmark | xxi |
| **IDI6149** | xxi | Human | Denmark | xxi |
| **IDI6150** | xxi | Human | Denmark | xxi |
| **IDI6151** | xxi | Human | Denmark | xxi |
| **IDI6152** | xxi | Human | Denmark | xxi |
| **IDI6153** | xxi | Human | Denmark | xxi |
| **IDI6154** | xxi | Human | Denmark | xxi |
| **IDI6155** | xxi | Human | Denmark | xxi |
| **IDI6156** | xxi | Human | Denmark | xxi |
| **IDI6157** | xxi | Human | Denmark | xxi |
| **IDI6158** | xxi | Human | Denmark | xxi |
| **IDI6159** | xxi | Human | Denmark | xxi |
| **IDI6160** | xxi | Human | Denmark | xxi |
| **IDI6161** | xxi | Human | Denmark | xxi |
| **IDI6162** | xxi | Human | Denmark | xxi |
| **IDI6163** | xxi | Human | Denmark | xxi |
| **IDI6164** | xxi | Human | Denmark | xxi |
| **IDI6165** | xxi | Human | Denmark | xxi |
| **IDI6166** | xxi | Human | Denmark | xxi |

| | | | | |
|---|---|---|---|---|
| **IDI6167** | xxi | Human | Denmark | xxi |
| **IDI6168** | xxi | Human | France, Aix en provence | xxi |
| **IDI6170** | xxi | Human | France, Limoges | xxi |
| **IDI6171** | xxi | Human | France, Aix en provence | xxi |

DNA was isolated from the clinical samples above known to harbor *mecC* using the BD GeneOhm™ Lysis kit (Becton Dickinson) pursuant to manufacturer's instructions. The PCR reactions were prepared as follows: 0.2-0.7 µM each of SEQ ID NOs: 182, 183 and 187 0.3µM dNTPs (Roche), 4mM MgCl₂ (SIGMA), 2.8 units FASTSTART® Taq polymerase (Roche), 100mM Tris, pH 8.3 (EMD), 10mM KCl (LaboratoireMat), 5mM (NH₄)₂SO₄ (SIGMA), 0.15 mg/mL BSA (SIGMA) 4% trehalose (SIGMA).

Reactions were performed on the BD MAX® system, using the FAM channel.

The data indicate that 50 of 51 MRSA strains that harbored the *mecC* gene contained an MREJ type xxi sequence, thus providing evidence of high ubiquity of detection of *mecC* MRSA strains using the MREJ type xxi sequence.

## Claims

1. The nucleic acids of the sequence shown in Figure 1 or the complement of said sequence.

2. An oligonucleotide comprising the nucleic acids of SEQ ID NO.: 2, or the complement thereof, that specifically hybridizes to the sequence of claim 1.

3. A composition for the detection of methicillin-resistant *Staphylococcus aureus* (MRSA) comprising MREJ type xxi nucleic acids, said *S. aureus* comprising a *Staphylococcal* cassette chromosome *mec (SCCmec)* element including a *mecA* homolog (*mecA*_{LGA251})*,* said SCC*mec* cassette being inserted into *S. aureus* chromosomal DNA, thereby generating a polymorphic right extremity junction (MREJ) type xxi sequence that comprises polymorphic sequences from the right extremity and chromosomal DNA adjoining the polymorphic right extremity, said composition comprising:
a first amplification primer, said first amplification primer between 10 and 45 nucleotides in length, wherein said first amplification primer specifically hybridizes under standard PCR conditions to the polymorphic right extremity sequences of the MREJ type xxi nucleic acids of the sequence of claim 1 and is fully complementary to the polymorphic right extremity sequences of the MREJ type xxi nucleic acids of the sequence of claim 1, and
a second amplification primer, said second amplification primer between 10 and 45 nucleotides in length, wherein said second amplification primer specifically hybridizes under standard PCR conditions to *S. aureus* chromosomal sequences located within 1 kilobase from the insertion point of the SCC*mec* element into the chromosomal DNA, and wherein said first and second amplification primer together generate an amplicon of the right extremity junction of MREJ type xxi nucleic acids under the standard PCR conditions in the presence of MRSA comprising MREJ type xxi nucleic acids, wherein said amplicon comprises a MREJ type xxi-specific sequence.

4. The composition of Claim 3, further comprising a probe, wherein said probe specifically hybridizes to the amplicon of the MREJ type xxi nucleic acids under the standard PCR conditions.

5. The composition of Claim 3, further comprising a primer pair that specifically hybridizes within and is capable of amplification of a *mecA* sequence within at least one of SEQ ID NOs:156, 157 or 158, respectively.

6. The composition of Claim 3, further comprising one or more additional amplification primers, wherein said one or more additional amplification primers are between 10 and 45 nucleotides in length, and wherein said one or more additional amplification primers specifically hybridize to one or more polymorphic SCC*mec* right extremity sequences from an MREJ type i to xx MRSA.

7. The composition of Claim 6, wherein said one or more polymorphic SCC*mec* right extremity sequence is selected from the group consisting of SEQ ID NOs: 5 to 29.

8. The composition of Claim 3, wherein the first amplification primer is at least 80% identical to SEQ ID NO:2.

9. The composition of Claim 8, wherein the second amplification primer is at least 80% identical to SEQ ID NO:3.

10. The composition of Claim 9, further comprising a probe, wherein the probe is at least 80% identical to SEQ ID NO:4 or 82.

11. A method for the detection of methicillin-resistant *Staphylococcus aureus* (MRSA) comprising MREJ type xxi nucleic acids, said *S. aureus* comprising a *Staphylococcal* cassette chromosome mec (SCC*mec*) element including a *mecA* homolog (*mecA*_{LGA251})*,* said SCC*mec* cassette being inserted into *S. aureus* chromosomal DNA, thereby generating a polymorphic right extremity junction (MREJ) type xxi sequence that comprises polymorphic sequences from the right extremity and chromosomal DNA adjoining the polymorphic right extremity, said method comprising:
contacting a test sample with a first amplification primer, said first amplification primer between 10 and 45 nucleotides in length, wherein said first amplification primer specifically hybridizes under standard PCR conditions to the polymorphic right extremity sequences of the MREJ type xxi sequence of the sequence of claim 1;
contacting the sample with a second amplification primer between 10 and 45 nucleotides in length, wherein said second amplification primer hybridizes under the standard PCR conditions to the *orfX* gene of *S. aureus,* and wherein said first and second amplification primer together generate an amplicon of the SCC*mec* right extremity junction (MREJ) xxi nucleic acids under the standard PCR conditions in the presence of MRSA comprising MREJ type xxi nucleic acids, wherein said amplicon comprises a MREJ type xxi-specific sequence; and
determining whether an amplicon of the MREJ type xxi-specific nucleic acids is generated following the contacting step.

12. The method of Claim 11, wherein said contacting step further comprises contacting the sample with one or more additional amplification primers, wherein said one or more additional amplification primers are between 10 and 45 nucleotides in length, and wherein said one or more additional amplification primers specifically hybridizes to one or more polymorphic SCC*mec* right extremity sequence from an MREJ type i to xx MRSA.

13. The method of Claim 11, wherein said contacting step further comprises contacting the sample with one or more additional amplification primer pairs, wherein said one or more additional amplification primer pairs specifically hybridizes to, and is capable of generating an amplicon of, *nuc* sequences under the standard PCR conditions.

14. The method of Claim 11, wherein said contacting step further comprises contacting the sample with one or more additional amplification primer pairs, wherein said one or more additional amplification primer pairs specifically hybridizes to, and is capable of generating an amplicon of, *mecA* and/or *mecC* sequences under the standard PCR conditions.

15. The method of claim 11, wherein said step of determining whether an amplicon of the MREJ type xxi-specific nucleic acids is generated includes identification of an MREJ type xxi-specific sequence.

## Patentansprüche

1. Nucleinsäuren der in Abbildung 1 gezeigten Sequenz oder Komplement der Sequenz.

2. Oligonucleotid, umfassend die Nucleinsäuren von SEQ ID NO:2 oder das Komplement davon, das spezifisch an die Sequenz nach Anspruch 1 hybridisiert.

3. Zusammensetzung zum Nachweis von Methicillin-resistentem *Staphylococcus aureus* (MRSA), umfassend Nucleinsäuren vom MREJ-Typ xxi, wobei der *S*. *aureus* ein Staphylococcen-Kassettenchromosom *mec* (SCC*mec*)-Element (*Staphylococcal* cassette chromosome *mec* (SCC*mec*) element) einschließlich eines *mecA-*Homologs (*mecA*_{LGA251}) umfasst, wobei die SCCmec-Kassette in chromosomale DNA von *S*. *aureus* inseriert ist, wodurch eine polymorphe Verbindungs-Sequenz des rechten äußeren Endes vom MREJ-Typ xxi (polymorphic right extremity junction (MREJ) type xxi sequence) erzeugt wird, die polymorphe Sequenzen des rechten äußeren Endes und der chromosomalen DNA umfasst, die an das polymorphe rechte äußere Ende angrenzt, wobei die Zusammensetzung umfasst:
einen ersten Amplifikationsprimer, wobei der erste Amplifikationsprimer zwischen 10 und 45 Nucleotide lang ist, wobei der erste Amplifikationsprimer unter Standard-PCR-Bedingungen mit den polymorphen Sequenzen des rechten äußeren Endes der Nucleinsäuren vom MREJ-Typ xxi der Sequenz nach Anspruch 1 spezifisch hybridisiert und vollständig komplementär zu den polymorphen Sequenzen des rechten äußeren Endes der Nucleinsäuren vom MREJ-Typ xxi der Sequenz nach Anspruch 1 ist, und
einen zweiten Amplifikationsprimer, wobei der zweite Amplifikationsprimer zwischen 10 und 45 Nucleotide lang ist, wobei der zweite Amplifikationsprimer unter Standard-PCR-Bedingungen mit chromosomalen Sequenzen von *S*. *aureus* spezifisch hybridisiert, die sich innerhalb 1 Kilobase vom Insertionspunkt des SCCmec-Elements in der chromosomalen DNA befinden, und wobei der erste und der zweite Amplifikationsprimer zusammen ein Amplicon der Verbindungs-Nucleinsäuren des rechten äußeren Endes vom MREJ-Typ xxi unter den Standard PCR-Bedingungen in Anwesenheit des MRSA erzeugen, der Nucleinsäuren vom MREJ-Typ xxi umfasst, wobei das Amplicon eine MREJ-Typ xxi-spezifische Sequenz umfasst.

4. Zusammensetzung nach Anspruch 3, des Weiteren umfassend eine Sonde, wobei die Sonde unter den Standard-PCR-Bedingungen mit dem Amplicon der Nucleinsäuren vom MREJ-Typ xxi spezifisch hybridisiert.

5. Zusammensetzung nach Anspruch 3, des Weiteren umfassend ein Primerpaar, das innerhalb einer mecA-Sequenz innerhalb mindestens einer aus SEQ ID NO:s 156, 157 bzw. 158 spezifisch hybridisiert und in der Lage ist, diese zu amplifizieren.

6. Zusammensetzung nach Anspruch 3, des Weiteren umfassend einen oder mehrere zusätzliche Amplifikationsprimer, wobei der eine oder die mehreren zusätzlichen Amplifikationsprimer zwischen 10 und 45 Nucleotide lang ist/sind, und wobei der eine oder die mehreren zusätzlichen Amplifikationsprimer mit einer oder mehreren polymorphen SCCmec-Sequenzen des rechten äußeren Endes eines MRSA vom MREJ-Typ i bis xx spezifisch hybridisiert/hybridisieren.

7. Zusammensetzung nach Anspruch 6, wobei die eine oder die mehreren polymorphen SCCmec-Sequenzen des rechten äußeren Endes ausgewählt ist/sind aus der Gruppe bestehend aus SEQ ID NO:s 5 bis 29.

8. Zusammensetzung nach Anspruch 3, wobei der erste Amplifikationsprimer mindestens 80% identisch mit SEQ ID NO:2 ist.

9. Zusammensetzung nach Anspruch 8, wobei der zweite Amplifikationsprimer mindestens 80% identisch mit SEQ ID NO:3 ist.

10. Zusammensetzung nach Anspruch 9, des Weiteren umfassend eine Sonde, wobei die Sonde mindestens 80% identisch mit SEQ ID NO:4 oder 82 ist.

11. Verfahren zum Nachweis von Methicillin-resistentem *Staphylococcus aureus* (MRSA), umfassend Nucleinsäuren vom MREJ-Typ xxi, wobei der *S*. *aureus* ein Staphylococcen-Kassettenchromosom *mec* (SCC*mec*)-Element (*Staphylococcal* cassette chromosome *mec* (SCC*mec*) element) einschließlich eines *mecA*-Homologs (*mecA*_{LGA251}) umfasst, wobei die SCC*mec*-Kassette in chromosomale DNA von *S*. *aureus* inseriert ist, wodurch eine polymorphe Verbindungs-Sequenz des rechten äußeren Endes vom MREJ-Typ xxi (polymorphic right extremity junction (MREJ) type xxi sequence) erzeugt wird, die polymorphe Sequenzen des rechten äußeren Endes und der chromosomalen DNA umfasst, die an das polymorphe rechte äußere Ende angrenzt, wobei das Verfahren umfasst:
Inkontaktbringen einer Testprobe mit einem ersten Amplifikationsprimer, wobei der erste Amplifikationsprimer zwischen 10 und 45 Nucleotide lang ist, wobei der erste Amplifikationsprimer unter Standard-PCR-Bedingungen mit den polymorphen Sequenzen des rechten äußeren Endes der Sequenz vom MREJ-Typ xxi der Sequenz nach Anspruch 1 spezifisch hybridisiert;
Inkontaktbringen der Probe mit einem zweiten Amplifikationsprimer, der zwischen 10 und 45 Nucleotide lang ist, wobei der zweite Amplifikationsprimer unter den Standard-PCR-Bedingungen an das *orfX-Gen* von *S*. *aureus* hybridisiert, und wobei der erste und der zweite Amplifikationsprimer zusammen ein Amplicon der SCCmec-Verbindungs-Nucleinsäuren des rechten äußeren Endes vom (MREJ)-Typ xxi unter Standard-PCR-Bedingungen in Anwesenheit des MRSA erzeugen, der Nucleinsäuren vom MREJ-Typ xxi umfasst, wobei das Amplicon eine MREJ-Typ xxi-spezifische Sequenz umfasst; und
Bestimmen, ob ein Amplicon der MREJ-Typ xxi-spezifischen Nucleinsäuren nach dem Schritt des Inkontaktbringens erzeugt wird.

12. Verfahren nach Anspruch 11, wobei der Schritt des Inkontaktbringens des Weiteren das Inkontaktbringen der Probe mit einem oder mehreren zusätzlichen Amplifikationsprimern umfasst, wobei der eine oder die mehreren zusätzlichen Amplifikationsprimer zwischen 10 und 45 Nucleotide lang ist/sind, und wobei der eine oder die mehreren zusätzlichen Amplifikationsprimer mit einer oder mehreren polymorphen SCCmec-Sequenzen des rechten äußeren Endes eines MRSA vom MREJ-Typ i bis xx spezifisch hybridisiert/hybridisieren.

13. Verfahren nach Anspruch 11, wobei der Schritt des Inkontaktbringens des Weiteren das Inkontaktbringen der Probe mit einem oder mehreren zusätzlichen Amplifikationsprimerpaaren umfasst, wobei das eine oder die mehreren zusätzlichen Amplifikationsprimerpaare unter den Standard-PCR-Bedingungen mit *nuc*-Sequenzen spezifisch hybridisiert/hybridisieren und in der Lage ist/sind, ein Amplicon von diesen zu erzeugen.

14. Verfahren nach Anspruch 11, wobei der Schritt des Inkontaktbringens des Weiteren das Inkontaktbringen der Probe mit einem oder mehreren zusätzlichen Amplifikationsprimerpaaren umfasst, wobei das eine oder die mehreren zusätzlichen Amplifikationsprimerpaare unter den Standard-PCR-Bedingungen mit *mecA-* und/oder *mecC*-Sequenzen spezifisch hybridisiert/hybridisieren und in der Lage ist/sind, ein Amplicon von diesen zu erzeugen.

15. Verfahren nach Anspruch 11, wobei der Schritt des Bestimmens, ob ein Amplicon von MREJ-Typ xxi-spezifischen Nucleinsäuren erzeugt wurde, die Identifizierung einer MREJ-Typ xxi-spezifischen Sequenz umfasst.

## Revendications

1. Acides nucléiques ayant la séquence indiquée sur la Figure 1 ou le complément de ladite séquence.

2. Oligonucléotide comprenant les acides nucléiques de la SEQ ID NO : 2 ou son complément, qui s'hybride spécifiquement à la séquence de la revendication 1.

3. Composition pour la détection de Staphylococcus *aureus* résistant à la méthicilline (SARM) comprenant des acides nucléiques de type xxi de MREJ, ledit *S. aureus* comprenant un élément de chromosome de cassette staphylococcique *mec* (SCCmec) contenant un homologue de *mecA* (*mecA*_{LGA251})*,* ladite cassette SCCmec étant insérée dans l'ADN chromosomique de S. *aureus,* en générant ainsi une séquence de type xxi de jonction d'extrémité droite (MREJ) polymorphe qui comprend des séquences polymorphes provenant de l'extrémité droite et de l'ADN chromosomique joignant l'extrémité droite polymorphe, ladite composition comprenant :
une première amorce d'amplification, ladite première amorce d'amplification ayant une longueur comprise entre 10 et 45 nucléotides, laquelle première amorce d'amplification s'hybride spécifiquement, dans des conditions de PCR standard, aux séquences polymorphes d'extrémité droite des acides nucléiques de type xxi de MREJ de la séquence de la revendication 1 et est entièrement complémentaire des séquences polymorphes d'extrémité droite des acides nucléiques de type xxi de MREJ de la séquence de la revendication 1, et
une deuxième amorce d'amplification, ladite deuxième amorce d'amplification ayant une longueur comprise entre 10 et 45 nucléotides, laquelle deuxième amorce d'amplification s'hybride spécifiquement, dans des conditions de PCR standard, aux séquences chromosomiques de S. *aureus* situées dans l'intervalle de 1 kilobase à partir du point d'insertion de l'élément SCCmec dans l'ADN chromosomique, et
dans laquelle les première et deuxième amorces d'amplification génèrent ensemble un amplicon de la jonction d'extrémité droite des acides nucléiques de type xxi de MREJ dans les conditions de PCR standard en présence de SARM comprenant les acides nucléiques de type xxi de MREJ, et dans laquelle ledit amplicon comprend une séquence spécifique du type xxi de MREJ.

4. Composition selon la revendication 3, comprenant en outre une sonde, dans laquelle ladite sonde s'hybride spécifiquement à l'amplicon des acides nucléiques de type xxi de MREJ dans les conditions de PCR standard.

5. Composition selon la revendication 3, comprenant en outre une paire d'amorces qui s'hybride spécifiquement à l'intérieur de et est capable d'amplifier une séquence de *mecA* à l'intérieur d'au moins l'une des SEQ ID NO : 156, 157 et 158 respectivement.

6. Composition selon la revendication 3, comprenant en outre une ou plusieurs amorces d'amplification additionnelles, dans laquelle lesdites une ou plusieurs amorces d'amplification additionnelles ont une longueur comprise entre 10 et 45 nucléotides, et dans laquelle lesdites une ou plusieurs amorces d'amplification additionnelles s'hybrident spécifiquement à une ou plusieurs séquences polymorphes d'extrémité droite de SCC*mec* provenant d'un SARM de types i à xx de MREJ.

7. Composition selon la revendication 6, dans laquelle lesdites une ou plusieurs séquences polymorphes d'extrémité droite de SCC*mec* sont choisies dans le groupe constitué par les SEQ ID NO : 5 à 29.

8. Composition selon la revendication 3, dans laquelle la première amorce d'amplification est identique à au moins 80 % à la SEQ ID NO : 2.

9. Composition selon la revendication 8, dans laquelle la deuxième amorce d'amplification est identique à au moins 80 % à la SEQ ID NO : 3.

10. Composition selon la revendication 9, comprenant en outre une sonde, dans laquelle la sonde est identique à au moins 80 % à la SEQ ID NO : 4 ou 82.

11. Méthode pour la détection de *Staphylococcus aureus* résistant à la méthicilline (SARM) comprenant des acides nucléiques de type xxi de MREJ, ledit *S. aureus* comprenant un élément de chromosome de cassette staphylococcique *mec* (SCCmec) contenant un homologue de *mecA* (*mecA*_{LGA251})*,* ladite cassette SCCmec étant insérée dans l'ADN chromosomique de S. *aureus,* en générant ainsi une séquence de type xxi de jonction d'extrémité droite (MREJ) polymorphe qui comprend des séquences polymorphes provenant de l'extrémité droite et de l'ADN chromosomique joignant l'extrémité droite polymorphe, ladite méthode comprenant :
la mise en contact d'un échantillon de test avec une première amorce d'amplification, ladite première amorce d'amplification ayant une longueur comprise entre 10 et 45 nucléotides, dans laquelle ladite première amorce d'amplification s'hybride spécifiquement, dans des conditions de PCR standard, aux séquences polymorphes d'extrémité droite de la séquence de type xxi de MREJ de la séquence de la revendication 1 ; la mise en contact de l'échantillon avec une deuxième amorce d'amplification ayant une longueur comprise entre 10 et 45 nucléotides, dans laquelle ladite deuxième amorce d'amplification s'hybride, dans les conditions de PCR standard, au gène *orfX* de *S*. *aureus,* et dans laquelle lesdites première et deuxième amorces d'amplification génèrent ensemble un amplicon des acides nucléiques xxi de jonction d'extrémité droite (MREJ) de SCCmec dans les conditions de PCR standard en présence de SARM comprenant des acides nucléiques de type xxi de MREJ, dans laquelle ledit amplicon comprend une séquence spécifique du type xxi de MREJ ; et
la détermination qu'un amplicon des acides nucléiques spécifiques du type xxi de MREJ est ou non généré après l'étape de mise en contact.

12. Méthode selon la revendication 11, dans laquelle ladite étape de mise en contact comprend en outre la mise en contact de l'échantillon avec une ou plusieurs amorces d'amplification additionnelles, dans laquelle lesdites une ou plusieurs amorces d'amplification additionnelles ont une longueur comprise entre 10 et 45 nucléotides, et dans laquelle lesdites une ou plusieurs amorces d'amplification additionnelles s'hybrident spécifiquement à une ou plusieurs séquences polymorphes d'extrémité droite de SCC*mec* provenant d'un SARM de types i à xx de MREJ.

13. Méthode selon la revendication 11, dans laquelle ladite étape de mise en contact comprend en outre la mise en contact de l'échantillon avec une ou plusieurs paires d'amorces d'amplification additionnelles, dans laquelle lesdites une ou plusieurs paires d'amorces d'amplification additionnelles s'hybrident spécifiquement à des, et sont capables de générer un amplicon de, séquences de *nuc* dans les conditions de PCR standard.

14. Méthode selon la revendication 11, dans laquelle ladite étape de mise en contact comprend en outre la mise en contact de l'échantillon avec une ou plusieurs paires d'amorces d'amplification additionnelles, dans laquelle lesdites une ou plusieurs paires d'amorces d'amplification additionnelles s'hybrident spécifiquement à des, et sont capables de générer un amplicon de, séquences de *mecA* et/ou *mecC* dans les conditions de PCR standard.

15. Méthode selon la revendication 11, dans laquelle ladite étape de détermination qu'un amplicon des acides nucléiques spécifiques du type xxi de MREJ est ou non généré comprend l'identification d'une séquence spécifique du type xxi de MREJ.


### SEQUENCE LISTING

<110> GENEOHM SCIENCES CANADA, INC.
<120> SEQUENCES FOR DETECTION AND
   IDENTIFICATION OF METHICILLIN-RESISTANT STAPHYLOCOCCUS
AUREUS (MRSA) OF
MREJ TYPE XXI
<130> X3286 EP S3
<140> EP 13 77 2940.6
   <141> 2013-03-14
<150> PCT/IB2013/000900
   <151> 2013-03-14
<150> 61/621368
   <151> 2012-04-06
<160> 188
<170> FastSEQ for Windows Version 4.0
<210> 1
   <211> 772
   <212> DNA
   <213> Staphylococcus aureus
<220>
   <223> n= c, a, t, or g
<220>
   <221> misc_feature
   <222> 4, 6
   <223> n = A,T,C or G
<400> 1
   tcangnggga tcaaacggcc tgcacaagga cgtcttacaa cgcagtaact acgcactatc 60
   attcagcaaa atgacatttc cacatcaaat gatgcgggtt gtgttaattg agcaagtgta 120
   tagagcgttt aagattatgc gcggagaagc gtatcacaaa tgatgcggtt tttttaacct 180
   ctttacgtat gaggtttatg agaattgccg ttatgttttg cgagagttat caatcttttt 240
   gatagtaaga aagtacatag aaactaaaag agtattttta tctacaatag catttataat 300
   ttattctatt attgtatact tattttaatt attagtatca ttgctgagat gttacttgat 360
   attctatgtc tattttttag gaaattctat actattaaaa ttatggtatt ttatacgcaa 420
   taaaggacta atctatttta tacagattag tcctttattg tagtctttaa aaactagtta 480
   ctcattaata ttttttagta caatttcagc aacctcactt actattttgt cattaggttt 540
   accatctttt ctatctttat ttgtaaatat caccagaatt ataggtttat cttgtccatc 600
   tggataaaca aagcgaacat cgtttcttga accgtatgtt agtgcttgac cgctcttatc 660
   cataacttta aagtttgaag gtgcaccatc cttaattaat gtatcgccac ttttattttt 720
   gaacattaga ttaagtaaga aatctttgtt tgctttgcta agatctccat ca         772
<210> 2
   <211> 21
   <212> DNA
   <213> Artificial Sequence
<220>
   <223> synthetic oligonucleotide
<400> 2
   cggcaattct cataaacctc a 21
<210> 3
   <211> 19
   <212> DNA
   <213> Artificial Sequence
<220>
   <223> synthetic oligonucleotide
<400> 3
   ggatcaaacg gcctgcaca 19
<210> 4
   <211> 24
   <212> DNA
   <213> Artificial Sequence
<220>
   <223> synthetic oligonucleotide
<400> 4
   tagttactgc gttgtaagac gtcc 24
<210> 5
   <211> 3050
   <212> DNA
   <213> Staphylococcus aureus
<220>
   <223> synthetic oligonucleotide
<400> 5
   tcgtgccatt gatgcagagg gacatacatt agatatttgg ttgcgtaagc aacgagataa 60
   tcattcagca tatgcgttta tcaaacgtct cattaaacaa tttggtaaac ctcaaaaggt 120
   aattacagat caggcacctt caacgaaggt agcaatggct aaagtaatta aagcttttaa 180
   acttaaacct gactgtcatt gtacatcgaa atatctgaat aacctcattg agcaagatca 240
   ccgtcatatt aaagtaagaa agacaaggta tcaaagtatc aatacagcaa agaatacttt 300
   aaaaggtatt gaatgtattt acgctctata taaaaagaac cgcaggtctc ttcagatcta 360
   cggattttcg ccatgccacg aaattagcat catgctagca agttaagcga acactgacat 420
   gataaattag tggttagcta tattttttta ctttgcaaca gaaccgaaaa taatctcttc 480
   aatttatttt tatatgaatc ctgtgactca atgattgtaa tatctaaaga tttcagttca 540
   tcatagacaa tgttcttttc aacatttttt atagcaaatt gattaaataa attctctaat 600
   ttctcccgtt tgatttcact accatagatt atattatcat tgatatagtc aatgaataat 660
   gacaaattat cactcataac agtcccaacc cctttatttt gatagactaa ttatcttcat 720
   cattgtaaaa caaattacac cctttaaatt taactcaact taaatatcga caaattaaaa 780
   aacaataaaa ttacttgaat attattcata atatattaac aactttatta tactgctctt 840
   tatatataaa atcattaata attaaacaag ccttaaaata tttaactttt ttgtgattat 900
   tacacattat cttatctgct ctttatcacc ataaaaatag aaaaaacaag attcctaaag 960
   aatataggaa tcttgtttca gactgtggac aaactgattt tttatcagtt agcttattta 1020
   gaaagtttta tttaaattac agtttctatt tttattagat cacaatttta ttttagctct 1080
   tgttcaagta atcatttttc gccaaaaact ttatactgaa tagcttctac attaaatact 1140
   ttgtcaatga gatcatctac atctttaaat tcagaataat ttgcatatgg atctataaaa 1200
   taaaattgtg gttctttacc ggaaacatta aatattctta atattaaata tttctgctta 1260
   tattctttca tagcaaacat ttcatttagc gacataaaaa atggttcctc aatactagaa 1320
   gatgtagatg ttttaatttc aataaatttt tctacagctt tatctgtatt tgttggatca 1380
   aaagctacta aatcatagcc atgaccgtgt tgagagcctg gattatcatt taaaatattc 1440
   ctaaactgtt ctttcttatc ttcgtctatt ttattatcaa ttagctcatt aaagtaattt 1500
   agcgctaatt tttctccaac tttaccggtt aatttattct ctttatttga tttttcaatt 1560
   tctgaatcat ttttagtagt ctttgataca ccttttttat attttggaat tattccttta 1620
   ggtgcttcca cttccttgag tgtcttatct ttttgtgctg ttctaatttc ttcaatttcg 1680
   ctgtcttcct gtatttcgtc tatgctattg accaagctat cataggatgt ttttgtaact 1740
   tttgaagcta attcattaaa tagttctaaa aatttcttta aatcctctag catatcttct 1800
   tctgtgaatc cttcattcaa atcataatat ttgaatctta ttgatccatg agaatatcct 1860
   gatggataat cattttttaa atcataagat gaatctttat tttctgcgta ataaaatctt 1920
   ccagtattaa attcatttga tgtaatatat ttattgagtt cggaagataa agttaatgct 1980
   ctttgttttg cagcattttt atcccgcgga aacatatcac ttatctttga ccatccttga 2040
   ttcaaagata agtatatgcc ttctccttcc ggatgaaaaa gatataccaa ataatatcca 2100
   tcctttgttt cttttgttat attctcatca tatattgaaa tccaaggaac tttactatag 2160
   ttcccagtag caaccttccc tacaactgaa tatttatctt cttttatatg cacttttaac 2220
   tgcttgggta acttatcatg gactaaagtt ttatatagat cacctttatc ccaatcagat 2280
   tttttaacta cattattggt acgtttctct ttaattaatt taaggacctg cataaagttg 2340
   tctatcattt gaaattccct cctattataa aatatattat gtctcatttt cttcaatatg 2400
   tacttattta tattttaccg taatttacta tatttagttg cagaaagaat tttctcaaag 2460
   ctagaacttt gcttcactat aagtattcag tataaagaat atttcgctat tatttacttg 2520
   aaatgaaaga ctgcggaggc taactatgtc aaaaatcatg aacctcatta cttatgataa 2580
   gcttctcctc gcataatctt aaatgctctg tacacttgtt caattaacac aacccgcatc 2640
   atttgatgtg ggaatgtcat tttgctgaat gatagtgcgt agttactgcg ttgtaagacg 2700
   tccttgtgca ggccgtttga tccgccaatg acgaaaacaa agtcgctttg cccttgggtc 2760
   atgcgttggt tcaattcttg ggccaatcct tcggaagata gcatctttcc ttgtatttct 2820
   aatgtaatga ctgtggattg tggtttgatt ttggctagta ttcgttggcc ttctttttct 2880
   tttacttgct caatttcttt gtcactcata ttttctggtg ctttttcgtc tggaacttct 2940
   atgatgtcta tcttggtgta tgggcctaaa cgtttttcat attctgctat ggcttgcttc 3000
   caatatttct cttttagttt ccctacagct aaaatggtga ttttcatgtc           3050
<210> 6
   <211> 3050
   <212> DNA
   <213> Staphylococcus aureus
<400> 6
   acctcattga gcaagatcac cgtcatatta aagtaagaaa gacaaggtat caaagtatca 60
   atacagcaaa gaatacttta aaaggtattg aatgtattta cgctctatat aaaaagaacc 120
   gcaggtctct tcagatctac ggattttcgc catgccacga aattagcatc atgctagcaa 180
   gttaagcgaa cactgacatg ataaattagt ggttagctat atttttttac tttgcaacag 240
   aaccgaaaat aatctcttca atttattttt atatgaatcc tgtgactcaa tgattgtaat 300
   atctaaagat ttcagttcat catagacaat gttcttttca acatttttta tagcaaattg 360
   attaaataaa ttctctaatt tctcccgttt gatttcacta ccatagatta tattatcatt 420
   gatatagtca atgaataatg acaaattatc actcataaca gtcccaaccc ctttcttttg 480
   atagactaat tatcttcatc attgtaaaac aaattacacc ctttaaattt aactcaactt 540
   aaatatcgac aaattaaaaa acaataaaat tacttgaata ttattcataa tatattaaca 600
   actttattat actgctcttt atatataaaa tcattaataa ttaaacaagc cttaaaatat 660
   ttaacttttt tgtgattatt acacattatc ttatctgctc tttatcacca taaaaataga 720
   aaaaacaaga ttcctaaaga atataggaat cttgtttcag actgtggaca aactgatttt 780
   ttatcagtta gcttatttag aaagttttat ttaaattaca gtttctattt ttattagatc 840
   acaattttat tttagctctt gttcaagtaa tcatttttcg ccaaaaactt tatactgaat 900
   agcttctaca ttaaatactt tgtcaatgag atcatctaca tctttaaatt cagaataatt 960
   tgcatatgga tctataaaat aaaattgtgg ttctttaccg gaaacattaa atattcttaa 1020
   tattaaatat ttctgcttat attctttcat agcaaacatt tcatttagcg acataaaaaa 1080
   tggttcctca atactagaag atgtagatgt tttaatttca ataaattttt ctacagcttt 1140
   atctgtattt gttggatcaa aagctactaa atcatagcca tgaccgtgtt gagagcctgg 1200
   attatcattt aaaatattcc taaactgttc tttcttatct tcgtctattt tattatcaat 1260
   tagctcatta aagtaattta gcgctaattt ttctccaact ttaccggtta atttattctc 1320
   tttatttgat ttttcaattt ctgaatcatt tttagtagtc tttgatacac cttttttata 1380
   ttttggaatt attcctttag gtgcttccac ttccttgagt gtcttatctt tttgtgctgt 1440
   tctaatttct tcaatttcgc tgtcttcctg tatttcgtct atgctattga ccaagctatc 1500
   ataggatgtt tttgtaactt ttgaagctaa ttcattaaat agttctaaaa atttctttaa 1560
   atcctctagc atatcttctt ctgtgaatcc ttcattcaaa tcataatatt tgaatcttat 1620
   tgatccatga gaatatcctg atggataatc attttttaaa tcataagatg aatctttatt 1680
   ttctgcgtaa taaaatcttc cagtattaaa ttcatttgat gtaatatatt tattgagttc 1740
   ggaagataaa gttaatgctc tttgttttgc agcattttta tcccgcggaa acatatcact 1800
   tatctttgac catccttgat tcaaagataa gtatatgcct tctccttccg gatgaaaaag 1860
   atataccaaa taatatccat cctttgtttc ttttgttata ttctcatcat atattgaaat 1920
   ccaaggaact ttactatagt tcccagtagc aaccttccct acaactgaat atttatcttc 1980
   ttttatatgc acttttaact gcttgggtaa cttatcatgg actaaagttt tatatagatc 2040
   acctttatcc caatcagatt ttttaactac attattggta cgtttctctt taattaattt 2100
   aaggacctgc ataaagttgt ctatcatttg aaattccctc ctattataaa atatattatg 2160
   tctcattttc ttcaatatgt acttatttat attttaccgt aatttactat atttagttgc 2220
   agaaagaatt ttctcaaagc tagaactttg cttcactata agtattcagt ataaagaata 2280
   tttcgctatt atttacttga aatgaaagac tgcggaggct aactatgtca aaaatcatga 2340
   acctcattac ttatgataag cttcttaaaa acataacagc aattcacata aacctcatat 2400
   gttctgatac attcaaaatc cctttatgaa gcggctgaaa aaaccgcatc atttatgata 2460
   tgcttctcca cgcataatct taaatgctct atacacttgc tcaattaaca caacccgcat 2520
   catttgatgt gggaatgtca ttttgctgaa tgatagtgcg tagttactgc gttgtaagac 2580
   gtccttgtgc aggccgtttg atccgccaat gacgaataca aagtcgcttt gcccttgggt 2640
   catgcgttgg ttcaattctt gggccaatcc ttcggaagat agcatctttc cttgtatttc 2700
   taatgtaatg actgtggatt gtggtttaat tttggctagt attcgttggc cttctttttc 2760
   ttttacttgc tcaatttctt tgtcgctcat attttctggt gctttttcgt ctggaacttc 2820
   tatgatgtct atcttggtgt atgggcctaa acgtttttca tattctgcta tggcttgctt 2880
   ccaatatttc tcttttagtt tccctacagc taaaatggtg attttcatgt cgtttggtcc 2940
   tccaaattgt tatcaacttt ccagttatcc acaagttatt aacttgttca cactgttccc 3000
   tcttattata ccaatatttt ttgcagtttt tgatattttc ctgacattta            3050
<210> 7
   <211> 1256
   <212> DNA
   <213> Staphylococcus aureus
<400> 7
   ttcagaaaaa tgattaatgt gtttcaataa aatctctcct tctttgtgaa catattcatt 60
   tttatactaa ttaatataat ttccaaaaaa gtttctgttt aaaagtgaaa aatattattt 120
   accgtttgac ttaaatcttc aatatatagg tgtttatatg tatcattttg cgccaatttg 180
   aataaacggg aatcaagtct gtttctgagt ttatttcaac tttcttatag taaacattgt 240
   cttaatatga tgaacttcaa taaaactttc cctatgcccc ataaaatttt ctcaaaatca 300
   aaaataacat accttacaac ttttaccgtc gatatcaatt gctcttttct taatttagga 360
   ttgctttcaa attttgtact ataacgtgaa actacttttc cttctttata attaaaattt 420
   actaattcac aatcattttt acttccattt acaaaaacat ccactgtttc taacacaaaa 480
   tctaataaac ttccttttat taatcgtagg cattgtatat ttcctttcat tctttcttga 540
   ttccattagt ttaaatttaa aatttcatcc atcaatttct taatttaatt gtagttccat 600
   aatcaatata atttgtacag ttattatata ttctagatca tcaatagttg aaaaatggtt 660
   tattaaacac tctataaaca tcgtatgata ttgcaaggta taatccaata tttcatatat 720
   gtaattcctc cacatctcat taaattttta aattatacac aacctaattt ttagttttat 780
   ttatgatacg cttctccacg cataatctta aatgctctgt acacttgttc aattaacaca 840
   acccgcatca tttgatgtgg gaatgtcatt ttgctgaatg atagtgcgta gttactgcgt 900
   tgtaagacgt ccttgtgcag gccgtttgat ccgccaatga cgaatacaaa gtcgctttgc 960
   ccttgggtca tgcgttggtt caattcttgg gccaatcctt cggaagatag catctttcct 1020
   tgtatttcta atgtaatgac tgttgattgt ggtttgattt tggctagtat tcgttggcct 1080
   tctttttctt ttacttgctc aatttctttg tcgctcatat tttctggtgc tttttcgtct 1140
   ggaacttcta tgatgtctat cttggtgtat gggcctaaac gtttttcata ttctgctatg 1200
   gcttgcttcc aatatttctc ttttagtttc cctacagcta aaatggtgat tttcat     1256
<210> 8
   <211> 2122
   <212> DNA
   <213> Staphylococcus aureus
<400> 8
   ggaaactaaa agagaaatat tggaagcaag ccatagcaga atatgaaaaa cgtttaggcc 60
   catacaccaa gatagacatc atagaagttc cagacgaaaa agcaccagaa aatatgagcg 120
   acaaagaaat tgagcaagta aaagaaaaag aaggccaacg aatactagcc aaaatcaaac 180
   cacaatcaac agtcattaca ttagaaatac aaggaaagat gctatcttcc gaaggattgg 240
   cccaagaatt gaaccaacgc atgacccaag ggcaaagcga ctttgtattc gtcattggcg 300
   gatcaaacgg cctgcacaag gacgtcttac aacgcagtaa ctacgcacta tcattcagca 360
   aaatgacatt cccacatcaa atgatgcggg ttgtgttaat tgaacaagtg tacagagcat 420
   ttaagattat gcgtggagaa gcgtaccaca aatgatgcgg ttttttatcc agttttttgt 480
   ttaatgaaca aggtaaatta cgagataata tttgaagaaa acaataaagt agagatggat 540
   ttccatatcc tctttagtag cggtttttat ctgtaaggtt tattaataat taaataaata 600
   ggcgggatag ttatatatag cttattaatg aaagaatatg attattaatt tagtattata 660
   ttttaatatt aaaaagaaga tatgaaataa ttattcatac cttccacctt acaataatta 720
   gttttcaatc gaatattaag attattagta gtcttaaaag ttaagacttc cttatattaa 780
   tgacctaatt tattatttgc ctcatgaatt atctttttat ttctttgata tgtcccaaac 840
   cacatcgtga tatacactac aataaatatt atgatgaaac taataatatt ctcaaagttc 900
   agatggaacc aacctgctag aatagcgagt gggaagaata ggattatcat caatataaag 960
   tgaactacag tctgttttgt tatactccaa tcggtatctg taaatatcaa attaccataa 1020
   gtaaacaaaa ttccaatcaa tgcccatagt gctacacata ttagcataat aaccgcttca 1080
   ttaaagtttt cataataaat tttacccata aaagaatctg gatatagtgg tacatattta 1140
   tcccttgaaa aaaataagtg aagtaatgac agaaatcata agaccagtga acgcaccttt 1200
   ttgaacagcg tggaataatt ttttcatagt gagatggacc attccatttg tttctaactt 1260
   caagtgatca atgtaattta gattgataat ttctgatttt gaaatacgca cgaatattga 1320
   accgacaagc tcttcaattt ggtaaagtcg ctgataaagt tttaaagctt tattattcat 1380
   tgttatcgca tacctgttta tcttctacta tgaactgtgc aatttgttct agatcaattg 1440
   ggtaaacatg atggttctgt tgcaaagtaa aaaaatatag ctaaccacta atttatcatg 1500
   tcagtgttcg cttaacttgc tagcatgatg ctaatttcgt ggcatggcga aaatccgtag 1560
   atctgatgag acctgcggtt ctttttatat agagcgtaaa tacattcaat accttttaaa 1620
   gtattctttg ctgtattgat actttgatac cttgtctttc ttactttaat atgacggtga 1680
   tcttgctcaa tgaggttatt cagatatttc gatgtacaat gacagtcagg tttaagttta 1740
   aaagctttaa ttactttagc cattgctacc ttcgttgaag gtgcctgatc tgtaattacc 1800
   ttttgaggtt taccaaattg tttaatgaga cgtttgataa acgcatatgc tgaatgatta 1860
   tctcgttgct tacgcaacca aatatctaat gtatgtccct ctgcatcaat ggcacgatat 1920
   aaatagctcc attttccttt tattttgatg tacgtctcat caatacgcca tttgtaataa 1980
   gcttttttat gctttttctt ccaaatttga tacaaaattg gggcatattc ttgaacccaa 2040
   cggtagaccg ttgaatgatg aacgtttaca ccacgttccc ttaatatttc agatatatca 2100
   cgataactca atgtatatct ta                                         2122
<210> 9
   <211> 1696
   <212> DNA
   <213> Staphylococcus aureus
<400> 9
   aaagagaaat attggaagca agccatagca gaatatgaaa aacgtttagg cccatacacc 60
   aagatagaca tcatagaagt tccagacgaa aaagcaccag aaaatatgag tgacaaagaa 120
   attgagcaag taaaagaaaa agaaggccaa cgaatactag ccaaaatcaa accacaatcc 180
   acagtcatta cattagaaat acaaggaaag atgctatctt ccgaaggatt ggcccaagaa 240
   ttgaaccaac gcatgaccca agggcaaagc gactttgttt tcgtcattgg cggatcaaac 300
   ggcctgcaca aggacgtctt acaacgcagt aactacgcac tatcattcag caaaatgaca 360
   ttcccacatc aaatgatgcg ggttgtgtta attgaacaag tgtacagagc atttaagatt 420
   atgcgaggag aagcatatca taaatgatgc ggttatttca gccgtaattt tataatataa 480
   agcagagttt attaaatttt aatgattact ttttattaag aattaattct agttgatata 540
   ttataatgtg aaacacaaaa taataatttg taattgttag tttataggca tctgtatttg 600
   gaattttttg tagactattt aaaaaatagt gtatataagt attgagttca tgtattaact 660
   gtcttttttc atcgttcatc aagtataagg atgtagagat ttgttggata atttcttcgg 720
   atgtttttaa aattatcatt aaattagatg gtatctgatc ttgagttttg tttttagtgt 780
   atgtatattt taaaaaattt ttgattgttg ttatttgact ctcttttaat ttgacaccct 840
   catcaataaa tgtgttaaat atatcttcat ttgtacttaa atcatcaaaa tttgccaaca 900
   aatatttgaa cgtctctaaa tcattatgtt tgagttccgt tttgctattc cataattcca 960
   aaccatttgg tagaaagccc aagctgtgat tttgatctcc ccatatagct gaatttaaat 1020
   cagtgagttg attaattttt tcaacacaga aatgtaattt tggaatgagg aatcgaagtt 1080
   gttcttctac ttgctgtact tttcttttgt tttcaataaa atttctacac catactgtta 1140
   tcaaaccgcc aattattgtg cacaatcctc caatgattgt agataaaatt gacaatatat 1200
   tacacacctt tcttagaggt ttattaacat ctatttttga atttaaaatt attactttgg 1260
   tagcgttata acctatttaa cagattagag aaaaattgaa tgatcgattg aagaatttcc 1320
   aaaataccgt cccatatgcg ttgaaggaga tttctatttt cttctgtatt caaatctttg 1380
   gctttatcct ttgctttatt caataaatca tctgagtttt tttcaatatt ttttaataca 1440
   tctttggcat tttgtttaaa tactttagga tcggaagtta gggcattaga gtttgccaca 1500
   ttaatcatat tattattaat catttgaatt tgattatctg ataatatctc tgataaccta 1560
   cgctcatcga ggactttatt aacagtgtct tcaacttgtt gttgtgtgat ttgtttatct 1620
   tgattttgtt taatatctgc aagttgttct ttaatatctg ctatagaagc atttaaagct 1680
   tcatctgaat acccat                                                 1696
<210> 10
   <211> 991
   <212> **DNA**
   <213> Staphylococcus aureus
<400> 10
   accattttag ctgtagggaa actaaaagag aaatactgga agcaagccat agcagaatat 60
   gaaaaacgtt taggcccata caccaagata gacatcatag aagttccaga cgaaaaagca 120
   ccagaaaata tgaactacaa agaaattgag caagtaaaag aaaaagaagg ccaacgaata 180
   ctagccaaaa tcaaaccaca atcaacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcga ggagaagcgt atcataagtg atggtaaaaa 480
   atatgagtaa gtagatgaag agtgaaaatc agattaatta ataataatgt atcaaattta 540
   aataaagggg tttttaagta tgaatttaag aggtcatgaa aatagactta aatttcatgc 600
   gaaatatgat gtgacaccta tatcacattt aaaattatta gaaggtcaaa agaaagacgg 660
   tgaaggcggc atactgacag atagctatta ctgtttttca tacagcttaa aaggtaattc 720
   taaaaaagtt ttaggtacgt ttaattgtgg ttatcatatt gctgaagatt tactaaaatt 780
   atcaaatcaa gataaattac ctttatttaa cccgtttaaa gtaattaatg aaggtaatca 840
   attgcagggc gtaacgaata aaggtaattt aaatattaat aggcaaagaa aacagtataa 900
   tgaagtggct ttacagcttt caaatgctat taatttaatc ataatttgtt atgaggataa 960
   tattaaagaa ccactttcaa cgataaaata c                                991
<210> 11
   <211> 1282
   <212> **DNA**
   <213> Staphylococcus aureus
<400> 11
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atccacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactat 360
   gcactatcat ttagcaaaat gacattccca catcaaatga tgcgggttgt gttaattgaa 420
   caagtgtata gagcatttaa gattatgcgt ggagaagcgt accacaaata aaactaaaaa 480
   atatgagaaa attattaaat tagctcaaat ctttgaagaa taaaaagtga atattaagtt 540
   tgataattta ggtacaagta aagattaaga atttccatta tttaatacat ggtgtgtaaa 600
   tcgacttctt tttgtattag atgtttgcag taagcgatgt aaagaagatg ctaataaata 660
   tgtgaggaat gattacgata ctagataagc ggctaatgaa attttttaaa gtacatatat 720
   agacatattt ttcatttagt aaaattttga atttcacttt gctaagacta gtgtctagaa 780
   atttataatg atttattaac acctatttga aacttaagta taataaatga ttcggatttt 840
   atttttaata aagacaaact tgaacgtagc aaagtagttt ttatgataaa taataagttt 900
   taataatgtg acgcttttat ataagcacat tattatgaac aatgtgaatt gagcatctac 960
   aattacatta ataaatatat aaatgatgat ttaaattcac atatatttat aatacacata 1020
   ctatatgaaa gttttgatta tccgaataaa tgctaaaatt aataaaataa ttaaaggaat 1080
   catacttatt atacgtatac gtttagctac tgaactactg gattcatttg gagattctag 1140
   tagttctttt tcaatctcta aatctaaatc agttttgtaa taaccattaa ttcctaatct 1200
   ttcatctagc tctgtacttt tttcatcatt tttatctttg ttgatatgtt ccattttctc 1260
   gcctcttttt aatcaagtag aa                                          1282
<210> 12
   <211> 1530
   <212> DNA
   <213> Staphylococcus aureus
<400> 12
   ttagctgtag ggaaactaaa agagaaatat tggaagcaag ccatagcaga atatgaaaaa 60
   cgtttaggcc catacaccaa gatagacatc atagaagttc cagacgaaaa agcaccagaa 120
   aatatgagcg acaaagaaat tgagcaagta aaagaaaaag aaggccaacg aatactagcc 180
   aaaatcaaac cacaatccac agtcattaca ttagaaatac aaggaaagat gctatcttcc 240
   gaaggattgg cccaagaatt gaaccaacgc atgacccaag ggcaaagcga ctttgtattc 300
   gtcattggcg gatcaaacgg cctgcacaag gacgtcttac aacgcagtaa ctatgcacta 360
   tcatttagca aaatgacatt cccacatcaa atgatgcggg ttgtgttaat tgaacaagtg 420
   tatagagcat ttaagattat gcgtggagaa gcatatcata aatgatgcgg ttttttcagc 480
   cgcttcataa aggggggtga tcatatcgga acgtatgagg tttatgagaa ttgctgctat 540
   gtttttatga agcgtatcat aaatgatgca gtttttgata attttttctt tatcagagat 600
   tttactaaaa atcccctcaa agtttgtttt tttcaacttc aactttgaag ggaataaata 660
   aggaacttat ttatatttat cctttatctc attaatatct atttttttat taataatatt 720
   ataaatatta aattctttag aaaagtcact atcactctta ttcttcatac taaacgttat 780
   taatctaata atatcagcta ctatttcttt aaattctatt gcatcttctt ttttataagt 840
   agcgcctgta tgaacaattt tatttctcat accatagtaa tctttcatat atttttttac 900
   acaattttta atttcattag aattatccaa atctagatta tcaattgtct ttaataaatg 960
   atcattaaca acattagcat acccacatcc aagcttcttt tttatctctt catcacttaa 1020
   attttcatct aatttataat atctttctaa aaaatttgtg ataaaaactt ctaatgcagt 1080
   ctgaatttgt acaattgcta aattatagtc agatttataa aaagaacgtt caccttttct 1140
   catagccaaa acataaatat tgctaggatg attattgaaa atattataat tttttttaat 1200
   atttaataaa tcactttttt tgatagatga atactgatct tcttctatct ttccaggcat 1260
   gtcaatcatg aaaatactca tctcttttat atttccatct atagtatata ttatataata 1320
   tggaatactt aatatatccc ctaatgatag ctggtatata ttatgatact gatatttaac 1380
   gctaataatt ttaataagat tatttagaca attaaattgc ttattaaaaa ttttcgttag 1440
   actattactt ttctttgatt ccctagaagt agaatttgat ttcaattttt taaactgatt 1500
   gtgcttgatt attgaagtta tttcaacata                                  1530
<210> 13
   <211> 1256
   <212> DNA
   <213> Staphylococcus aureus
<400> 13
   gctgtaggga aactaaaaga gaaatattgg aagcaagcca tagcagaata tgaaaaacgt 60
   ttaggcccat acaccaagat agacatcata gaagttccag acgaaaaagc accagaaaat 120
   atgagcgaca aagaaattga gcaagtaaaa gaaaaagaag gccaacgaat actagccaaa 180
   attaaaccac aatccacagt cattacatta gaaatacaag gaaagatgct atcttccgaa 240
   ggattggccc aagaattgaa ccaacgcatg acccaagggc aaagcgactt tgtattcgtc 300
   attggcggat caaacggcct gcacaaggac gtcttacaac gcagtaacta cgcactatca 360
   ttcagcaaaa tgacattccc acatcaaatg atgcgggttg tgttaattga gcaagtgtat 420
   agagcattta agattatgcg tggagaagca tatcataaat gatgcggttt tttcagccgc 480
   ttcataaagg gattttgaat gtatcagaac atatgaggtt tatgtgaatt gctgttatgt 540
   ttttaagaag catatcataa gtgatgcggt ttttattaat tagttgctaa aaaatgaagt 600
   atgcaatatt aattattatt aaattttgat atatttaaag aaagattaag tttagggtga 660
   atgaatggct tatcaaagtg aatatgcatt agaaaatgaa gtacttcaac aacttgagga 720
   attgaactat gaaagagtaa atatacataa tattaaatta gaaattaatg aatatctcaa 780
   agaactagga gtgttgaaaa atgaataagc agacaaatac tccagaacta agatttccag 840
   agtttgatga ggaatggaaa aaaaggaaat taggtgaagt agtaaattat aaaaatggtg 900
   gttcatttga aagtttagtg aaaaaccatg gtgtatataa actcataact cttaaatctg 960
   ttaatacaga aggaaagttg tgtaattctg gaaaatatat cgatgataaa tgtgttgaaa 1020
   cattgtgtaa tgatacttta gtaatgatac tgagcgagca agcaccagga ctagttggaa 1080
   tgactgcaat tatacctaat aataatgagt atgtactaaa tcaacgagta gcagcactag 1140
   tgcctaaaca atttatagat agtcaatttc tatctaagtt aattaataga aaccagaaat 1200
   atttcagtgt gagatctgct ggaacaaaag tgaaaaatat ttctaaagga catgta     1256
<210> 14
   <211> 748
   <212> **DNA**
   <213> Staphylococcus aureus
<400> 14
   atcgtttaac gtgtcacatg atgcgataga tccgcaattt tatattttcc ataataacta 60
   taagaagttt acgattttaa cagatacggg ttacgtgtct gatcgtatga aaggtatgat 120
   acgtggcagc gatgcattta tttttgagag taatcatgac gtcgatatgt tgagaatgtg 180
   tcgttatcca tggaagacga aacaacgcat tttaggcgat atgggtcatg tatctaatga 240
   ggatgcgggt catgcgatga cagacgtgat tacaggtaac acgaaacgta tttacttatc 300
   gcatttatca caagataata atatgaaaga tttggcgcgt atgagtgttg gccaagtatt 360
   gaacgaacac gatattgata cggaaaaaga agtattgcta tgtgatacgg ataaagctat 420
   tccaacacca atatatacaa tataaatgag agtcatccga taaagttccg cactgctgtg 480
   aaacgacttt atcgggtgct tttttatgtt gttggtggga aatggctgtt gttgagttga 540
   atcggattga ttgaaatgtg taaaataatt cgatattaaa tgtaatttat aaataattta 600
   cataaaatca aacattttaa tataaggatt atgataatat attggtgtat gacagttaat 660
   ggagggaacg aaatgaaagc tttattactt aaaacaagtg tatggctcgt tttgcttttt 720
   agtgtgatgg gattatggca tgtctcga                                     748
<210> 15
   <211> 1100
   <212> DNA
   <213> Staphylococcus aureus
<400> 15
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa ttaaaccaca atccacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgag 420
   caagtgtata gagcatttaa gattatgcgt ggagaagcgt atcacaaata aaactaaaaa 480
   ataagttgta tataacttat tttgaaattg gttaagtata taatatctcc aataaaatgt 540
   agttaactta cgataatgct gaactatagc tttgtaaact aaaatgtaaa taattacaat 600
   caaattgcaa caatatagtt caagaatgct acaatttgag gacagattga tagcattaat 660
   ccctttaaaa tgaagctagg agataactta cattatgatt agtaaacaaa taaaggattt 720
   acgaaagcaa cataattata ctcaagaaga gctagctgaa aaattaaata cttcaagaca 780
   aacaatttct aaatgggaac aaggtatttc agaaccagac ttaattatgc ttatgcaatt 840
   gtcacaatta ttttctgtta gtacagacta tctcattaca ggaagtgaca atattattaa 900
   aaaagataat aaaagctatt atgaaatgaa tttttgggca tttatgtctg aaaaatggtg 960
   ggtaattatt attatagtaa tcataatttg tggaacaata ggacaaattt tttcaaacta 1020
   atgtaagtat ctctcaaata ttttgggagg ttttattatg aaaatcaaaa aattattaaa 1080
   gacattatta attattttat                                             1100
<210> 16
   <211> 1109
   <212> DNA
   <213> Staphylococcus aureus
<400> 16
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atcaacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcgt ggagaagcgt atcacaaata aaactaaaaa 480
   ataagttgta tataacttat tttgaaattg gttaagtata tagtatctcc aataaaatgt 540
   agttaactta cgataatgct gaactatagc tttgtaaact aaaatgtaaa taattacaat 600
   caaattgcaa caatatagtt caagaatgct acaatttgag gacagattga tagcattaat 660
   ccctttaaaa tgaagctagg agataactta cattatgatt agtaaacaaa taaaggattt 720
   acgaaagcaa cataattata ctcaagaaga gctagctgaa aaattaaata cttcaagaca 780
   aacaatttct aaatgggaac aaggtatttc agaaccagac ttaattatgc ttatgcaatt 840
   gtcacaatta ttttctgtta gtacagacta tctcattaca ggaagtgaca atattattaa 900
   aaaagataat aaaagctatt atgaaatgaa tttttgggca tttatgtctg aaaaatggtg 960
   ggtaattatt attatagtaa tcataatttg tggaacaata ggacaaattt tttcaaacta 1020
   atgtaagtat ctctcaaata ttttgggagg ttttattatg aaaatcaaaa aattattaaa 1080
   gacattatta attattttat tatgttttg                                   1109
<210> 17
   <211> 1159
   <212> DNA
   <213> Staphylococcus aureus
<400> 17
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atcaacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcgt ggagaagcgt atcacaaata aaactaaaaa 480
   ataagttgta tataacttat tttgaaattg gttaagtata tagtatctcc aataaaatgt 540
   agttaactta cgataatgct gaactatagc tttgtaaact aaaatgtaaa taattacaat 600
   caaattgcaa caatatagtt caagaatgct acaatttgag gacagattga tagcattaat 660
   ccctttaaaa tgaagctagg agataactta cattatgatt agtaaacaaa taaaggattt 720
   acgaaagcaa cataattata ctcaagaaga gctagctgaa aaattaaata cttcaagaca 780
   aacaatttct aaatgggaac aaggtatttc agaaccagac ttaattatgc ttatgcaatt 840
   gtcacaatta ttttctgtta gtacagacta tctcattaca ggaagtgaca atattattaa 900
   aaaagataat aaaagctatt atgaaatgaa tttttgggca tttatgtctg aaaaatggtg 960
   ggtaattatt attatagtaa tcataatttg tggaacaata ggacaaattt tttcaaacta 1020
   atgtaagtat ctctcaaata ttttgggagg ttttattatg aaaatcaaaa aattattaaa 1080
   gacattatta attattttat tatgttttgt attgtctgtt attgtgcaaa atatttcaat 1140
   gctatggcat attgtgagc                                               1159
<210> 18
   <211> 1125
   <212> DNA
   <213> Staphylococcus aureus
<400> 18
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atccacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactat 360
   gcactatcat ttagcaaaat gacattccca catcaaatga tgcgggttgt gttaattgaa 420
   caagtgtata gagcatttaa gattatgcgt ggagaggcgt atcataaata aaactaaaaa 480
   acggattgtg tataatatat tttaaatata aaaaggattg attttatgtt aaataaatta 540
   gaaaatgtta gttataaatc attcgataat tacactagtg aagatgattt gactaaagta 600
   aatatatttt ttggaagaaa tgggagtgga aaaagctcat taagtgaatg gttaagaaga 660
   ctagataatg aaaaaagtgt tatctttaat actggttact taaaaaataa tattgaagaa 720
   gttgaagaaa tagatggtgt gaatttggtt attggagaag aatctataaa tcatagtgac 780
   caaattaagc atttaaatag cgctataaat agtttagaaa attttattac tcggaaaaat 840
   agtgaactta agcattcaaa agaaagaatt tacaataaaa tgaatatcag actaaatgaa 900
   gctagagaaa gatttgaaat aggtagtaat gtggttaagc agaagaggaa tgctgacaaa 960
   gatccagtta atgcttttta tagttggaag aaaaatgcta acgatataat tcaagagatg 1020
   actattgaat ctttagatga attagaagaa agaataacaa gaaaagaagt cttattaaat 1080
   aatataaaaa caccaatttt agcttttgat tataatgatt ttagt                  1125
<210> 19
   <211> 4149
   <212> DNA
   <213> Staphylococcus aureus
<400> 19
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa ttaaaccaca atccacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgag 420
   caagtgtata gagcatttaa gattatgcgt ggagaagcat atcataagtg atgcggtttt 480
   tattaattag ttgctaaaaa atgaagtatg caatattaat tattattaaa ttttgatata 540
   tttaaagaaa gattaagttt agggtgaatg aatggcttat caaagtgaat atgcattaga 600
   aaatgaagta cttcaacaac ttgaggaatt gaactatgaa agagtaaata tacataatat 660
   taaattagaa attaatgaat atctcaaaga actaggagtg ttgaaaaatg aataagcaga 720
   caaatactcc agaactaaga tttccagagt ttgatgagga atggaaaaaa aggaaattag 780
   gtgaagtagt aaattataaa aatggtggtt catttgaaag tttagtgaaa aaccatggtg 840
   tatataaact cataactctt aaatctgtta atacagaagg aaagttgtgt aattctggaa 900
   aatatatcga tgataaatgt gttgaaacat tgtgtaatga tactttagta atgatactga 960
   gcgagcaagc accaggacta gttggaatga ctgcaattat acctaataat aatgagtatg 1020
   tactaaatca acgagtagca gcactagtgc ctaaacaatt tatagatagt caatttctat 1080
   ctaagttaat taatagaaac cagaaatatt tcagtgtgag atctgctgga acaaaagtga 1140
   aaaatatttc taaaggacat gtagaaaact ttaatttttt atctcctaat tacactgaac 1200
   aacaaaaaat aggtaatttc ttcagcaaac tcgaccgcca gattgagtta gaagaagaga 1260
   aacttgaact cttagagcaa caaaagcgtg gatatattca gaagattttt tctcaagatt 1320
   taagatttaa agatgaaaat ggaaacagtt atcctgattg gtctattaaa aagattgaag 1380
   atatttctaa agttaataaa gggtttactc caaatacaaa aaatgataaa tactgggatg 1440
   aattaaatga aaattggtta tctatagcag gtatgacaca gaaatatttg tataaaggaa 1500
   ataaaggaat tactgaaaaa ggtgcatcaa agcatgtaaa agtagataaa gatactctaa 1560
   taatgagttt taaattgact ttaggtaagt tagctatagt aaaagagcct atctatacaa 1620
   atgaagctat atgccatttc gtatggaaag aaagtaatgt taatactgag tatatgtact 1680
   actatttaaa ttctataaat ataagtactt ttggtgcaca ggcagttaaa ggagtaacat 1740
   taaataacga tgcaattaat agtattatag taaagttacc agtgatacaa gaacaaaata 1800
   aaatagcata ctttttcaat aaattagata aattaattga aaaacaatct tctaaagtag 1860
   aattattaaa acaacgcaaa caaggatttt tacagaaaat gtttgtttaa ttcttataaa 1920
   gttctattat gtaaaatatt aaatagagat aacattatga aagcgagccc aagacataaa 1980
   gtttttgaat aaataaaaaa gataatttct atcaaattaa tatagaaatt gtctttttta 2040
   taaatttttt gattattttt agctgattga gctgttactt ttcttataat aagtgctatt 2100
   agcacaaatc ctagttctct tttggctttg tttattcctc ttacggacat tcgagtgaaa 2160
   cccattttaa ttttattaga agtaatttag gtttgaaccc acctaaataa atatatgagt 2220
   tattttttta tgctacaaaa tatattcaga tttcaataat gacataaaat aggcatcttt 2280
   atatttacct ttagtgtaga attgctcttt gagtaatcct tctgttttaa atccttgtga 2340
   ctcgtatata tgcacagctt ttttgttatc tgtatcaaca tatagataaa ttttgtgcat 2400
   gtttaatata tcgaatgcat aatttatcgc tttttcgaat gcgaattttg cataaccttt 2460
   accactgaac tcaggtttaa taattatttg tatttcacaa ttacgatgga tgtaattaat 2520
   ttctactaat tcaacaatac ctacgacttg attttcatct tcaacaataa aacgtctctc 2580
   tgattcatct aataaatgct tatcaaataa atattgaagt tccgttaagg attcatatgg 2640
   ttcttcaaac caataagaca taatagaata ttcattattt aattcatgaa caaaaagtaa 2700
   atcactatac tctaatgctc ttagtttcat aattccactc ccaaaatttt ctcatatatt 2760
   tgcattataa atataaataa cgaataagtc atcattcact gtgaatactc tattttaaca 2820
   attcaccaca tactaattct cattttcttg ttattctcga tttattactc ttactatgaa 2880
   acctataaaa ttctcacatt tgtttgtatt aagaataaat acgtcgatag taacaataaa 2940
   aaaataaata ataaagcatc cctcaccgta aaagtgaagg atgctctagt tttattgaaa 3000
   tatacatttc attttgttaa ataattatta ataatatttt gaaaatcatt attacgtgaa 3060
   atcttcatag attttatcaa gtatttcttt gccttcaatt gctgtgaagt gatgtaccaa 3120
   tctattttta caatcatatg taattttgtg acgctaggta attagtaatt gttcgtcagt 3180
   ctgattgtat agtatcaagt ttcatagata atactctttg attttaatgt ccactttgac 3240
   gtgctttaag attgagtata tacataatgt cattgtggaa tgttaaaaat cctacaaatg 3300
   tttattcatc tgcaggattt ttaaatctcc aagaataaaa atcatcatag gacaactgga 3360
   ttattgtttg gataaataac gtaaacaata attaggtact attatttatt tttgtttatt 3420
   ctttttccta acaaaataaa gaaaagaata aacgcaattg ttaaaaatat gtgtcctaaa 3480
   ccagcaatac cagcaatagc aggacttaca cttagatctt taatggtaga aataccgttc 3540
   acgaattgca ttgccacagt aacaagcaca cctaaatggt atatataaaa gaaactgtta 3600
   aacagctttg tatgagttgt taatttgaat tggccctcga taatcatgaa aattaagaac 3660
   ataattgtac ctagtactaa taaatgtgta tgtgtaacat ttaattgaga aaaaccgcta 3720
   aaatcttccg cttttgtcat ttctctataa aatagaccac ttaataaccc taatagtgta 3780
   tagagcgctg aactatacat taatcttttc attttaattc cccctatttt taattacgag 3840
   ataagtatag cggtagttta tgaactgagt atgaacttac aacaaaaaaa ttaatgaagt 3900
   actttacaat aaactcaatt tattagatgg tggagggacg aaaaaggatt ttagaaaaat 3960
   aaattaatat atttttattt tgataagtaa taattaataa tatcttggaa atcattgtta 4020
   agtattgttg taatacaatc gtcattcata aaatcttcat agtttttatc aagaatttct 4080
   tcatcttcga tagatgtgaa atgattagct aaccctttta taatttaagt gtaatttgtg 4140
   aatctaaac                                                         4149
<210> 20
   <211> 1081
   <212> DNA
   <213> Staphylococcus aureus
<400> 20
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atcaacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcgt ggagaagcat atcataagtg atgcggtttt 480
   tattaattag ttgctaaaaa atgaagtatg caatattaat tattattaaa ttttgatata 540
   tttaaagaaa gattaagttt agggtgaatg aatggcttat caaagtgaat atgcattaga 600
   aaatgaagta cttcaacaac ttgaggaatt gaactatgaa agagtaaata tacataatat 660
   taaattagaa attaatgaat atctcaaaga actaggagtg ttgaaaaatg aataagcaga 720
   caaatactcc agaactaaga tttccagagt ttgatgagga atggaaaaaa aggaaattag 780
   gtgaagtagt aaattataaa aatggtggtt catttgaaag tttagtgaaa aaccatggtg 840
   tatataaact cataactctt aaatctgtta atacagaagg aaagttgtgt aattctggaa 900
   aatatatcga tgataaatgt gttgaaacat tgtgtaatga tactttagta atgatactga 960
   gcgagcaagc accaggacta gttggaatga ctgcaattat acctaataat aatgagtatg 1020
   tactaaatca acgagtagca gcactagtgc ctaaacaatt tatagatagt caatttctat 1080
   c                                                                 1081
<210> 21
   <211> 1054
   <212> **DNA**
   <213> Staphylococcus aureus
<400> 21
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa tcaaaccaca atcaacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcgt ggagaagcat atcataagtg atgcggtttt 480
   tattaattag ttgctaaaaa atgaagtatg caatattaat tattattaaa ttttgatata 540
   tttaaagaaa gattaagttt agggtgaatg aatggcttat caaagtgaat atgcattaga 600
   aaatgaagta cttcaacaac ttgaggaatt gaactatgaa agagtaaata tacataatat 660
   taaattagaa attaatgaat atctcaaaga actaggagtg ttgaaaaatg aataagcaga 720
   caaatactcc agaactaaga tttccagagt ttgatgagga atggaaaaaa aggaaattag 780
   gtgaagtagt aaattataaa aatggtggtt catttgaaag tttagtgaaa aaccatggtg 840
   tatataaact cataactctt aaatctgtta atacagaagg aaagttgtgt aattctggaa 900
   aatatatcga tgataaatgt gttgaaacat tgtgtaatga tactttagta atgatactga 960
   gcgagcaagc accaggacta gttggaatga ctgcaattat acctaataat aatgagtatg 1020
   tactaaatca acgagtagca gcactagtgc ctaa                             1054
<210> 22
   <211> 4353
   <212> DNA
   <213> Staphylococcus aureus
<400> 22
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagcgacaa agaaattgag caagtaaaag aaaaagaagg ccaacgaata 180
   ctagccaaaa ttaaaccaca atccacagtc attacattag aaatacaagg aaagatgcta 240
   tcttccgaag gattggccca agaattgaac caacgcatga cccaagggca aagcgacttt 300
   gtattcgtca ttggcggatc aaacggcctg cacaaggacg tcttacaacg cagtaactac 360
   gcactatcat tcagcaaaat gacattccca catcaaatga tgcgggttgt gttaattgag 420
   caagtgtata gagcatttaa gattatgcgt ggagaagcat atcataaatg atgcggtttt 480
   ttcagccgct tcataaaggg attttgaatg tatcagaaca tatgaggttt atgtgaattg 540
   ctgttatgtt tttaagaagc atatcataaa tgatgcggtt ttttcagccg cttcataaag 600
   ggattttgaa tgtatcagaa catatgaggt ttatgtgaat tgctgttatg tttttaagaa 660
   gcatatcata agtgatgcgg tttttattaa ttagttgcta aaaaatgaag tatgcaatat 720
   taattattat taaattttga tatatttaaa gaaagattaa gtttagggtg aatgaatggc 780
   ttatcaaagt gaatatgcat tagaaaatga agtacttcaa caacttgagg aattgaacta 840
   tgaaagagta aatatacata atattaaatt agaaattaat gaatatctca aagaactagg 900
   agtgttgaaa aatgaataag cagacaaata ctccagaact aagatttcca gagtttgatg 960
   aggaatggaa aaaaaggaaa ttaggtgaag tagtaaatta taaaaatggt ggttcatttg 1020
   aaagtttagt gaaaaaccat ggtgtatata aactcataac tcttaaatct gttaatacag 1080
   aaggaaagtt gtgtaattct ggaaaatata tcgatgataa atgtgttgaa acattgtgta 1140
   atgatacttt agtaatgata ctgagcgagc aagcaccagg actagttgga atgactgcaa 1200
   ttatacctaa taataatgag tatgtactaa atcaacgagt agcagcacta gtgcctaaac 1260
   aatttataga tagtcaattt ctatctaagt taattaatag aaaccagaaa tatttcagtg 1320
   tgagatctgc tggaacaaaa gtgaaaaata tttctaaagg acatgtagaa aactttaatt 1380
   ttttatctcc taattacact gaacaacaaa aaataggtaa tttcttcagc aaactcgacc 1440
   gccagattga gttagaagaa gagaaacttg aactcttaga gcaacaaaag cgtggatata 1500
   ttcagaagat tttttctcaa gatttaagat ttaaagatga aaatggaaac agttatcctg 1560
   attggtctat taaaaagatt gaagatattt ctaaagttaa taaagggttt actccaaata 1620
   caaaaaatga taaatactgg gatgaattaa atgaaaattg gttatctata gcaggtatga 1680
   cacagaaata tttgtataaa ggaaataaag gaattactga aaaaggtgca tcaaagcatg 1740
   taaaagtaga taaagatact ctaataatga gttttaaatt gactttaggt aagttagcta 1800
   tagtaaaaga gcctatctat acaaatgaag ctatatgcca tttcgtatgg aaagaaagta 1860
   atgttaatac tgagtatatg tactactatt taaattctat aaatataagt acttttggtg 1920
   cacaggcagt taaaggagta acattaaata acgatgcaat taatagtatt atagtaaagt 1980
   taccagtgat acaagaacaa aataaaatag catacttttt caataaatta gataaattaa 2040
   ttgaaaaaca atcttctaaa gtagaattat taaaacaacg caaacaagga tttttacaga 2100
   aaatgtttgt ttaattctta taaagttcta ttatgtaaaa tattaaatag agataacatt 2160
   atgaaagcga gcccaagaca taaagttttt gaataaataa aaaagataat ttctatcaaa 2220
   ttaatataga aattgtcttt tttataaatt ttttgattat ttttagctga ttgagctgtt 2280
   acttttctta taataagtgc tattagcaca aatcctagtt ctcttttggc tttgtttatt 2340
   cctcttacgg acattcgagt gaaacccatt ttaattttat tagaagtaat ttaggtttga 2400
   acccacctaa ataaatatat gagttatttt tttatgctac aaaatatatt cagatttcaa 2460
   taatgacata aaataggcat ctttatattt acctttagtg tagaattgct ctttgagtaa 2520
   tccttctgtt ttaaatcctt gtgactcgta tatatgcaca gcttttttgt tatctgtatc 2580
   aacatataga taaattttgt gcatgtttaa tatatcgaat gcataattta tcgctttttc 2640
   gaatgcgaat tttgcataac ctttaccact gaactcaggt ttaataatta tttgtatttc 2700
   acaattacga tggatgtaat taatttctac taattcaaca atacctacga cttgattttc 2760
   atcttcaaca ataaaacgtc tctctgattc atctaataaa tgcttatcaa ataaatattg 2820
   aagttccgtt aaggattcat atggttcttc aaaccaataa gacataatag aatattcatt 2880
   atttaattca tgaacaaaaa gtaaatcact atactctaat gctcttagtt tcataattcc 2940
   actcccaaaa ttttctcata tatttgcatt ataaatataa ataacgaata agtcatcatt 3000
   cactgtgaat actctatttt aacaattcac cacatactaa ttctcatttt cttgttattc 3060
   tcgatttatt actcttacta tgaaacctat aaaattctca catttgtttg tattaagaat 3120
   aaatacgtcg atagtaacaa taaaaaaata aataataaag catccctcac cgtaaaagtg 3180
   aaggatgctc tagttttatt gaaatataca tttcattttg ttaaataatt attaataata 3240
   ttttgaaaat cattattacg tgaaatcttc atagatttta tcaagtattt ctttgccttc 3300
   aattgctgtg aagtgatgta ccaatctatt tttacaatca tatgtaattt tgtgacgcta 3360
   ggtaattagt aattgttcgt cagtctgatt gtatagtatc aagtttcata gataatactc 3420
   tttgatttta atgtccactt tgacgtgctt taagattgag tatatacata atgtcattgt 3480
   ggaatgttaa aaatcctaca aatgtttatt catctgcagg atttttaaat ctccaagaat 3540
   aaaaatcatc ataggacaac tggattattg tttggataaa taacgtaaac aataattagg 3600
   tactattatt tatttttgtt tattcttttt cctaacaaaa taaagaaaag aataaacgca 3660
   attgttaaaa atatgtgtcc taaaccagca ataccagcaa tagcaggact tacacttaga 3720
   tctttaatgg tagaaatacc gttcacgaat tgcattgcca cagtaacaag cacacctaaa 3780
   tggtatatat aaaagaaact gttaaacagc tttgtatgag ttgttaattt gaattggccc 3840
   tcgataatca tgaaaattaa gaacataatt gtacctagta ctaataaatg tgtatgtgta 3900
   acatttaatt gagaaaaacc gctaaaatct tccgcttttg tcatttctct ataaaataga 3960
   ccacttaata accctaatag tgtatagagc gctgaactat acattaatct tttcatttta 4020
   attcccccta tttttaatta cgagataagt atagcggtag tttatgaact gagtatgaac 4080
   ttacaacaaa aaaattaatg aagtacttta caataaactc aatttattag atggtggagg 4140
   gacgaaaaag gattttagaa aaataaatta atatattttt attttgataa gtaataatta 4200
   ataatatctt ggaaatcatt gttaagtatt gttgtaatac aatcgtcatt cataaaatct 4260
   tcatagattt tatcaagaat ttcttcatct tcgatagatg tgaaatgatt agctaaccct 4320
   tttataattt aagtgtaatt tgtgaatcta aac                               4353
<210> 23
   <211> 2031
   <212> DNA
   <213> Staphylococcus aureus
<400> 23
   accattttag ctgtagggaa actaaaagag aaatattgga agcaagccat agcagaatat 60
   gaaaaacgtt taggcccata caccaagata gacatcatag aagttccaga cgaaaaagca 120
   ccagaaaata tgagtgacaa agaaattgag caagtaaaag aaaaagaagg ccaacgaata 180
   ctagccaaaa tcaaaccaca atccacagtc attacattag aaatacaagg aaagatgcta 240
   tcttccgaag gattggccca agaattgaac caacgcatga cccaagggca aagcgacttt 300
   gttttcgtca ttggcggatc aaacggcctg cacaaggacg tcttacaacg cagtaactac 360
   gcactatcat tcagcaaaat gacattccca catcaaatga tgcgggttgt gttaattgaa 420
   caagtgtaca gagcatttaa gattatgcga ggagaagcgt atcacaaata aaactaaaaa 480
   atagattgtg tataatataa aaggagcgga tttatattaa aactttgaat tcaaaaatta 540
   ttgaaaggga agctacctta gaaattgaat ctatggcaac taatacattg aaaataaacc 600
   cggatattaa ttcaaacgat acaaaaatgt ctttcgatgg agaattggaa gtgtatgatt 660
   ctgaaaattt gagtaaaaaa aatttcgttg gaaaaataca agttcaagtt aaaggaaagg 720
   aagtagctaa aagaggaggt aaggttattc atcgaagtaa tgtcaaaatg aatgatttaa 780
   aggcatacca acgagaaggt ggtgtgtatt actttgtcgt gtatttaatc gttgagaata 840
   aaaaagttgt tgagaagcag gtttatggta aacaattaca tcaattagat ttacaatttt 900
   tactgcaaaa aaagcagaaa agcgtcacta taaaaatgta tgaaattgaa aatgaaaaaa 960
   ttttatataa taattgcgta aaatatataa atgaaaagag attacaaaat caagtaggtc 1020
   aagttaaagt aaagaacata gaaaaagctt tatcatatat agctacacct gaaaatattg 1080
   tgatggatca tagaggttta ccattaaatg atttttatgg ctatataaaa attaattcat 1140
   ctgaattaga tgtaactata ccagatggag tattaagtat ggaaaaagta aaaagagtga 1200
   acaaaaagca gataataaaa gaaggcaagt tattatttga aggtatggta ggtattgaaa 1260
   cttcaaagga gtccatctct ataactatag atgatatttt caaaattcaa acatttgaaa 1320
   gtgataacaa aagtatatat acaatgttac catttaaaaa attaaatata gccgaacaat 1380
   cttttaatgt tataaacgaa ttgtcgaaag gcggcgaatt ctttttggat caaatccaat 1440
   tagtaatcca accttttgaa attaatatta ttgaaataaa agaaacaata aataagttga 1500
   atattaaatt atcagagtat agtaacttgc tttcgtttga tgtgagtcta aaatctacag 1560
   aatttgataa gcagatgaat gagataaaag gtttattaga attattggaa tataaaaatt 1620
   ttaaagattt taaaatgcat aataatggat actataaaat gaagttttgt ggaaaattta 1680
   tattattatt taaagacaat acatcattgt ataatgtcta ctctaatgac tttgtagata 1740
   gatttgaggc tgttacaaaa gaaagagttg tacagatgcc aattgtttac acattaacaa 1800
   gagatatgat tgtagatgta ctgaattttg atataaatgt tattaaagaa tgtattgaat 1860
   cagataaaat tgctattcaa tctgacatta aatgggagaa attaaataac tttgcattag 1920
   aattaatcgc agcctatgat gaaactcaaa gaaccgattt attagaatta gctgaatatg 1980
   tattaaataa tttgctgaat tttgataatg ataaaatatt tatgaactta a           2031
<210> 24
   <211> 818
   <212> DNA
   <213> Staphylococcus aureus
<400> 24
   aatattggaa gcaagccata gcagaatatg aaaaacgttt aggcccatac accaagatag 60
   acatcataga agttccagac gaaaaagcac cagaaaatat gagcgacaaa gaaattgagc 120
   aagtgtatag agcatttaag attatgcgtg gagaagcata tcataaatga tgcggttttt 180
   tcagccgctt cataaaggga ttttgaatgt atcagaacat atgaggttta tgtgaattgc 240
   tgttatgttt ttaagaagct tatcataagt aatgaggttc atgatttttg acatagttag 300
   cctccgcagt ctttcatttc aagtaaataa tagcgaaata ttctttatac tgaatactta 360
   tagtgaagca aagttctagc tttgagaaaa ttctttctgc aactaaatat agtaaattac 420
   ggtaaaatat aaataagtac atattgaaga aaatgagaca taatatattt tataatagga 480
   gggaatttca aatgatagac aactttatgc aggtccttaa attaattaaa gagaaacgta 540
   ccaataatgt agttaaaaaa tctgattggg ataaaggtga tctatataaa actttagtcc 600
   atgataagtt acccaagcag ttaaaagtgc atataaaaga agataaatat tcagttgtag 660
   ggaaggttgc tactgggaac tatagtaaag ttccttggat ttcaatatat gatgagaata 720
   taacaaaaga aacaaaggat ggatattatt tggtatatct ttttcatccg gaaggagaag 780
   gcatatactt atcttgaatc aaggatggtc aaagataa 818
<210> 25
   <211> 1073
   <212> DNA
   <213> Staphylococcus aureus
<400> 25
   tattggaagc aagccatagc agaatatgaa aaacgtttag gcccatacac caagatagac 60
   atcatagaag ttccagacga aaaagcacca gaaaatatga gcgacaaaga aattgagcaa 120
   gtaaaagaaa aagaaggcca acgaatacta gccaaaatca aaccacaatc cacagtcatt 180
   acattagaaa tacaaggaaa gatgctatct tccgaaggat tggcccaaga attgaaccaa 240
   cgcatgaccc aagggcaaag cgactttgta ttcgtcattg gcggatcaaa cggcctgcac 300
   aaggacgtct tacaacgcag taactatgca ctatcattta gcaaaatgac attcccacat 360
   caaatgatgc gggttgtgtt aattgaacaa gtgtatagag catttaagat tatgcgtgga 420
   gaggcgtatc ataagtgatg cttgttagaa tgatttttaa caatatgaaa tagctgtgga 480
   agcttaaaca atttgtttat ctaagtactt atttaataat tgattgaact gtgattggca 540
   ccaggctgtc tggtaaattg agaagttggg ttttggagcg tataaatgat agaattaata 600
   taaaattcaa tttgaggagt aggagattat gtcgaatata aaaacaacac tagagacgtc 660
   cgtaggacta gaaaaagaca acgataagct atttgattat ataactgaat tagagattca 720
   aaacacgcct gaaaaccggg aagcaaaagt tgttattgaa gaaaggttac ataaagaata 780
   taaatatgaa ttagatcaaa tgacaccaga gtatggaata caaaaaggca gtgttagaat 840
   aggtcatgca gatgttgtaa tatttcatga ttctaaagat aaatctcaag agaatattaa 900
   aataatagta gagtgtaaaa gaaagaatcg cagggatggt attgaacaat taaaaacata 960
   tcttgcaggg tgtgagtctg cagaatacgg cgtttggttt aatggagaag atatagtata 1020
   tataaaacga ttgaaaaaag caccacattg gaaaacagta tttaatatac cga 1073
<210> 26
   <211> 756
   <212> DNA
   <213> Staphylococcus aureus
<400> 26
   tgacattccc acatcaaatg atgcgggttg tgttaattga gcaagtgtat agagcattta 60
   agattatgcg tggagaagcg tatcacaaat aaaactaaaa aataggttgc gcataatata 120
   attagaaagg aattagacat aaattaggag tccttcacag aatagcgaag gactcccatt 180
   aaatatatta tggtgtaaag aaatcacaaa tcaatatata tacttaatac catatattaa 240
   cttgtactat tataaagtac gacatcagta ttaggtatca ctttgaacac atgaatttca 300
   ttatcacttt tattattcac aaaaaatttt ccaattctca attactgaat tatgtgtata 360
   catgttgtta aaaattaata aaggatattt atgtttgttt aaagcatatc acaagtgatg 420
   cggtttttta taaagattta cttgttagtg attttgataa aaatgcttaa tactatttca 480
   ataatatgta tttaaaaatt agattaatag tatttaactt caaatggcct cgtataaact 540
   catagcaaat taacgtaaat caatgaaata aaatgaaaac aatttcaaga atacattata 600
   aacataaagt atacaaaaaa taaatgagcg tatttgttta aacgtataca ctcattttta 660
   ttaaattaat ttattatatt ttacgattgt tatttatgaa attaacaaat tccatttttg 720
   atagtgaaat taaaagcttt atcacttatt attgat 756
<210> 27
   <211> 771
   <212> **DNA**
   <213> Staphylococcus aureus
<400> 27
   tgacattccc acatcaaatg atgcgggttg tgttaattga gcaagtgtat agagcattta 60
   agattatgcg tggagaagcg tatcacaaat aaaactaaaa aataggttgc gcataatata 120
   attagaaagg aattagacat aaattaggag tccttcacag aatagcgaag gactcccatt 180
   aaatatatta tggtgtaaag aaatcacaaa tcaatatata tacttaatac catatattaa 240
   cttgtactat tataaagtac gacatcagta ttaggtatca ctttgaacac atgaatttca 300
   ttatcacttt tattattcac aaaaaatttt ccaattctca attactgaat tatgtgtata 360
   catgttgtta aaaattaata aaggatattt atgtttgttt aaagcatatc acaagtgatg 420
   cggtttttta taaagattta cttgttagtg attttgataa aaatgcttaa tactatttca 480
   ataatatgta tttaaaaatt agattaatag tatttaactt caaatggcct cgtataaact 540
   catagcaaat taacgtaaat caatgaaata aaatgaaaac aatttcaaga atacattata 600
   aacataaagt atacaaaaaa taaatgagcg tatttgttta aacgtataca ctcattttta 660
   ttaaattaat ttattatatt ttacgattgt tatttatgaa attaacaaat tccatttttg 720
   atagtgaaat taaaagcttt atcacttatt attgataatt ttgactgcat c 771
<210> 28
   <211> 680
   <212> DNA
   <213> Staphylococcus aureus
<400> 28
   ttcagcaaaa tgacattccc acatcaaatg atgcgggttg tgttaattga acaagtgtac 60
   agagcattta agattatgcg tggagaagcg tatcataagt agcggaggag ttttttacct 120
   tgtgacttat cataaagtac gatgtttatg taagtgatta tcattattta agcaggtttt 180
   tcaaattaaa taataacaag aataaaatgc acttagcgac attgaaattt attaatctag 240
   taaactaata gatttataga aaattttatt tgcaagggga taattttgaa aagtagtatt 300
   ttctatcttt ccataataca ttgtaattac aacggagggg atattgtgat gaagtgtata 360
   gataaaacgt gggttagcta ttataaagaa ttagctgata agttaacaga ttatcaaaat 420
   aaacgttatg aattaattga aatagtgaag gaagtatata aaaaaacggg aataaaattc 480
   cctactttag caagtgataa tgtattgatg gacatagatc cttttacaat atttgcatta 540
   tttaataaaa attccatgag agaaactaat aaggtaaaaa tattaacaga attagcttcg 600
   gaattgaata ttaagtccaa aattccgtca gtttttgaca gtattccaac agtcaataat 660
   ctgaatgcta catattataa 680
<210> 29
   <211> 1119
   <212> DNA
   <213> Staphylococcus aureus
<400> 29
   gacattccca catcaaatga tgcgggttgt gttaattgag caagtgtata gagcatttaa 60
   gattatgcgt ggagaggcgt atcataagta aaactaaaaa attctgtatg aggagataat 120
   aatttggagg gtgttaaatg gtggacatta aatccacgtt cattcaatat ataagatata 180
   tcacgataat tgcgcatata acttaagtag tagctaacag ttgaaattag gccctatcaa 240
   attggtttat atctaaaatg attaatatag aatgcttctt tttgtcctta ttaaattata 300
   aaagtaactt tgcaatagaa acagttattt cataatcaac agtcattgac gtagctaagt 360
   aatgataaat aatcataaat aaaattacag atattgacaa aaaatagtaa atataccaat 420
   gaagtttcaa aagaacaatt ccaagaaatt gagaatgtaa ataataaggt caaagaattt 480
   tattaagatt tgaaagagta tcaatcaaga aagatgtagt tttttaataa actatttgga 540
   aaataattat cataatttaa aaactgacaa tttgcgagac tcataaaatg taataatgga 600
   aatagatgta aaatataatt aaggggtgta atatgaagat taatatttat aaatctattt 660
   ataattttca ggaaacaaat acaaattttt tagagaatct agaatcttta aatgatgaca 720
   attatgaact gcttaatgat aaagaacttg ttagtgattc aaatgaatta aaattaatta 780
   gtaaagttta tatacgtaaa aaagacaaaa aactattaga ttggcaatta ttaataaaga 840
   atgtatacct agatactgaa gaagatgaca atttattttc agaatccggt catcattttg 900
   atgcaatatt atttctcaaa gaagatacta cattacaaaa taatgtatat attatacctt 960
   ttggacaagc atatcatgat ataaataatt tgattgatta tgacttcgga attgattttg 1020
   cagaaagagc aatcaaaaat gaagacatag ttaataaaaa tgttaatttt tttcaacaaa 1080
   acaggcttaa agagattgtt aattatagaa ggaatagtg                        1119
<210> 30
   <211> 24
   <212> DNA
   <213> Artificial Sequence
<220>
   <223> synthetic oligonucleotide
<400> 30
   caaattgtag cattcttgaa ctat 24
<210> 31
   <211> 26
   <212> DNA
   <213> Artificial Sequence
<220>
   <223> synthetic oligonucleotide
<400> 31
   ctattgttcc acaaattatg attact 26
<210> 32
   <211> 24
   <212> DNA
   <213> Artificial Sequence
<220>
   <223> synthetic oligonucleotide
<400> 32
   ctcccatttc ttccaaaaaa tata 24
<210> 33
   <211> 26
   <212> DNA
   <213> Artificial Sequence
<220>
   <223> synthetic oligonucleotide
<400> 33
   cttcttttct tgttattctt tcttct 26
<210> 34
   <211> 24
   <212> DNA
   <213> Artificial Sequence
<220>
   <223> synthetic oligonucleotide
<400> 34
   taatttcctt tttttccatt cctc 24
<210> 35
   <211> 24
   <212> DNA
   <213> Artificial Sequence
<220>
   <223> synthetic oligonucleotide
<400> 35
   gagcggattt atattaaaac tttg 24
<210> 36
   <211> 24
   <212> DNA
   <213> Artificial Sequence
<220>
   <223> synthetic oligonucleotide
<400> 36
   gttgccatag attcaatttc taag 24
<210> 37
   <211> 20
   <212> DNA
   <213> Artificial Sequence
<220>
   <223> synthetic oligonucleotide
<400> 37
   cgaggagaag cgtatcacaa 20
<210> 38
   <211> 27
   <212> DNA
   <213> Artificial Sequence
<220>
   <223> synthetic oligonucleotide
<400> 38
   agttgccata gattcaattt ctaaggt 27
<210> 39
   <211> 29
   <212> DNA
   <213> Artificial Sequence
<220>
   <223> synthetic oligonucleotide
<400> 39
   gtcaaaaatc atgaacctca ttacttatg 29
<210> 40
   <211> 19
   <212> DNA
   <213> Artificial Sequence
<220>
   <223> synthetic oligonucleotide
<400> 40
   caccctgcaa gatatgttt 19
<210> 41
   <211> 22
   <212> DNA
   <213> Artificial Sequence
<220>
   <223> synthetic oligonucleotide
<400> 41
   cgtggagagg cgtatcataa gt 22
<210> 42
   <211> 22
   <212> DNA
   <213> Artificial Sequence
<220>
   <223> synthetic oligonucleotide
<400> 42
   gctccaaaac ccaacttctc aa 22
<210> 43
   <211> 25
   <212> DNA
   <213> Artificial Sequence
<220>
   <223> synthetic oligonucleotide
<400> 43
   aatggaattt gttaatttca taaat 25
<210> 44
   <211> 23
   <212> DNA
   <213> Artificial Sequence
<220>
   <223> synthetic oligonucleotide
<400> 44
   ttccgaagct aattctgtta ata 23
<210> 45
   <211> 25
   <212> DNA
   <213> Artificial Sequence
<220>
   <223> synthetic oligonucleotide
<400> 45
   ttccgaagtc ataatcaatc aaatt 25
<210> 46
   <211> 23
   <212> DNA
   <213> Artificial Sequence
<220>
   <223> synthetic oligonucleotide
<400> 46
   gccaaaatca aaccacaatc cac 23
<210> 47
   <211> 23
   <212> DNA
   <213> Artificial Sequence
<220>
   <223> synthetic oligonucleotide
<400> 47
   gccaaaatca aaccacaatc aac 23
<210> 48
   <211> 23
   <212> DNA
   <213> Artificial Sequence
<220>
   <223> synthetic oligonucleotide
<400> 48
   accacgaatg tttgctgcta atg 23
<210> 49
   <211> 36
   <212> DNA
   <213> Artificial Sequence
<220>
   <223> synthetic oligonucleotide
<400> 49
   cgcttgccac atcaaatgat gcgggttgtg caagcg 36
<210> 50
   <211> 36
   <212> DNA
   <213> Artificial Sequence
<220>
   <223> synthetic oligonucleotide
<400> 50
   cccaccccac atcaaatgat gcgggttgtg ggtggg 36
<210> 51
   <211> 37
   <212> DNA
   <213> Artificial Sequence
<220>
   <223> synthetic oligonucleotide
<400> 51
   cccgcgcgta gttactgcgt tgtaagacgt ccgcggg 37
<210> 52
   <211> 48
   <212> DNA
   <213> Artificial Sequence
<220>
   <223> synthetic oligonucleotide
<400> 52
   cgaccggatt cccacatcaa atgatgcggg ttgtgttaat tccggtcg 48
<210> 53
   <211> 37
   <212> DNA
   <213> Artificial Sequence
<220>
   <223> synthetic oligonucleotide
<400> 53
   cccgcgcrta gttactrcgt tgtaagacgt ccgcggg 37
<210> 54
   <211> 29
   <212> DNA
   <213> Artificial Sequence
<220>
   <223> synthetic oligonucleotide
<400> 54
   ccccgtagtt actgcgttgt aagacgggg 29
<210> 55
   <211> 37
   <212> DNA
   <213> Artificial Sequence
<220>
   <223> synthetic oligonucleotide
<400> 55
   cccgcgcata gttactgcgt tgtaagacgt ccgcggg 37
<210> 56
   <211> 37
   <212> DNA
   <213> Artificial Sequence
<220>
   <223> synthetic oligonucleotide
<400> 56
   cccgcgcgta gttactacgt tgtaagacgt ccgcggg 37
<210> 57
   <211> 19
   <212> DNA
   <213> Artificial Sequence
<220>
   <223> synthetic oligonucleotide
<400> 57
   ggatcaaacg gcctgcaca 19
<210> 58
   <211> 21
   <212> DNA
   <213> Artificial Sequence
<220>
   <223> synthetic oligonucleotide
<400> 58
   atcaaatgat gcgggttgtg t 21
<210> 59
   <211> 19
   <212> DNA
   <213> Artificial Sequence
<220>
   <223> synthetic oligonucleotide
<400> 59
   tcattggcgg atcaaacgg 19
<210> 60
   <211> 22
   <212> DNA
   <213> Artificial Sequence
<220>
   <223> synthetic oligonucleotide
<400> 60
   acaacgcagt aactacgcac ta 22
<210> 61
   <211> 22
   <212> DNA
   <213> Artificial Sequence
<220>
   <223> synthetic oligonucleotide
<400> 61
   taactacgca ctatcattca gc 22
<210> 62
   <211> 22
   <212> DNA
   <213> Artificial Sequence
<220>
   <223> synthetic oligonucleotide
<400> 62
   acatcaaatg atgcgggttg tg 22
<210> 63
   <211> 22
   <212> DNA
   <213> Artificial Sequence
<220>
   <223> synthetic oligonucleotide
<400> 63
   tcaaatgatg cgggttgtgt ta 22
<210> 64
   <211> 24
   <212> DNA
   <213> Artificial Sequence
<220>
   <223> synthetic oligonucleotide
<400> 64
   caaatgatgc gggttgtgtt aatt 24
<210> 65
   <211> 29
   <212> DNA
   <213> Artificial Sequence
<220>
   <223> synthetic oligonucleotide
<400> 65
   gtcaaaaatc atgaacctca ttacttatg 29
<210> 66
   <211> 28
   <212> DNA
   <213> Artificial Sequence
<220>
   <223> synthetic oligonucleotide
<400> 66
   ctatgtcaaa aatcatgaac ctcattac 28
<210> 67
   <211> 23
   <212> DNA
   <213> Artificial Sequence
<220>
   <223> synthetic oligonucleotide
<400> 67
   ggaggctaac tatgtcaaaa atc 23
<210> 68
   <211> 27
   <212> DNA
   <213> Artificial Sequence
<220>
   <223> synthetic oligonucleotide
<400> 68
   gctgaaaaaa ccgcatcatt trtgrta 27
<210> 69
   <211> 27
   <212> DNA
   <213> Artificial Sequence
<220>
   <223> synthetic oligonucleotide
<400> 69
   gctgaaaaaa ccgcatcatt tatgata 27
<210> 70
   <211> 29
   <212> DNA
   <213> Artificial Sequence
<220>
   <223> synthetic oligonucleotide
<400> 70
   atttcatata tgtaattcct ccacatctc 29
<210> 71
   <211> 29
   <212> DNA
   <213> Artificial Sequence
<220>
   <223> synthetic oligonucleotide
<400> 71
   tttagtttta tttatgatac gcttctcca 29
<210> 72
   <211> 25
   <212> DNA
   <213> Artificial Sequence
<220>
   <223> synthetic oligonucleotide
<400> 72
   ctctataaac atcgtatgat attgc 25
<210> 73
   <211> 29
   <212> DNA
   <213> Artificial Sequence
<220>
   <223> synthetic oligonucleotide
<400> 73
   caaatattat ctcgtaattt accttgttc 29
<210> 74
   <211> 29
   <212> DNA
   <213> Artificial Sequence
<220>
   <223> synthetic oligonucleotide
<400> 74
   ctctgcttta tattataaaa ttacggctg 29
<210> 75
   <211> 22
   <212> DNA
   <213> Artificial Sequence
<220>
   <223> synthetic oligonucleotide
<400> 75
   ttcgttccct ccattaactg tc 22
<210> 76
   <211> 22
   <212> DNA
   <213> Artificial Sequence
<220>
   <223> synthetic oligonucleotide
<400> 76
   tcaccgtctt tcttttgacc tt 22
<210> 77
   <211> 27
   <212> DNA
   <213> Artificial Sequence
<220>
   <223> synthetic oligonucleotide
<400> 77
   cactttttat tcttcaaaga tttgagc 27
<210> 78
   <211> 28
   <212> DNA
   <213> Artificial Sequence
<220>
   <223> synthetic oligonucleotide
<400> 78
   atggaaattc ttaatcttta cttgtacc 28
<210> 79
   <211> 23
   <212> DNA
   <213> Artificial Sequence
<220>
   <223> synthetic oligonucleotide
<400> 79
   cagcaattcw cataaacctc ata 23
<210> 80
   <211> 27
   <212> DNA
   <213> Artificial Sequence
<220>
   <223> synthetic oligonucleotide
<400> 80
   acaaactttg aggggatttt tagtaaa 27
<210> 81
   <211> 24
   <212> DNA
   <213> Artificial Sequence
<220>
   <223> synthetic oligonucleotide
<400> 81
   tgataagcca ttcattcacc ctaa 24
<210> 82
   <211> 24
   <212> DNA
   <213> Artificial Sequence
<220>
   <223> synthetic oligonucleotide
<400> 82
   tagttactgt gttgtaagac gtcc 24
<210> 83
   <211> 26
   <212> DNA
   <213> Artificial Sequence
<220>
   <223> synthetic oligonucleotide
<400> 83
   ctactatgaa ctgtgcaatt tgttct 26
<210> 84
   <211> 26
   <212> DNA
   <213> Artificial Sequence
<220>
   <223> synthetic oligonucleotide
<400> 84
   gcaattcaca taaacctcat atgttc 26
<210> 85
   <211> 24
   <212> DNA
   <213> Artificial Sequence
<220>
   <223> synthetic oligonucleotide
<400> 85
   acctcatatg ttctgataca ttca 24
<210> 86
   <211> 22
   <212> DNA
   <213> Artificial Sequence
<220>
   <223> synthetic oligonucleotide
<400> 86
   gcaattcaca taaacctcat at 22
<210> 87
   <211> 25
   <212> DNA
   <213> Artificial Sequence
<220>
   <223> synthetic oligonucleotide
<400> 87
   cataacagca attcacataa acctc 25
<210> 88
   <211> 22
   <212> DNA
   <213> Artificial Sequence
<220>
   <223> synthetic oligonucleotide
<400> 88
   taacagcaat tcacataaac ct 22
<210> 89
   <211> 27
   <212> DNA
   <213> Artificial Sequence
<220>
   <223> synthetic oligonucleotide
<400> 89
   cgctattatt tacttgaaat gaaagac 27
<210> 90
   <211> 21
   <212> DNA
   <213> Artificial Sequence
<220>
   <223> synthetic oligonucleotide
<400> 90
   cttgaaatga aagactgcgg a 21
<210> 91
   <211> 54
   <212> DNA
   <213> Artificial Sequence
<220>
   <223> synthetic oligonucleotide
<400> 91
   ttgcttcact ataagtattc agtataaaga atttacttga aatgaaagac tgcg 54
<210> 92
   <211> 24
   <212> DNA
   <213> Artificial Sequence
<220>
   <223> synthetic oligonucleotide
<400> 92
   atttacttga aatgaaagac tgcg 24
<210> 93
   <211> 29
   <212> DNA
   <213> Artificial Sequence
<220>
   <223> synthetic oligonucleotide
<400> 93
   aaagaatatt tcgctattat ttacttgaa 29
<210> 94
   <211> 29
   <212> DNA
   <213> Artificial Sequence
<220>
   <223> synthetic oligonucleotide
<400> 94
   tcagtataaa gaatatttcg ctattattt 29
<210> 95
   <211> 20
   <212> DNA
   <213> Artificial Sequence
<220>
   <223> synthetic oligonucleotide
<400> 95
   tgaaatgaaa gactgcggag 20
<210> 96
   <211> 27
   <212> DNA
   <213> Artificial Sequence
<220>
   <223> synthetic oligonucleotide
<400> 96
   aacctcatat gttctgatac attcaaa 27
<210> 97
   <211> 28
   <212> DNA
   <213> Artificial Sequence
<220>
   <223> synthetic oligonucleotide
<400> 97
   tatgtcaaaa atcatgaacc tcattact 28
<210> 98
   <211> 25
   <212> DNA
   <213> Artificial Sequence
<220>
   <223> synthetic oligonucleotide
<400> 98
   cataacagca attcacataa acctc 25
<210> 99
   <211> 17
   <212> DNA
   <213> Artificial Sequence
<220>
   <223> synthetic oligonucleotide
<400> 99
   gactgcggag gctaact 17
<210> 100
   <211> 18
   <212> DNA
   <213> Artificial Sequence
<220>
   <223> synthetic oligonucleotide
<400> 100
   atccctttat gaagcggc 18
<210> 101
   <211> 20
   <212> DNA
   <213> Artificial Sequence
<220>
   <223> synthetic oligonucleotide
<400> 101
   tgaaatgaaa gactgcggag 20
<210> 102
   <211> 30
   <212> DNA
   <213> Artificial Sequence
<220>
   <223> synthetic oligonucleotide
<400> 102
   gcaaggtata atccaatatt tcatatatgt 30
<210> 103
   <211> 29
   <212> DNA
   <213> Artificial Sequence
<220>
   <223> synthetic oligonucleotide
<400> 103
   agttccataa tcaatataat ttgtacagt 29
<210> 104
   <211> 23
   <212> DNA
   <213> Artificial Sequence
<220>
   <223> synthetic oligonucleotide
<400> 104
   acatcgtatg atattgcaag gta 23
<210> 105
   <211> 26
   <212> DNA
   <213> Artificial Sequence
<220>
   <223> synthetic oligonucleotide
<400> 105
   ctttcattct ttcttgattc cattag 26
<210> 106
   <211> 27
   <212> DNA
   <213> Artificial Sequence
<220>
   <223> synthetic oligonucleotide
<400> 106
   cactctataa acatcgtatg atattgc 27
<210> 107
   <211> 30
   <212> DNA
   <213> Artificial Sequence
<220>
   <223> synthetic oligonucleotide
<400> 107
   ttcttaattt aattgtagtt ccataatcaa 30
<210> 108
   <211> 30
   <212> DNA
   <213> Artificial Sequence
<220>
   <223> synthetic oligonucleotide
<400> 108
   aattatacac aacctaattt ttagttttat 30
<210> 109
   <211> 29
   <212> DNA
   <213> Artificial Sequence
<220>
   <223> synthetic oligonucleotide
<400> 109
   aatttttagt tttatttatg atacgcttc 29
<210> 110
   <211> 30
   <212> DNA
   <213> Artificial Sequence
<220>
   <223> synthetic oligonucleotide
<400> 110
   acacaaccta atttttagtt ttatttatga 30
<210> 111
   <211> 30
   <212> DNA
   <213> Artificial Sequence
<220>
   <223> synthetic oligonucleotide
<400> 111
   tttattaaac actctataaa catcgtatga 30
<210> 112
   <211> 31
   <212> DNA
   <213> Artificial Sequence
<220>
   <223> synthetic oligonucleotide
<400> 112
   tcacatctca ttaaattttt aaattataca c 31
<210> 113
   <211> 31
   <212> DNA
   <213> Artificial Sequence
<220>
   <223> synthetic oligonucleotide
<400> 113
   ccacatctca ttaaattttt aaattataca c 31
<210> 114
   <211> 30
   <212> DNA
   <213> Artificial Sequence
<220>
   <223> synthetic oligonucleotide
<400> 114
   atattataca caatccgttt tttagtttta 30
<210> 115
   <211> 29
   <212> DNA
   <213> Artificial Sequence
<220>
   <223> synthetic oligonucleotide
<400> 115
   acacaatccg ttttttagtt ttatttatg 29
<210> 116
   <211> 30
   <212> DNA
   <213> Artificial Sequence
<220>
   <223> synthetic oligonucleotide
<400> 116
   ttctaattta tttaacataa aatcaatcct 30
<210> 117
   <211> 33
   <212> DNA
   <213> Artificial Sequence
<220>
   <223> synthetic oligonucleotide
<400> 117
   caatcctttt tatatttaaa atatattata cac 33
<210> 118
   <211> 22
   <212> DNA
   <213> Artificial Sequence
<220>
   <223> synthetic oligonucleotide
<400> 118
   aagtcgcttt gcctttgggt ca 22
<210> 119
   <211> 26
   <212> DNA
   <213> Artificial Sequence
<220>
   <223> synthetic oligonucleotide
<400> 119
   tacaaagtcg ctttgccttt gggtca 26
<210> 120
   <211> 19
   <212> DNA
   <213> Artificial Sequence
<220>
   <223> synthetic oligonucleotide
<400> 120
   ggccgtttga tccgccaat 19
<210> 121
   <211> 21
   <212> DNA
   <213> Artificial Sequence
<220>
   <223> synthetic oligonucleotide
<400> 121
   aagtcgcttt gcccttgggt a 21
<210> 122
   <211> 20
   <212> DNA
   <213> Artificial Sequence
<220>
   <223> synthetic oligonucleotide
<400> 122
   aagtcgcttt gcccttgggt 20
<210> 123
   <211> 22
   <212> DNA
   <213> Artificial Sequence
<220>
   <223> synthetic oligonucleotide
<400> 123
   aagtcgcttt gcccttgggt ca 22
<210> 124
   <211> 19
   <212> DNA
   <213> Artificial Sequence
<220>
   <223> synthetic oligonucleotide
<400> 124
   aagtcgcttt gcccttggg 19
<210> 125
   <211> 20
   <212> DNA
   <213> Artificial Sequence
<220>
   <223> synthetic oligonucleotide
<400> 125
   caagaattga accaacgcat 20
<210> 126
   <211> 30
   <212> DNA
   <213> Artificial Sequence
<220>
   <223> synthetic oligonucleotide
<400> 126
   caatgacgaa tacatagtcg ctttgccctt 30
<210> 127
   <211> 21
   <212> DNA
   <213> Artificial Sequence
<220>
   <223> synthetic oligonucleotide
<400> 127
   cgtttgatcc gccaatgacg a 21
<210> 128
   <211> 22
   <212> DNA
   <213> Artificial Sequence
<220>
   <223> synthetic oligonucleotide
<400> 128
   gccaatcctt cggaagatag ca 22
<210> 129
   <211> 18
   <212> DNA
   <213> Artificial Sequence
<220>
   <223> synthetic oligonucleotide
<400> 129
   attaacacaa cccgcatc 18
<210> 130
   <211> 19
   <212> DNA
   <213> Artificial Sequence
<220>
   <223> synthetic oligonucleotide
<400> 130
   gtcgctttgc ccttgggtc 19
<210> 131
   <211> 20
   <212> DNA
   <213> Artificial Sequence
<220>
   <223> synthetic oligonucleotide
<400> 131
   tcgctttgcc cttgggtcat 20
<210> 132
   <211> 19
   <212> DNA
   <213> Artificial Sequence
<220>
   <223> synthetic oligonucleotide
<400> 132
   ggccgtttga tccgccaat 19
<210> 133
   <211> 22
   <212> DNA
   <213> Artificial Sequence
<220>
   <223> synthetic oligonucleotide
<400> 133
   gtccttgtgc aggccgtttg at 22
<210> 134
   <211> 24
   <212> DNA
   <213> Artificial Sequence
<220>
   <223> synthetic oligonucleotide
<400> 134
   cttgggtcat gcgttggttc aatt 24
<210> 135
   <211> 27
   <212> DNA
   <213> Artificial Sequence
<220>
   <223> synthetic oligonucleotide
<400> 135
   cgaatacaaa gtcgctttgc ccttggg 27
<210> 136
   <211> 20
   <212> DNA
   <213> Artificial Sequence
<220>
   <223> synthetic oligonucleotide
<400> 136
   atgcgttggt tcaattcttg 20
<210> 137
   <211> 20
   <212> DNA
   <213> Artificial Sequence
<220>
   <223> synthetic oligonucleotide
<400> 137
   gcgttggttc aattcttggg 20
<210> 138
   <211> 20
   <212> DNA
   <213> Artificial Sequence
<220>
   <223> synthetic oligonucleotide
<400> 138
   acccaagggc aaagcgactt 20
<210> 139
   <211> 24
   <212> DNA
   <213> Artificial Sequence
<220>
   <223> synthetic oligonucleotide
<400> 139
   ggtaatgcgt tggttcaatt cttg 24
<210> 140
   <211> 25
   <212> DNA
   <213> Artificial Sequence
<220>
   <223> synthetic oligonucleotide
<400> 140
   acaaagtcgc tatgcccttg ggtca 25
<210> 141
   <211> 29
   <212> DNA
   <213> Artificial Sequence
<220>
   <223> synthetic oligonucleotide
<400> 141
   ctttccttgt atttctaatg taatgactg 29
<210> 142
   <211> 27
   <212> DNA
   <213> Artificial Sequence
<220>
   <223> synthetic oligonucleotide
<400> 142
   ttgatgtggg aatgtcattt tgctgaa 27
<210> 143
   <211> 25
   <212> DNA
   <213> Artificial Sequence
<220>
   <223> synthetic oligonucleotide
<400> 143
   gcgttggttc aattcttggg ccaat 25
<210> 144
   <211> 29
   <212> DNA
   <213> Artificial Sequence
<220>
   <223> synthetic oligonucleotide
<400> 144
   gttggttcaa ttcttgggcc aatccttcg 29
<210> 145
   <211> 26
   <212> DNA
   <213> Artificial Sequence
<220>
   <223> synthetic oligonucleotide
<400> 145
   cgaatacaaa gtcgctttgc ccttgg 26
<210> 146
   <211> 29
   <212> DNA
   <213> Artificial Sequence
<220>
   <223> synthetic oligonucleotide
<400> 146
   gccaatgacg aatacaaagt cgctttgcc 29
<210> 147
   <211> 25
   <212> DNA
   <213> Artificial Sequence
<220>
   <223> synthetic oligonucleotide
<400> 147
   tgggccaatc cttcggaaga tagca 25
<210> 148
   <211> 20
   <212> DNA
   <213> Artificial Sequence
<220>
   <223> synthetic oligonucleotide
<400> 148
   atgcgttggt tcgattcttg 20
<210> 149
   <211> 21
   <212> DNA
   <213> Artificial Sequence
<220>
   <223> synthetic oligonucleotide
<400> 149
   catgcgttgg ttcgattctt g 21
<210> 150
   <211> 19
   <212> DNA
   <213> Artificial Sequence
<220>
   <223> synthetic oligonucleotide
<400> 150
   aagtcgcttt gcccttggg 19
<210> 151
   <211> 21
   <212> DNA
   <213> Artificial Sequence
<220>
   <223> synthetic oligonucleotide
<400> 151
   catgcgttgg ttcgattctt g 21
<210> 152
   <211> 23
   <212> DNA
   <213> Artificial Sequence
<220>
   <223> synthetic oligonucleotide
<400> 152
   aagtcgcttt gcccttgggt cat 23
<210> 153
   <211> 25
   <212> DNA
   <213> Artificial Sequence
<220>
   <223> synthetic oligonucleotide
<400> 153
   tgctcaatta acacaacccg catca 25
<210> 154
   <211> 33
   <212> DNA
   <213> Artificial Sequence
<220>
   <223> synthetic oligonucleotide
<400> 154
   gccgcgctgc tcaattaaca caacccgcgc ggc 33
<210> 155
   <211> 32
   <212> DNA
   <213> Artificial Sequence
<220>
   <223> synthetic oligonucleotide
<400> 155
   gccgcgcatg cgttggttca attctgcgcg gc 32
<210> 156
   <211> 2123
   <212> DNA
   <213> Staphylococcus aureus
<400> 156
   ctccatatca caaaaattat aacattattt tgacataaat actacatttg taatatacta 60
   caaatgtagt cttatataag gaggatattg atgaaaaaga taaaaattgt tccacttatt 120
   ttaatagttg tagttgtcgg gtttggtata tatttttatg cttcaaaaga taaagaaatt 180
   aataatacta ttgatgcaat tgaagataaa aatttcaaac aagtttataa agatagcagt 240
   tatatttcta aaagcgataa tggtgaagta gaaatgactg aacgtccgat aaaaatatat 300
   aatagtttag gcgttaaaga tataaacatt caggatcgta aaataaaaaa agtatctaaa 360
   aataaaaaac gagtagatgc tcaatataaa attaaaacaa actacggtaa cattgatcgc 420
   aacgttcaat ttaattttgt taaagaagat ggtatgtgga agttagattg ggatcatagc 480
   gtcattattc caggaatgca gaaagaccaa agcatacata ttgaaaattt aaaatcagaa 540
   cgtggtaaaa ttttagaccg aaacaatgtg gaattggcca atacaggaac agcatatgag 600
   ataggcatcg ttccaaagaa tgtatctaaa aaagattata aagcaatcgc taaagaacta 660
   agtatttctg aagactatat caaacaacaa atggatcaaa attgggtaca agatgatacc 720
   ttcgttccac ttaaaaccgt taaaaaaatg gatgaatatt taagtgattt cgcaaaaaaa 780
   tttcatctta caactaatga aacagaaagt cgtaactatc ctctagaaaa agcgacttca 840
   catctattag gttatgttgg tcccattaac tctgaagaat taaaacaaaa agaatataaa 900
   ggctataaag atgatgcagt tattggtaaa aagggactcg aaaaacttta cgataaaaag 960
   ctccaacatg aagatggcta tcgtgtcaca atcgttgacg ataatagcaa tacaatcgca 1020
   catacattaa tagagaaaaa gaaaaaagat ggcaaagata ttcaactaac tattgatgct 1080
   aaagttcaaa agagtattta taacaacatg aaaaatgatt atggctcagg tactgctatc 1140
   caccctcaaa caggtgaatt attagcactt gtaagcacac cttcatatga cgtctatcca 1200
   tttatgtatg gcatgagtaa cgaagaatat aataaattaa ccgaagataa aaaagaacct 1260
   ctgctcaaca agttccagat tacaacttca ccaggttcaa ctcaaaaaat attaacagca 1320
   atgattgggt taaataacaa aacattagac gataaaacaa gttataaaat cgatggtaaa 1380
   ggttggcaaa aagataaatc ttggggtggt tacaacgtta caagatatga agtggtaaat 1440
   ggtaatatcg acttaaaaca agcaatagaa tcatcagata acatttcttt tgctagagta 1500
   gcactcgaat taggcagtaa gaaatttgaa aaaggcatga aaaaactagg tgttggtgaa 1560
   gatataccaa gtgattatcc attttataat gctcaaattt caaacaaaaa tttagataat 1620
   gaaatattat tagctgattc aggttacgga caaggtgaaa tactgattaa cccagtacag 1680
   atcctttcaa tctatagcgc attagaaaat aatggcaata ttaacgcacc tcacttatta 1740
   aaagacacga aaaacaaagt ttggaagaaa aatattattt ccaaagaaaa tatcaatcta 1800
   ttaactgatg gtatgcaaca agtcgtaaat aaaacacata aagaagatat ttatagatct 1860
   tatgcaaact taattggcaa atccggtact gcagaactca aaatgaaaca aggagaaact 1920
   ggcagacaaa ttgggtggtt tatatcatat gataaagata atccaaacat gatgatggct 1980
   attaatgtta aagatgtaca agataaagga atggctagct acaatgccaa aatctcaggt 2040
   aaagtgtatg atgagctata tgagaacggt aataaaaaat acgatataga tgaataacaa 2100
   aacagtgaag caatccgtaa cga                                         2123
<210> 157
   <211> 1998
   <212> **DNA**
   <213> Staphylococcus aureus
<400> 157
   ttactgatct atatcaaatt gagtttttcc attatcatac aaatcatcat aaacttttcc 60
   agatatagta gcattatagc tggccatccc tttattttga acgtctttaa cattaatcgc 120
   cattaacata ttaggattat ttttattata tgaaacaaac caacctattt gtcttccagt 180
   ttccccttga ttcattttta attctgctgt gccagattta ccaataattc gggcataatt 240
   tttgtatata tcatccctat gtgttttatt aactacacgt tccataccat tagttaatat 300
   atctatgtct tttttaggta taatatcttt tttccatatt tgagattttg ttttacgtaa 360
   aacatgagga ttttgtatat ttccgttatt ttctaaagca ctgtatattg ataaaatttg 420
   tatagggttt actagtatct cgccttggcc atatcctgaa tctgctaata atatttcatt 480
   ttttaaatta ctatttgaga tttgtgcttt ataaaaggga taatcactcg ggatattttc 540
   accgattccc aaatcttgca taccttgctc aaattttttg gctcctaatg ctaatgcaat 600
   gcgggcaaaa aatatgttgt ctgatgattc tattgcttgc tttaaatcga tattgccgtc 660
   tactacttta aatcttgtga tattataatt accccatgat gcatcttttt gccaaccctt 720
   accataaata tcaaaattag tatttttgtc tagtttattt tcttttaagg ctataataga 780
   tgttaatatt ttttgggttg aacctggtga tgtagtgatt tgaaatttgt tgagcaaagg 840
   ctctttttta ttgttagtta atttacggta gtcattattg cttaatccat tcatgaatgg 900
   ataaacatcg tacgatgggg tacttaccaa agctaaaatt tctccagttt ttggttgtaa 960
   tgctgtacca gatccatcgt catttttcat atgtttataa atactttctt gtactctagc 1020
   atctatagtt aaatgaagat cttttccgtt ttcagccttt ttctccaata atgtgtctaa 1080
   aggtttattg tcataagtat ttgcaatgga taccttaaaa ccatcagtgt tttgcaattg 1140
   tttatcatag aggcgttcta agcctttttt tccaataaca gtatttttgc tatagtttct 1200
   aaattgctta ctttttaact cgtcagaatt aattggaccc acataaccta aaaggtgtac 1260
   tgttgcttcg ttcaatggat aaacacggct ttttatagtg tttatttgta aattgtatga 1320
   tttaattaat ttgtctatat attcatcttg tttatttatc tttttaattg gtacaaatga 1380
   atctggctga acccattttt gattaacttt attggttata gcttttgtat caatttgtaa 1440
   gtcacgagca atatcatcat atttttcttt gggtgttttg ttagggacaa taccgatttc 1500
   atatgtattt ccagttttag ctaattctat accatttcta tcttttattt tgcctcgctc 1560
   tgattttaat gtttctatat taattttctg tccatttttc aaaccaggta ctattacgtc 1620
   tggtctccaa tctaatttcc aatgcttatc ttcataaata aagtttaatt gtgtattacg 1680
   tcgtatagtt ccatattttg tatatatgtt atatttaaca tcaacttgct ttttatcttt 1740
   tccagttttt ttaatttcat gattagtaat ttttaagtta ttgacactta aatctttgta 1800
   aattttttta ttcctatcta caatttcttc ttctccatat gccagtttag atttttctga 1860
   actattttta tatacttcgt tatagtttcc tttttcaata gaactaattg ttttctcaat 1920
   atcgtcatct ttgaataacc aagttattat aatcataatt agtaaaagaa ctagcacact 1980
   aatataaatt tttttcat                                                1998
<210> 158
   <211> 686
   <212> DNA
   <213> Staphylococcus aureus
<400> 158
   atgacagaat acttattaag tgctggcata tgtatggcaa ttgtttcaat attacttata 60
   gggatggcta tcagtaatgt ttcgaaaggg caatacgcaa agaggttttt ctttttcgct 120
   actagttgct tagtgttaac tttagttgta gtttcaagtc taagtagctc agcaaatgca 180
   tcacaaacag ataacggcgt aaatagaagt ggttctgaag atccaacagt atatagtgca 240
   acttcaacta aaaaattaca taaagaacct gcgacattaa ttaaagcgat tgatggtgat 300
   acggttaaat taatgtacaa aggtcaacca atgacattca gactattatt agttgataca 360
   cctgaaacaa agcatcctaa aaaaggtgta gagaaatatg gccctgaagc aagtgcattt 420
   acgaaaaaaa tggtagaaaa tgcaaataaa attgaagtcg agtttgacaa aggtcaaaga 480
   actgataaat atggacgtgg cttagcgtat atttatgctg atggaaaaat ggtaaacgaa 540
   gctttagttc gtcaaggctt ggctaaagtt gcttatgttt ataaacctaa caatacacat 600
   gaacaacttt taagaaaaag tgaagcacaa gcgaaaaaag agaaattaaa tatttggagc 660
   gaagacaacg ctgattcagg tcaata 686
<210> 159
   <211> 686
   <212> DNA
   <213> Staphylococcus aureus
<400> 159
   atgacagaat acttattaag tgctggcata tgtatggcaa ttgtttcaat attacttata 60
   gggatggcta tcagtaatgt ttcgaaaggg caatacgcaa agaggttttt ctttttcgct 120
   actagttgct tagtgttaac tttagttgta gtttcaagtc taagtagctc agcaaatgca 180
   tcacaaacag ataacggcgt aaatagaagt ggttctgaag atccaacagt atatagtgca 240
   acttcaacta aaaaattaca taaagaacct gcgacattaa ttaaagcgat tgatggtgat 300
   acggttaaat taatgtacaa aggtcaacca atgacattca gactattatt agttgataca 360
   cctgaaacaa agcatcctaa aaaaggtgta gagaaatatg gccctgaagc aagtgcattt 420
   acgaaaaaaa tggtagaaaa tgcaaataaa attgaagtcg agtttgacaa aggtcaaaga 480
   actgataaat atggacgtgg cttagcgtat atttatgctg atggaaaaat ggtaaacgaa 540
   gctttagttc gtcaaggctt ggctaaagtt gcttatgttt ataaacctaa caatacacat 600
   gaacaacttt taagaaaaag tgaagcacaa gcgaaaaaag agaaattaaa tatttggagc 660
   gaagacaacg ctgattcagg tcaata 686
<210> 160
   <211> 442
   <212> **DNA**
   <213> Staphylococcus aureus
<400> 160
   gatcaatctt tgtcggtaca cgatattctt cacgactaaa taaacgctca ttcgcgattt 60
   tataaatgaa tgttgataac aatgttgtat tatctactga aatctcatta cgttgcatcg 120
   gaaacattgt gttctgtatg taaaagccgt cttgataatc tttagtagta ccgaagctgg 180
   tcatacgaga gttatatttt ccagccaaaa cgatattttt ataatcatta cgtgaaaaag 240
   gtttcccttc attatcacac aaatatttta gcttttcagt ttctatatca actgtagctt 300
   ctttatccat acgttgaata attgtacgat tctgacgcac catcttttgc acacctttaa 360
   tgttatttgt tttaaaagca tgaataagtt tttcaacaca acgatgtgaa tcttctaaga 420
   agtcaccgta aaatgaagga tc 442
<210> 161
   <211> 24
   <212> DNA
   <213> Artificial Sequence
<220>
   <223> Synthetic oligonucleotide
<400> 161
   acagaatact tattaagtgc tggc 24
<210> 162
   <211> 27
   <212> DNA
   <213> Artificial Sequence
<220>
   <223> Synthetic oligonucleotide
<400> 162
   gtttcaatat tacttatagg gatggct 27
<210> 163
   <211> 24
   <212> DNA
   <213> Artificial Sequence
<220>
   <223> Synthetic oligonucleotide
<400> 163
   ctgatggaaa aatggtaaac gaag 24
<210> 164
   <211> 22
   <212> DNA
   <213> Artificial Sequence
<220>
   <223> Synthetic oligonucleotide
<400> 164
   atggaaaaat ggtaaacgaa gc 22
<210> 165
   <211> 22
   <212> DNA
   <213> Artificial Sequence
<220>
   <223> Synthetic oligonucleotide
<400> 165
   tgctgagcta cttagacttg aa 22
<210> 166
   <211> 22
   <212> DNA
   <213> Artificial Sequence
<220>
   <223> Synthetic oligonucleotide
<400> 166
   gcatttgctg agctacttag ac 22
<210> 167
   <211> 25
   <212> DNA
   <213> Artificial Sequence
<220>
   <223> Synthetic oligonucleotide
<400> 167
   gttgttcatg tgtattgtta ggttt 25
<210> 168
   <211> 22
   <212> DNA
   <213> Artificial Sequence
<220>
   <223> Synthetic oligonucleotide
<400> 168
   ttattgacct gaatcagcgt tg 22
<210> 169
   <211> 30
   <212> DNA
   <213> Artificial Sequence
<220>
   <223> Synthetic oligonucleotide
<400> 169
   cgaaacatta ctgatagcca tccctataag 30
<210> 170
   <211> 40
   <212> DNA
   <213> cgcaccgaaa cattactgat agccatccct ataaggtgcgArtific
<220>
   <223> Synthetic oligonucleotide
<400> 170
   cgcaccgaaa cattactgat agccatccct ataaggtgcg 40
<210> 171
   <211> 29
   <212> DNA
   <213> Artificial Sequence
<220>
   <223> Synthetic oligonucleotide
<400> 171
   acataagcaa ctttagccaa gccttgacg 29
<210> 172
   <211> 41
   <212> DNA
   <213> Artificial Sequence
<220>
   <223> Synthetic oligonucleotide
<400> 172
   cgcgcaacat aagcaacttt agccaagcct tgacgtgcgc g 41
<210> 173
   <211> 30
   <212> DNA
   <213> Artificial Sequence
<220>
   <223> Synthetic oligonucleotide
<400> 173
   aatctttgtc ggtacacgat attcttcacg 30
<210> 174
   <211> 30
   <212> DNA
   <213> Artificial Sequence
<220>
   <223> Synthetic oligonucleotide
<400> 174
   cgtaatgaga tttcagtaga taatacaaca 30
<210> 175
   <211> 20
   <212> DNA
   <213> Artificial Sequence
<220>
   <223> Synthetic oligonucleotide
<400> 175
   ttacagagtt aactgttacc 20
<210> 176
   <211> 20
   <212> DNA
   <213> Artificial Sequence
<220>
   <223> Synthetic oligonucleotide
<400> 176
   atacaaatcc agcacgctct 20
<210> 177
   <211> 19
   <212> DNA
   <213> Artificial Sequence
<220>
   <223> Synthetic oligonucleotide
<400> 177
   tkggaccmac ataacctaa 19
<210> 178
   <211> 20
   <212> DNA
   <213> Artificial Sequence
<220>
   <223> Synthetic oligonucleotide
<400> 178
   ggacccacat aacctaaaag 20
<210> 179
   <211> 20
   <212> DNA
   <213> Artificial Sequence
<220>
   <223> Synthetic oligonucleotide
<400> 179
   ctaacaaaac acccaaagaa 20
<210> 180
   <211> 23
   <212> DNA
   <213> Artificial Sequence
<220>
   <223> Synthetic oligonucleotide
<400> 180
   cctgaatctg ctaataatat ttc 23
<210> 181
   <211> 20
   <212> DNA
   <213> Artificial Sequence
<220>
   <223> Synthetic oligonucleotide
<400> 181
   tcaccaggtt caacccaaaa 20
<210> 182
   <211> 21
   <212> DNA
   <213> Artificial Sequence
<220>
   <223> Synthetic oligonucleotide
<400> 182
   ggccatatcc tgaatctgct a 21
<210> 183
   <211> 23
   <212> DNA
   <213> Artificial Sequence
<220>
   <223> Synthetic oligonucleotide
<400> 183
   gcaagcaata gaatcatcag aca 23
<210> 184
   <211> 27
   <212> DNA
   <213> Artificial Sequence
<220>
   <223> Synthetic oligonucleotide
<400> 184
   ttggtacaaa tgaatctggc tgaaccc 27
<210> 185
   <211> 26
   <212> DNA
   <213> Artificial Sequence
<220>
   <223> Synthetic oligonucleotide
<400> 185
   tgcttcgttc aatggataaa cacggc 26
<210> 186
   <211> 26
   <212> DNA
   <213> Artificial Sequence
<220>
   <223> Synthetic oligonucleotide
<400> 186
   tggctcctaa tgctaatgca atgcgg 26
<210> 187
   <211> 27
   <212> DNA
   <213> Artificial Sequence
<220>
   <223> Synthetic oligonucleotide
<400> 187
   ccgcattgca ttagcattag gagccaa 27
<210> 188
   <211> 774
   <212> DNA
   <213> Staphylococcus aureus
<220>
   <221> misc_feature
   <222> 4, 6
   <223> n= c, t, a, or g
<400> 188
   tcangnggga tcaaacggcc tgcacaagga cgtcttacaa cgcagtaact acgcactatc 60
   attcagcaaa atgacatttc cacatcaaat gatgcgggtt gtgttaattg agcaagtgta 120
   tagagcgttt aagattatgc gcggagaagc gtatcacaaa tgatgcggtt tttttaacct 180
   ctttacgtat gaggtttatg agaattgccg ttatgttttg cgagagttat caatcttttt 240
   gatagtaaga aagtacatag aaactaaaag agtattttta tctacaatag catttataat 300
   ttattctatt attgtatact ttattttaat tattagtatc attgctgaga tgttacttga 360
   tattctatgt ctatttttta ggaaattcta tactattaaa attatggtat tttatacgca 420
   ataaaggact aatctatttt atacagatta gtcctttatt gtagtcttta aaaactagtt 480
   actcattaat attttttagt acaatttcag caacctcact tactattttg tcattaggtt 540
   taccatcttt tctatcttta tttgtaaata tcaccagaat tataggttta tcttgtccat 600
   ctggataaac aaacgcaaca tcgtttcttg aaccgtatgt tagtgcttga ccgctcttat 660
   ccataacttt aaagtttgaa ggtgcaccat ccttaattaa tgtatcgcca cttttatttt 720
   tgaacattag attaagtaag aaatctttgt ttgctttgct aagatctcca tcag       774